(19) Europäisches Patentamt
European Patent Office
Office européen des brevets

(11) **EP 4 186 500 A1**

(12) **EUROPEAN PATENT APPLICATION**

(43) Date of publication:
**31.05.2023 Bulletin 2023/22**

(21) Application number: **21210256.0**

(22) Date of filing: **24.11.2021**

(51) International Patent Classification (IPC):
***A61K 31/196*** *(2006.01)* ***A61P 31/14*** *(2006.01)*

(52) Cooperative Patent Classification (CPC):
**A61K 31/196; A61P 31/14**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Atriva Therapeutics GmbH
72072 Tübingen (DE)**

(72) Inventors:
- **PLANZ, Oliver
72581 Dettingen an der Erm (DE)**
- **FÜLL, Yvonne
72072 Tübingen (DE)**

(74) Representative: **Schiweck Weinzierl Koch Patentanwälte Partnerschaft mbB
Ganghoferstraße 68 B
80339 München (DE)**

(54) **MEK INHIBITORS FOR THE PREVENTION AND TREATMENT OF LONG COVID SYNDROME**

(57) The present invention relates to MEK inhibitors for use in a method of treatment or prevention of a disease caused by a coronavirus in a human subject, wherein the disease comprises a long COVID syndrome. Also provided are compositions comprising such inhibitors for use in the treatment of a corona virus infection, such as COVID-19.

EP 4 186 500 A1

## Description

### FIELD OF THE INVENTION

The present invention relates to the use of MEK-inhibitors for the treatment or prevention of coronavirus infections and/or the treatment or prevention of long COVID Syndrome.

### BACKGROUND

The Coronavirus group consists of enveloped positive stranded RNA viruses belonging to the family *Coronaviridae* and comprise subtypes referred to as Alpha-, Beta-, Gamma- and Delta coronavirus. Alpha and Beta affect mammals, while Gamma affects birds and Delta can affect both. The coronavirus family comprises several well-known disease-causing members. The Betacoronavirus family, has so far posed the biggest risk to humans and now includes the most well-known virus targets including Severe Acute Respiratory Syndrome coronavirus (SARS-CoV) responsible for the deaths of 774 people in 2003, Middle East Respiratory Syndrome coronavirus (MERS-CoV), believed to have killed 858 people in 2012, and the newest form to emerge, the novel coronavirus, (SARS-CoV-2) which as of November 2021 has caused over 5.000.000 deaths, reported to the World Health Organization (WHO)worldwide. In all, coronaviruses represent a continued and ever evolving threat to human life.

While the different subtypes of coronaviruses can infect different host types, the overall biochemical structure of the virus is similar. Coronaviruses undergo frequent recombination. As mentioned above, SARS-CoV-2 is a member of the subgenus *Sarbecovirus* (beta-CoV lineage B). Its RNA sequence is approximately 30,000 bases in length, relatively long for a coronavirus. Its genome consists nearly entirely of protein-coding sequences, a trait shared with other coronaviruses. SARS-CoV-2 is unique among known betacoronaviruses in its incorporation of a polybasic site cleaved by furin, a characteristic known to increase pathogenicity and transmissibility in other viruses. By 12 January 2020, five genomes of SARS-CoV-2 had been isolated from Wuhan and reported by the Chinese Center for Disease Control and Prevention (CCDC) and other institutions; the number of genomes increased to 42 by 30 January 2020. A phylogenetic analysis of those samples showed they were "highly related with at most seven mutations relative to a common ancestor", implying that the first human infection occurred in November or December 2019. As of 7 May 2020, 4,690 SARS-CoV-2 genomes sampled on six continents were publicly available. The first SARS-CoV-2 genome sequenced from the isolate Wuhan-Hu-1 has the NCBI accession number 86693 and is considered to represent the wild-type SARS-CoV-2. In July 2020, scientists reported that a more infectious SARS-CoV-2 variant with spike protein variant G614 had replaced D614 as the dominant form in the pandemic. There are many thousands of variants of SARS-CoV-2, several of which are considered to be increasingly dominant in different regions, such as the British variant B.1.1.7, the South African variant B.1.351, the Brazilian variants P.1 and P.2 or the Indian variant B.1.617.

Despite the current attempts of developing vaccines against a human-pathogenic coronavirus infection and specifically against SARS-CoV-2, there remains a need to provide alternative or improved compositions and methods for treatment and/or prevention of diseases caused by human-pathogenic coronaviruses, especially in light of the emerging virus variants.

The COVID-19 pandemic in 2019/2021 caused by SARS-CoV-2 clearly demonstrates that coronaviruses have a strong impact on global health systems. No preventative vaccination was available, and only one antiviral drug, Remdesivir, was provisionally approved for COVID-19 but is now considered to be only moderately effective. As of May 2021, several vaccines were developed and available starting end of 2020. It is expected that a certain percentage of the population worldwide will be vaccinated by the end of 2021, however it remains unclear to what extent the vaccinations approved in 2021 are effective against current and emerging SARS-CoV-2 variants, such as the British variant B.1.1.7, the South African variant B.1.351, the Brazilian variants P.1 and P.2 or the Indian variant B.1.617. Thus, it is likely that SARS-CoV-2 variants will continue to emerge and cause COVID-19. While various attempts have been made to treat COVID-19 with available drugs, so far none has been very successful. This highlights the urgent need for additional effective antivirals to better control the infections of coronaviruses in general and SARS-CoV-2 in particular. Notably, in the early phase of a pandemic, when no vaccine is available, antivirals are the stand-alone treatment. The same applies to virus variants where the vaccines are not effective. According to current knowledge, all vaccines that are currently available protect very well against serious illness if they are fully vaccinated. An incomplete series of vaccinations (one of two doses) showed a reduced protective effect against mild disease courses with Delta compared to Alpha. A relevant part of the population - including all children under the age of 12 for whom no vaccine has yet been approved in some countries, such as Germany (as of November 2021) - does not yet have any immune protection against the virus.

In the case of COVID-19, the clinical spectrum of SARS-CoV-2 infection appears to be wide, encompassing asymptomatic infection, mild upper respiratory tract illness, and severe viral pneumonia with respiratory failure and even death, with many patients being hospitalized. The use of a 3-stage classification system is widely accepted, recognizing that COVID-19 illness exhibits three grades of increasing severity which correspond with distinct clinical findings, response

to therapy and clinical outcome. Despite high numbers of infections with the original Sars-CoV-2 virus, 80% of all positively tested patients experienced mild symptoms only, 20% showed signs of hypoxemia leading to hospitalization and only 5% required treatment in intensive care units (ICU). However, these statistics do not necessarily apply to the emerging virus variants, in particular the Indian variant seems to lead to higher numbers of symptomatic patients with severe hypoxemia requiring hospitalization and intensive care. To treat COVID-19, it is important to understand the stages of the infection. These stages are summarized in Hasan et al., entitled "COVID-19 Illness in Native and Immunosuppressed States: A Clinical-Therapeutic Staging Proposal" and summarized below.

The initial stage, termed Stage I, is a mild infection and occurs at the time of inoculation and early establishment of disease. For most people, this involves an incubation period associated with mild and often non-specific symptoms for some days such as malaise, fever, and a dry cough. In patients who can keep the virus limited to this stage of COVID-19, prognosis and recovery is excellent. Treatment at this stage is primarily targeted towards symptomatic relief. Should an antiviral therapy be proven beneficial, targeting selected patients during this stage may reduce duration of symptoms, minimize contagiousness, and prevent progression of severity. In addition, an early-acting antiviral would be useful to prevent progression of the disease in asymptomatic or Stage I COVID-19 patients. Due to the increase in general testing, more asymptomatic patients are identified that could benefit from a preventive treatment to avoid disease progression. Currently, no treatment for asymptomatic or Stage I COVID-19 patients is available. Additionally, no prophylactic or preventive treatment is known that could be used in cases where there has been close contact to a person known to SARS-CoV-2 positive.

In the second stage, termed Stage II, of an established pulmonary disease, viral multiplication and localized inflammation in the lung is the norm. Stage II includes pulmonary involvement, termed Stage IIa, without and Stage IIb with hypoxia. During this stage, patients develop a viral pneumonia, with cough, fever and possibly hypoxia. Over the course of the disease, dyspnea occurs after a median of 13 days after the first onset of symptoms (range 9-16.5 days). Dyspnea is a sign of serious disease of the airway, lungs, or heart and is characterized by difficult or labored breathing and shortness of breath. In the case of COVID-19, imaging with chest X-ray or computerized tomography reveals bilateral infiltrates or ground glass opacities. It is at this stage that most patients with COVID-19 need to be hospitalized for close observation and management. Treatment primarily consists of supportive measures and antiviral therapies, once available. It is possible that patients will nevertheless progress to Stage III to require mechanical ventilation in an intensive care unit (ICU).

In Stage II COVID-19, markers of systemic inflammation may be elevated, but not remarkably so. In early studies performed on the first group of patients in Wuhan, China, it was found that upon entry into the hospital, plasma IL1beta, IL1Ralpha, IL7, IL8, IL9, IL10, basic FGF, GCSF, GMCSF, IFNgamma, IP10, MCP1, MIP1$\alpha$, MIP1$\beta$, PDGF, TNF$\alpha$, and VEGF concentrations were higher than in healthy adults. In addition, it was found that patients in ICU had plasma concentrations of IL2, IL7, IL10, GCSF, IP10, MCP1, MIP1$\alpha$, and TNF$\alpha$ were higher than in patients upon admission to the hospital (Huang et al.; The Lancet; Vol 395; pp.: 497-506 February 15, 2020), indicating that an increase in these cytokines marks the transition from COVID-19 stage II to stage III.

A minority of COVID-19 patients will transition into the third and most severe stage of illness, termed Stage III, which manifests as an extra-pulmonary systemic hyperinflammation syndrome. In this stage, markers of systemic inflammation are elevated. Overall, the prognosis and recovery from this critical stage of illness is poor.

In addition to the above-described stages of the COVID-19 infection, symptoms have been described which are summarized as long COVID or post-COVID19 syndromes. Further nomenclature for long COVID include also terms such as post-acute sequelae of COVID-19 (PASC), or chronic COVID syndrome (CCS). Other proposed descriptions of long COVID are listed for example in Table 1 in the review article of Shin Jie Yong "Long COVID or post-COVID-19 syndrome: putative pathophysiology, risk factors, and treatments", INFECTIOUS DISEASES, 2021; VOL. 0, NO. 0, 1-18. The number of patients suffering from Long COVID can hardly be estimated. There are several studies, such as a study of Great Britain reporting that 20% have symptoms four weeks after infection (Greenhalgh, T., Knight, M., Court, C., Buxton, M., & Husain, L. (2020). Management of post-acute Covid-19 in primary care. BMJ, 370:m3026. DOI: 10.1136/bmj.m3026), a meta-analysis concerning Long COVID is showing that 80% of infected patients have long-term symptoms (Lopez-Leon, S., Wegman-Ostrosky, T., Perelman, C., Sepulveda, R., Rebolledo, P.A., Cuapio, A., & Villapol, S. (2021). More than 50 long-term effects of Covid-19: a systematic review and meta-analysis. medRxiv, 2021.01.27.21250617. DOI: https://doi.org/10.1101/2021.01.27.21250617), a Lancet study shows that 76% of COVID-19 patients have symptoms even after 6 months (Huang, C., Huang, L., Wang, Y., Li, X., Ren, L., Gu, X.,& Cao, B. (2021). 6-month consequences of Covid-19 patients discharged from hospital: a cohort study. Lancet, 397, 220-232. DOI: doi.org/10.1016/ S0140-6736(20)32656-8), an Irish Long COVID study discloses that 60% of COVID-19 patients have long-term effects after 75 days between diagnosis and follow-up examination (Townsend, L., Dowds, J., O'Brien, K., Sheill, G., Dyer, A., O'Kelly, B., ... & Bannan, C. (2021). Persistent poor health post-Covid-19 is not associated with respiratory complications or initial severity. AnnalsATS. DOI: https://doi.org/10.1513/AnnalsATS.202009-1175OC), and a study from Bangladesh repots post-COVID-19 syndrome at 46% of the patients (Mahmud, R., Rahman, M., Rassel, M., Monayem, F., Sayeed, S., Islam, M. S., & Islam, M. M. (2021). Post-Covid-19 syndrom among symptomatic Covid-19 patients. A prospective cohort study in a tertiary care center of Bangladesh. PLOS ONE, 16(4). DOI: https://doi.org/10.1371/journal.pone.0249644).

Long COVID affects COVID-19 survivors at all levels of disease severity, including children, younger children, and persons that have not been hospitalized. The World Health Organization (WHO) estimates that 10% of COVID-19 survivors - including both hospitalized and non-hospitalized individuals - have persistent problems 12 weeks after infection (Asher Mullard, "Long COVID's long R&D agenda, NATURE REVIEWS, DRUG DISCOVERY, Volume 20, May 2021, 329-331).

The symptoms are quite diverse affect nearly every organ system. The most common symptoms are fatigue and dyspnoea, other symptoms described for long COVID comprise cognitive and mental impairments, chest and joint pains, smell and taste dysfunctions, cough, headache, and gastrointestinal and cardiac issues.

The reasons for these diverse long COVID symptoms are discussed only to a limit extent so far. Long COVID may be driven by long-term tissue damage, for example lung, brain, and heart, and pathophysiological inflammation, for example due to viral persistence, immune dysregulation, and autoimmunity (Shin Jie Yong "Long COVID or post-COVID-19 syndrome: putative pathophysiology, rsik factors, and treatments", INFECTIOUS DISEASES, 2021; VOL. 0, NO. 0, 1-18).

Therefore, there is a need for compounds that can prevent progression of COVID-19 and reduce severity of the COVID-19 infection and mortality. There is also a need for compounds that can prevent or treat long COVID syndromes. Several multinational studies are ongoing focusing on compositions that have already been approved or undergone clinical studies for a different disease. Most of the compounds being considered for the treatment of COVID-19 belong to one of the two groups:

1. Antiviral medicines that were originally developed against HIV, Ebola, Hepatitis C, or Influenza. The idea is that these block the reproduction of the virus or prevent entry into the lung cells. Among others, this includes old malaria medicines that are known to be effective against viruses. These antivirals may be effective in stage I and early stage II of COVID-19 as they act early in the viral lifecycle.
2. Suppressive immunomodulators that were developed to treat rheumatoid arthritis or inflammatory bowel disease. The idea is that these would restrict the immune system so that these do not cause more damage than the virus itself. These could be effective in late Stage II and Stage III COVID-19 infection, as they suppress cytokine production.

Several multinational studies were started in 2020 to test the efficacy of Remdesivir (RNA polymerase inhibitor), Ritonavir or Lopinavir (HIV medication), Beta Interferons, and/or Chloroquine or Hydroxychloroquine (Malaria medication). However, none of these was found to be particularly effective to date, so there remains the urgent need for an effective treatment alternative for COVID-19 patients.

In early May 2020, an emergency authorization was provided by the US FDA for the use of the antiviral Remdesivir in the treatment of hospitalized COVID-19 patients. Remdesivir is an RNA polymerase inhibitor that was originally developed for the treatment of Ebola, where it was found to be ineffective. Remdesivir was only approved to treat patients in a hospital setting showing severe symptoms which we would classify as stage III COVID-19. However, while Remdesivir was found to decrease the length of hospitalization of the patients in trials, there was no significant effect on mortality.

A COVID-19 Long-Haulers Study, a phase 2 study, has been started in March 2021 to evaluate the safety and efficacy of leronlimab (PRO 140) in patients with prolonged symptoms caused by COVID-19 (https://clinicaltrials.gov/ct2/show/NCT04678830). Leronlimab is a humanized IgG4 monoclonal antibody (mAb) to the C-C chemokine receptor type 5 (CCR5). Previously, Leronlimab has been used in the therapy of HIV infections.

In late 2021, the biotechnology company Axcella plans to initiate a randomized, double-blind, placebo-controlled Phase 2a Clinical Trial to evaluate the efficacy and safety of AXA1125 (AXA1125 is an EMM (endogenous metabolic modulator) composition of six amino acids and derivatives in patients with exertional fatigue related to Long COVID. Approximately 40 patients will be enrolled and randomized evenly to receive either 67.8 grams per day of AXA1125 or a matched placebo in two divided doses for 28 days, with a one-week safety follow-up period (https://axcellatx.com/pipeline/axa1125/). In this context, it is described that mitochondrial dysfunction is increasingly being implicated as a key driver of Long COVID-induced fatigue, as the most common symptom associated with this condition. AXA1125 is described to be able to reverse mitochondrial dysfunction and improve energetic efficiency via increased fatty acid oxidation, restored cellular homeostasis, and reduced inflammation.

In view of the prior art and the multitude of ongoing studies, it is clear that there is the need of new compounds and compositions effective in the treatment and prevention of diseases caused by Coronavirus infections, particular in respiratory tract diseases such as COVID-19 and Long COVID syndromes. In particular, there is a need for medication to prevent early infection with Coronaviruses, specifically SARS-CoV-2, and to treat patients in Stage II and Stage III COVID-19, to prevent disease progression and to decrease mortality, and there is a need for prevention and therapy of Long-COVID syndromes.

## SUMMARY OF THE INVENTION

The solution to the above problem provided herein is the provision of MEK inhibitors for the treatment of coronavirus infections.

In particular, in a first aspect, the solution to the above problem is the provision of a MEK inhibitor for use in a method of treatment or prevention of a disease caused by a coronavirus in a human subject, wherein the disease comprises a long COVID syndrome.

MEK inhibitors were found to be useful in the treatment of Stage II COVID-19, as they both prevent viral propagation and exit from the host cells by blocking MEK kinase and also decrease the immune responses that ultimately lead to Stage III of COVID-19. Due to this dual efficacy, MEK inhibitors, and specifically ATR-002, also termed PD-0184264 or zapnometinib, are particularly promising for the treatment or prevention of long COVID. Accordingly, the dual efficacy of the MEK inhibitor of the present invention provides reduced inflammation, reduced tissue damage and reduced viral load, which are important effects for the prevention and treatment of long COVID syndromes.

A second aspect the invention relates to a MEK inhibitor for use in treating or preventing long COVID syndrome associated with COVID-19 in a subject infected by SARS-CoV-2 by improving disease progression in the subject. MEK inhibitors were found to be useful in the treatment of long COVID syndromes, since the MEK inhibitors are able to improve T cell mediated immune response, such that the clonal expansion of T cells is maintained or enhanced, and IFN-gamma producing T cells are enhanced.

MEK inhibitors are already known originally as chemotherapeutics and are being developed by the inventors for use in treating or preventing viral infections, specifically influenzavirus and hantavirus. ATR-002, also termed PD-0184264 or zapnometinib, is a metabolite of CI-1040 described first in the context of chemotherapeutics in 2004 (Wabnitz et al.) that is being developed by the inventors for use in the treatment or prevention of viral diseases, such as coronavirus infection. ATR-002 has the chemical structure shown below:

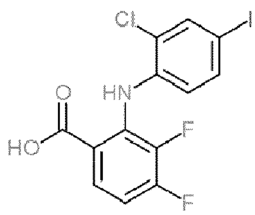

.

PD-0184264 is one of several metabolites of CI-1040 (Wabnitz et al., 2004, LoRusso et al., 2005). However, the mechanisms studied on PD-0184264 regarding the role of MEK in coronavirus infections apply equally to other MEK inhibitors.

For this reason, the present invention also discloses a MEK inhibitor for use in a method of treatment of a disease caused by a coronavirus in a human subject. The human subject may be hospitalized or not. In some aspects, the disease is an acute respiratory disease, such as those caused by SARS-CoV-1, SARS-CoV-2 or MERS. In the context of the invention, the coronavirus can be SARS-CoV-2 and the corresponding subject to be treated is suffering from COVID-19. The use of the MEK inhibitor of the invention is particularly useful when the COVID-19 symptoms are developing after infection and/or when the COVID-19 symptoms comprise long COVID syndromes.

As mentioned above, the MEK inhibitor can be selected from the group consisting of PD-0184264, CI-1040, GSK-1120212, GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof. Preferably, the MEK inhibitor is PD-0184264 or a pharmaceutically acceptable salt thereof.

In a preferred dosage form, the PD-0184264 or pharmaceutically acceptable salt thereof is administered to the human subject once daily in a dose of 100 to 1000 mg, preferably 300 to 900 mg, most preferably 300, 600 or 900 mg.

PD-0184264 is used to treat a human subject suffering from COVID-19 caused by SARS-CoV-2, in particular a human subject suffering from long COVID syndrome. The COVID-19 is at any stage associated with long COVID syndrome or post -COVID-19 syndrome or any syndromes which are involved at a later stage after SARS-CoV-2 infection. The PD-0184264 can be administered to the human subject on 1 to 28 consecutive days, preferably 5 to 18 or 7 to 14 consecutive days, starting with onset of clinical symptoms. Preferably, the PD-0184264 is administered to the human subject in an oral dosage form.

In a preferred embodiment, the MEK inhibitor of the invention is for use according to the invention, wherein the coronavirus is resistant to previous antiviral treatment, wherein the previous antiviral treatment is preferably administration of Remdesivir. In addition, the MEK inhibitor can be used to treat the human subject that is over 60 years of age or belongs to a high risk group for coronavirus infection.

The invention is further directed to the use of a MEK inhibitor in treating or preventing a COVID-19 cytokine

storm in a subject infected by a human coronavirus. The MEK inhibitor is preferably selected from the group consisting of PD-0184264, CI-1040, GSK-1120212, GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof.

The use of a MEK inhibitor in treating or preventing a COVID-19 cytokine storm in a subject may comprise reducing the level of IL-1β and/or TNF-α in the subject, preferably reducing the level of one or more, two or more, three or more, four or more, five or more or all six of TNF- a, IL-1β, IP-10, IL-8, IL-6, MCP-1, MIP-1α and MIP-1β in a subject.

In line with the above, the invention is also directed to a method of treating or preventing a COVID-19 cytokine storm in a subject suffering from a SARS-CoV-2 infection, wherein the method comprises administering to the subject a MEK inhibitor. This method may comprise reducing the level of IL-1β and/or TNF-α in the (blood or plasma) of the subject, preferably reducing the level of one or more, two or more, three or more, four or more, five or more or all six or TNF- a, IL-1β, IP-10, IL-6, IL-8, MCP-1, MIP-1α and MIP-1β in (the blood or plasma) of the subject.

The SARS-CoV-2 to be treated may either be the original wild-type strain and/or one or more variants. Examples of variants of SARS-CoV-2 that can be treated include, but are not limited to, the variants D614G, B.1.351, B.1.1.7, P1, P2, B.1.617, B.1.427, B.1.429, B.1.525 and B.1.526.

The invention also relates to a MEK inhibitor used in preventing development of symptoms caused by a human coronavirus infection in an asymptomatic subject infected with a human coronavirus or for preventing a human coronavirus infection in a subject who has been in close contact with a person infected with a human coronavirus, wherein the MEK inhibitor is preferably selected from the group consisting of PD-0184264, CI-1040, GSK-1120212, GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof. Again, the human coronavirus can be SARS-CoV, SARS-CoV-2 or MERS or a SARS-CoV-2 variant, preferably selected from the group consisting of D614G, B.1.351, B.1.1.7, P1, P2, B.1.617, B.1.427, B.1.429, B.1.525 or B.1.526.

Finally, a third aspect of the invention relates to a pharmaceutical composition comprising the MEK inhibitor for the use and medical treatments described.

## BRIEF DESCRIPTION OF THE FIGURES

**Figure 1: Fig. 1(A)** shows a lane view of Western Blot Analysis of SARS-CoV-2 infected and ATR-002 treated CaCo-2 cells

pERK1/2, ERK1/2 and Nucleocapsid protein were detected in Caco2 cells infected with SARS-CoV-2 treated with ATR-002. Uninfected cells (mock) were used as control.

**Fig.1 (B)-(D)** show a comparison of ERK phosphorylation and correlation of Nucleocapsid protein of SARS-CoV-2 infected Caco-2 cells, treated with ATR-002

Fig. 1(B) shows ERK-phosphorylation. The area under the peak was used to normalize pERK1/2 to ERK1/2 and to calculate the values of %ERK-phosphorylation.

Fig. 1(C) shows nucleoside protein concentration of Caco-2 cells treated with different concentrations of ATR-002 compared to DMSO control.

Fig. 1(D) shows virus titer (PFU/mL) of Caco-2 cells treated with different concentrations of ATR-002 compared to DMSO control.

**Figure 2**: (A) shows infection of Vero cells with SARS-CoV-2 upon MEK inhibition.
**(B)** shows detection of the viral gene RdRP during infection with SARS-CoV-2 upon MEK inhibition.

**Figure 3:** The replication ability of SARS-CoV-2 in Calu-3 cells and the possible ERK activation during the SARS-CoV-2

**Figure 4:** ERK knockdown leads to decreased production of progeny viral titers

**Figure 5:** ATR-002 treatment is effective against SARS-CoV-2 in different host cell systems

**Figure 6:** Inhibition of MEK by ATR-002 results in decreased viral titers

**Figure 7:** ATR-002 treatment is effective against the South African variant of SARS-CoV-2

**Figure 8:** ATR-002 reduces cytokine & chemokine gene expression in an acute lung injury (ALI) mouse model.

**Figure 9:** ATR-002 reduces the pro-inflammatory cytokine/chemokine response after SARS-CoV-2 infection of CaCo2 cells.

**Figure 10:** Inhibition of MEK by ATR-002 results in a decreased expression of pro-inflammatory cytokines

**Figure 11:** MEK inhibition by ATR-002 does not affect the IFN response but reduces proinflammatory IL-8 expression after poly (I:C) stimulation

**Figure 12:** ATR-002 reduces the pro-inflammatory cytokine/chemokine response in PMBC cells.

**Figure 13:** schematic representation of modulation of the overwhelming cytokine response after ATR-002 treatment.

**Figure 14:** ATR-002 was found to show significant, dose dependent inhibition of cytokine/chemokine stimulation with Anti-CD3 stimulation, ConA stimulation and PHA stimulation

**Figure 15:** The stages of COVID-19 according to Hasan et al.

**Figure 16:** Efficacy of ATR-002 against SARS-CoV-2 in a hamster infection model. (A-E) Individual data and median values are presented. (F) Weight loss at day 4 p.i. in percentage, compared to the body weight at the time of infection. Data were analyzed with the one-way ANOVA multiple comparison test. P values are presented.

**Figure 17:** ATR-002 blocks production of progeny virus particles in air-liquid-interface cultures.

**Figure 18:** ERK activation during SARS-CoV-2 infection depends on the expression of hACE2

**Figure 19:** ACE2 reduction post ATR-002 treatment in Calu-3 cells

**Figure 20:** ATR-002 can block replication of SARS-CoV-2 in ACE2 overexpressing cells

**Figure 21 and 22:** MEK-inhibitors can prevent infection of pseudotyped VSV carrying SARS-CoV-2 spike protein after pre-incubation

**Figure 23:** Histopathologic changes in hamsters at 4 days post infection, intranasally infected with SARS-CoV-2 and treated with zapnometinib at days 1, 2, and 3 post infection, or treated with the therapeutic vehicle only

**Figure 24:** Spot counts from three different donors evaluated in an IFN-y Fluorospot assay after a 12-day T cell reactivation period (clonal expansion) with CEF peptide stimulation. Application of ATR-002 (or DMSO solvent control) was tested with 5 or 10 pg/mL on three different time points followed by induction of clonal expansion with IL-2 24 hours after each treatment.

## DETAILED DESCRIPTION

The following description includes information that may be useful in understanding the present invention. It is not an admission that any of the information provided herein is prior art or relevant to the presently claimed inventions, or that any publication specifically or implicitly referenced is prior art.

The above being said, a first aspect of the present invention relates to MEK inhibitors for use in a method of treatment or prevention of a disease caused by a coronavirus in a human subject, wherein the disease comprises a long COVID syndrome.

In a preferred embodiment of the invention, the coronavirus is SARS-CoV, SARS-CoV-2 or MERS.

Further preferred, the coronavirus is SARS-CoV-2 and the human subject is suffering from long COVID syndrome associated with COVID-19.

Preferably, the MEK inhibitor is selected from the group consisting of PD-0184264, CI-1040, GSK-1120212,

GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof.

In a preferred embodiment of the invention is the MEK inhibitor PD-0184264 or a pharmaceutically acceptable salt thereof, wherein PD-0184264 or pharmaceutically acceptable salt thereof is preferably administered to the human subject once daily in a dose of 100 to 1000 mg, preferably 300 to 900 mg, most preferably 300, 600 or 900 mg.

Preferably, the human subject is suffering from long COVID syndrome associated with COVID-19 caused by SARS-CoV-2.

Further preferred, the human subject is or is yet asymptomatic or has only mild symptoms of COVID-19.

In a preferred embodiment, the SARS-CoV-2 is a variant, preferably selected from the group consisting of D614G, B.1.1.7, B.1.351, P1, P2, B.1.617, B.1.427, B.1.429, B.1.525 or B.1.526.

In a further preferred embodiment of the invention, it is envisioned that the MEK inhibitor PD-0184264 is administered to the human subject after a period of about four weeks after an acute, symptomatic or asymptomatic coronavirus infection on 1 to 21 consecutive days, preferably 5 to 18 or 7 to 14 consecutive days.

Preferably, the MEK inhibitor PD-0184264 is administered to the human subject in an oral dosage form.

The oral dosage form may be a solid oral dosage forms, such as tablets, capsules, sachets, powders, granules, orally dispersible films, or pills. The oral dosage form may be a liquid dosage form and semi solids, such as syrups, solutions, ampoules, dispersions, semi-solids, or softgels.

In a further preferred embodiment, the coronavirus is resistant to previous antiviral treatment, wherein the previous antiviral treatment is preferably administration of Remdesivir.

Preferably, the human subject is suffering from a long COVID syndrome comprising any one or more of the following symptoms fatigue, headaches, lung damage, shortness of breath, cough, inflammatory reactions, neurological disorders, heart palpitations, vertigo, anosmia (loss of smell), parosmia (distorted smell), insomnia, muscle weakness, low fever and/or cognitive dysfunction.

Long COVID syndrome is not clearly defined so far concerning the point in time of onset and duration of the symptoms. In some attempts, long COVID has been identified with the presence of at least one persistent symptom that includes fatigue or dyspnoea for at least three months after symptom onset, hospital admission, or diagnosis. Further, since acute COVID-19 may last for a few weeks before hospital discharge, symptoms lasting for at least two months after discharge is also considered as long COVID (Shin Jie Yong "Long COVID or post-COVID-19 syndrome: putative pathophysiology, risk factors, and treatments", INFECTIOUS DISEASES, 2021; VOL. 0, NO. 0, 1-18). The symptoms of long COVID are quite variable. The main symptoms comprise fatigue and dyspnoea. Further symptoms for long COVID are described and are also encompassed in the present invention which comprise long-term tissue damage and unresolved inflammation. Concerning long-term tissue damage, the fields of cardiology, pulmonary, and neurology are affected, which results in symptoms in the field of cardiology of chest pain, palpitations, tachycardia, and orthostatic intolerance; in the field of pulmonary of dyspnoea and cough; and in the field of neurology of cognitive impairments, headache, depression, anxiety, insomnia, smell and taste alteration, and fatigue. Concerning unresolved inflammation, long COVID symptoms have been described to comprise viral persistence, lymphopenia - which results in chronic inflammation, further causing myalgia, fatigue, joint pain -, gut dysbiosis - which results in gastrointestinal and neurological symptoms -, autoimmunity - which results in fatigue, joint pian, headache, cognitive impairments, orthostatic intolerance (Shin Jie Yong "Long COVID or post-COVID-19 syndrome: putative pathophysiology, risk factors, and treatments", INFECTIOUS DISEASES, 2021; VOL. 0, NO. 0, 1-18).

In accordance with the present invention, it is envisioned that the human subject suffering from a long COVID syndrome is preferably a subject which is infected by COVID-19 and reacts to the infection with the adaptive immune response. Thus, a subject suffering from long COVID may be a subject which is in the course of infection after the begin of the adaptive immune response.

A second aspect of the invention relates to a MEK inhibitor for use in treating or preventing long COVID syndrome associated with COVID-19 in a subject infected by SARS-CoV-2 by improving disease progression in the subject.

In a preferred embodiment, wherein improving disease progression comprises one or more, two or more, three or more, four or more, five or more, six or more, or all seven of the following reducing inflammation, reducing tissue damage, achieving immune modulation, reducing viral persistence, improving T cell responses, enhancing clonal expansion of T cells and enhancing IFN-gamma producing T cells.

In a preferred embodiment, the clonal expansion of T cells is enhanced. The inventors could surprisingly show that T cell response is improved in subjects which have received the MEK inhibitor according to the present invention compared to subjects not treated with the MEK inhibitor. This is in detail shown in **Example 11** and **Figure 24.** In the context of the present invention, the enhancement of the clonal expansion of the T cells may be understood such that the number of T cells of a subject treated with the MEK inhibitor of the present invention is maintained or elevated compared to a subject not treated with the MEK inhibitor.

Generally, it is known that the inhibition of the Raf/MEK/ERK signaling pathway leads to the inhibition of cell

proliferation. At the beginning of the immune response, clonal expansion of T cells starts, which clonal expansion also represents cell proliferation. However, said clonal expansion is independent from the Raf/MEK/ERK signaling pathway - as could be shown in the above-mentioned example. Actually, the example shows that the inhibition of MEK with the MEK inhibitor according to the present invention does not lead to the inhibition of the clonal expansion of the T cells. Accordingly, it could be shown that the MEK inhibitor of the present invention does not inhibit the immune response of T cells, which is of importance for the treatment of the SARS-CoV-2 infection and the clearance of persisting SARS-CoV-2 viruses. It is believed that long COVID syndromes may be caused by persisting viruses which cannot be completely cleared after a severe SARS-CoV-2 infection. In view of this, the positive influence of the MEK inhibitor of the present invention on clonal expansion of T cells provides an improved prevention or treatment of long COVID syndromes.

Preferably, the MEK inhibitor is also for use in treating or preventing a COVID-19 cytokine storm in a subject infected by SARS-CoV-2, wherein the MEK inhibitor is preferably selected from the group consisting of PD-0184264, CI-1040, GSK-1120212, GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof.

Therefore, the MEK inhibitor of the present invention is able to reduce excessive immune responses by inhibition of cytokine storm, and inhibition of viral replication without reducing natural immune response mediated by T cells of the human body. Accordingly, the MEK inhibitor of the present invention has the potential to reduce the severity of the COVID-19 course of infection and simultaneously to improve the immune response of the body against the infection, which may lead to the prevention or treatment of long COVID syndromes.

Preferably, IFN-gamma producing T cells are enhanced. In **Example 11** and **Figure 24,** it could be shown that in particular IFN-gamma producing T cells are enhanced in samples from subjects treated with the MEK inhibitor of the present invention compared to untreated control samples. Interferon gamma is known to be a key moderator of cell-mediated immunity with diverse, mainly pro-inflammatory actions on immunocytes and target tissue. It has been shown that IFN gamma may enhance anti-tumor and antiviral effects of CD8 T cells. Therefore, the enhancement of IFN-gamma producing T cells due to the MEK inhibitor of the present invention provides an improved immune response to allow prevention or treatment of long COVID syndromes.

In a preferred embodiment, the MEK inhibitor is preferably selected from the group consisting of PD-0184264, CI-1040, GSK-1120212, GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof.

Preferably, the SARS-CoV-2 is a variant, preferably selected from the group consisting of D614G, B.1.351, B.1.1.7, P1, P2, B.1.617, B.1.427, B.1.429, B.1.525 and B.1.526.

A third aspect of the invention relates to a pharmaceutical composition comprising the MEK inhibitor for the use of any one of herein described uses of the MEK inhibitors.

As shown in the further examples, the inventors showed that surprisingly MEK inhibitors have a dual effect in treating diseases such as COVID-19 caused by coronaviruses. The present invention thus also relates to treating or preventing COVID-19 cytokine storm in patients being infected with SARS-CoV-2. Furthermore, it was found that MEK inhibitors are effective in blocking expression of ACE-2 receptors and could therefore be useful in preventing development of symptoms caused by a human coronavirus infection in an asymptomatic subject infected with a human coronavirus or for preventing a human coronavirus infection in a subject who has been in close contact with a person infected with a human coronavirus. In this context, "close contact" is defined generally as having spent 10 minutes or more within a distance of less than 1.5 meters, either outside or in a closed room with a person that has been tested positive. The German Robert Koch Institute (RKI) as of May 19, 2021 defines persons having close contact with a person known to be infected with a human coronavirus as having an increased risk of infection if they have had:

1. Close contact (less than 1,5 meters) for more than 10 minutes without appropriate protection such as an FFP2 mask,
2. Face-to-Face conversation (less than 1,5 meters) independent of the length of the conversation without appropriate protection such as an FFP2 mask or with direct contact with respiratory secretions (coughing, sneezing), and/or
3. Simultaneous presence of contact persons and infectious persons in the same room with likely high concentration of infectious aerosols independent of distance for more than 10 minutes even if appropriate protection is worn.

Examples for constellations with close contact are

- persons from the same household,
- persons with direct contact to secretions or body fluids of an infected person, specifically to respiratory secretions such as through kissing, coughing, sneezing etc.,
- persons who have been in a room with infectious aerosols without adequate air circulation (such as through singing, sports, parties, meetings, school classes, religious ceremonies),

- persons who were exposed to an infected person on an airplane, independent of wearing an FFP-2 mask and persons that were sitting in the same row or in the two rows in front of or behind an infected person on an airplane, crew members or other passengers if there was a close contact, see point 2 above.

It is of course possible that the definition of "close contact" may change depending on the virus variant. It has been shown that some virus variants have higher transmittal rates which could affect the distance and time necessary for infection. For this reason, an antiviral having both a preventative effect and an effect on viral propagation would be particularly useful in the context of SARS-CoV-2 emerging variants.

Specifically, as demonstrated in the appended Examples, ATR-002 shows a direct effect on viral propagation of SARS-CoV-2 as well as an immunomodulatory effect leading to a decreased cytokine release. This dual effect is surprising and makes MEK inhibitors particularly suited to the treatment of COVID-19 patients. As described in the background section above and defined further on, these patients are hospitalized but are not yet in intensive care. In these patients, MEK inhibition has the dual effect of preventing the cytokine storm that then leads to Stage III COVID-19 while simultaneously suppressing viral propagation to stop disease progression and decrease mortality. Further, the MEK inhibitor of the present invention is able to improve T cell response, in particular clonal expansion of T cells and enhancing of IFN gamma producing T cells. This allows prevention and treatment of long COVID syndromes in form of a positive influence on reduction of persistent viruses, long-term tissue damage - which is also demonstrated in **Example 8** below - and unresolved inflammation - which is shown in **Example 11** below.

In the first days after SARS-CoV-2 infection, extravasation of macrophages, neutrophils and NK cells into infected lung tissue is seen. COVID-19 is then caused by release of inflammatory mediators by infected lung epithelium, monocytes, dendritic cells and alveolar macrophages after infection with the SARS-CoV-2 virus. The production and secretion of cytokines and chemokines (e.g. TNF-$\alpha$, IL-1$\beta$, IP-10, IL6, IL-8, MCP-1, MIP-1$\beta$) results in local and systemic acute inflammation and alterations of the adaptive immune response, which is termed the "COVID-19 cytokine storm" (which is also interchangeably referred to as "cytokine storm" herein) and marks the transition from Stage II to Stage III COVID-19. The term "COVID-19 cytokine storm" is used herein within its regular meaning as used in the art (see, in this respect, Jamilloux et al, 2020) to mean a cytokine storm that may occur in a subject that has been infected with a human-pathogenic coronavirus, in particular SARS-CoV-2. From preliminary studies presented in the examples, it appears that MEK inhibitors in general and ATR-002 in particular would be able to simultaneously decrease the virus propagation and prevent the cytokine storm by suppressing cytokine release (cf. **Figure 13** in this respect). This dual effect was unexpected and is different from the other antivirals currently being studied for their useful in the treatment of COVID-19, such as Remdesivir. Further, it was unexpected that the T cell response in a subject is improved due to the MEK inhibitor of the present invention.

In light of the COVID-19 pandemic, the inventors were interested in determining whether SARS-CoV-2 uses the MEK pathway for viral propagation. For this purpose, it was necessary to first obtain a SARS-CoV-2 isolate (Isolate "FI") and then determine which available cells could be infected with the virus. MEK inhibitors in general and ATR-002 in particular target the host cell factor MEK, the dose-response relationship of ATR-002 does not depend on the virus, but to the inhibition of the kinase. In *in vitro* experiments the antiviral efficacy of ATR-002 strongly depends on the activation status of MEK in the cell line used for the experiment. CaCo2 Cell-lines used for SARS-CoV-2 investigations below show a high constitutive MEK-activity. As a comparison, experiments in Vero cells were also performed. The specific methods used can be found in **Example 1** and the results are shown in **Figures 1** and **2.** Specifically, **Figure 1** shows that when CaCo-2 cells infected SARS-CoV-2 (MOI 0.1) were treated with different concentrations of ATR-002, inhibition of phosphorylation of ERK as well as SARS-CoV-2 and the nucleocapsid protein, which is a viral marker, was seen at concentrations of 50 and 100 $\mu$M ATR-002. This high amount of ATR-002 necessary for inhibition does not relate to the virus titer but is rather due to the choice of CaCo2 cells, which have constitutively active MEK, so that high amounts are necessary for inhibitor of MEK. These experiments show that SARS-CoV-2 uses the MEK pathway for virus export. Additionally, **Figure 2A** shows that Vero cells infected with SARS-CoV-2 and treated with 50 $\mu$M ATR-002 have a lower viral load than control cells throughout the whole observation period and measurement of the SARS-CoV-2 viral gene RdRP shown in **Figure 2B** confirmed this finding. This indicates that the inhibitor not just caused a delay in replication but sustainably inhibits viral propagation. From **Experiment 1,** the inventors concluded that SARS-CoV-2 uses the MEK pathway for viral propagation.

In order to verify the effects seen in CaCo2 and Vero cells, further tests were performed on the human bronchioepithelial cell line Calu-3. First it was tested whether SARS-CoV-2 can infect Calu-3 cells. After this, it was tested whether and when ERK is activated by SARS CoV-2 in Calu-3 cells. Furthermore, it was tested whether ATR-002 was capable of inhibiting replication of SARS-CoV-2 in Calu-3 cells.

The experiments described in **Example 2** showed that Calu-3 cells can also be infected by the SARS-CoV-2 virus. Calu-3 is a human lung cancer cell line commonly used in lung cancer research and drug development for respiratory diseases. Calu-3 cells are epithelial and can act as respiratory models in preclinical applications. Calu-3 cells are commonly used as both *in vitro* and *in vivo* models for drug development against lung cancer. The cells have been used in

studies of pulmonary drug delivery, demonstrating a capacity to intake low molecular weight substances. Calu-3 cells have served as respiratory models for air intake and lung injury due to their responsiveness to foreign substances. In **Example 2,** it could be shown that Calu-3 cells can be infected with SARS-CoV-2. To investigate whether ATR-002 can inhibit SARS-CoV-2 in Calu-3 cells, the experiments performed previously on CaCo2 and Vero cells were repeated and ATR-002 is able to inhibit SARS-CoV-2 in Calu-3 cells in a dose-dependent manner. Replication of SARS-CoV-2 in Calu-3 cells and ERK activation in the SARS CoV-2 lifecycle as well as inhibition with ATR-002. As can be seen from **Figure 3,** the SARS-CoV-2 was able to replicate in the Calu-3 cells. Additionally, it was seen that SARS-CoV-2 infection leads to an ERK activation on the very early stage of the viral life cycle. ERK activation was observed 1 h.p.i. No additional ERK-activation in the later stages of the viral life cycle could be observed, indicating a possible role of the pathway activation for early process during the viral infection.

To verify that the antiviral action of ATR-002 during the SARS-CoV-2 life cycle is not an off-target effect, the Raf/MEK/ERK pathway was blocked by genetic means. Therefore, the expression of kinase ERK, which is the direct downstream target of MEK, was knocked down by specific siRNA in Calu-3 cells as described in Example 2B. It was found that the ERK-knockdown results in decreased production of progeny viral titers, as can be seen in **Figure 4.** Thus, it was shown that that a knockdown of ERK leads to a decrease in the expression of the spike protein and the production of progeny viral particles, confirming the role of MEK/ERK during the viral life cycle.

It was then tested whether a MEK inhibitor could decrease SARS-CoV-2 titers in Calu-3 cells in a comparable way to the decrease seen in CaCo2 and Vero cells in **Example 1.** Specifically, for the virus yield reduction assay 24-well plates with 95% confluent Caco-2, Vero E6 and Calu-3 cells were prepared. The wells were washed once with Infection medium prior infection with SARS-CoV-2 (MOI 0.01) for 1 hour in 200 μL infection medium per well. Afterwards the virus inoculum was removed completely, the wells were washed once with Infection medium and then treated with 1 mL per well with different concentrations of ATR-002 (100 μM, 75 μM, 50 μM, 0 μM) in infection medium with 1% DMSO for 24 hours at 37°C, 5% $CO_2$. Supernatants were harvested 24 hours post infection and centrifuged at max. speed, 5 minutes, 4°C to remove cell debris. Aliquots a 140 μL were prepared of the supernatants and stored at -80°C. The virus titer of the samples was determined via RT-qPCR (Real Time Quantitative Polymerase Chain Reaction). The probe used for detection of the viral RNA copies was directed against the SARS-CoV-2 N gene and results are shown. As can be seen from **Figure 5,** the effect seen on all cell types was comparable.

In a next step, Calu-3 cells were incubated with increasing amounts of ATR-002 over 24, 48 or 72 hours. As shown in **Figure 6,** a concentration dependent decrease in the production of progeny viral particles was observed in a non-toxic concentration range. This effect was maintained over the total infection time of 72 hours indicating that the pathway inhibition with ATR-002 has a longtime effect on the viral life cycle. Additionally, Figure 6 D is interesting as it shows that cells treated with ATR-002 did not show the characteristic cell damage of cells infected with SARS-CoV-2, indicating that ATR-002 prevents lung damage.

In order to verify that MEK inhibition is an effective treatment of human coronaviruses independent of the species or variant, the in vitro efficacy of ATR-002 against SARS-CoV-2 SA variant B.1351 was tested in **Example 3.** The same experimental setup as in Examples 1 and 2 was used to see whether ATR-002 was effective against the South African SARS-CoV-2 variant B.1351. Vero cells and Calu-3 cells were infected with SARS-CoV-2 SA at a MOI of 0.1, 1 and 10 ATR-002 as can be seen from **Figure 7.** These experiments showed that MEK inhibitors, specifically ATR-002, are capable of inhibiting coronaviruses, and specifically SARS-CoV-2 variants.

In a further step, the inventors reviewed data that had been gained previously in an acute lung injury (ALI) mouse model, which is presented as **Example 4.** Specifically, in the mouse ALI model, LPS induced cytokine & chemokine expression allows investigation of the immunomodulatory efficacy of ATR-002 in the absence of a virus infection. **Figure 9** shows that ATR-002 treatment of ALI mice leads to a decrease in the cytokines TNF-α, IL-1β, IP-10, IL-8, MCP-1, and MIP-1β. As discussed in the background section above, according to Huang et al., all of these cytokines are increased in COVID-19 patients, and TNF-α, IP-10 and MIP-1β are increased in Stage III COVID-19 patients as compared to the levels upon hospitalization with Stage II COVID-19. This indicates that, independent of SARS-CoV-2 infection, ATR-002 is able to reduce cytokine and chemokine gene expression in mammals.

In order to confirm that ATR-002 reduces the pro-inflammatory cytokine/chemokine response after SARS-CoV-2 infection, further in vitro experiments were performed in CaCo2 cells as described in **Example 5.** CaCo2 cells were infected with SARS-CoV-2 and treated with ATR-002. Amounts of MCP-1 were measured and results are shown in Figure 9. Specifically, it was found that ATR-002 is able to reduce MCP-1 expression in SARS-CoV-2 infected cells. However, a high amount of ATR-002 is necessary for this, as discussed in Example 1, this is due to the constitutive activation of the MEK pathway in CaCo2 cells. Due to somatic driver mutation, high amounts of ATR-002 are required to inhibit MCP-1 expression in SARS-CoV-2 infected CaCo-2 cells. We further addressed the question if ATR-002 can decrease the expression of pro-inflammatory cytokines in Calu-3 cells. Therefore, we infected Calu-3 cells with SARS-CoV-2 and treated them with increasing amounts of ATR-002 as described in **Example 2.** Expression of IL-6, IL-8, MCP-1, IP-10 and CCL5 mRNA was analyzed by quantitative real-time PCR. Results are depicted in **Figure 10** as n-fold mRNA expression of mock infected cells. The results confirm that ATR-002 can not only decrease the production of

progeny viral particles, as shown in the previous examples, but furthermore, reduce the expression of pro-inflammatory cytokines. This is an additional benefit of the ATR-002 treatment, which reduces the possibility of a cytokine storm during a viral infection. In a second step, to investigate a general effect of the ATR-002 treatment on the expression of anti-viral interferon and pro-inflammatory cytokines, A549 cells were transfected with polyI:C, a synthetic analog commonly used to mimic action of viral RNA, for 24 h. In parallel, cells were treated with increasing amounts of ATR-002. It was found that MEK inhibition by ATR-002 does not affect the type I IFN response but reduces proinflammatory IL-8 expression after poly (I:C) stimulation. Results of these experiments are shown in **Figure 11.** It was found that the ATR-002 treatment did not affect the stimulation of the anti-viral type I interferon system, as exemplified for the constant expression of the strictly type I IFN dependent gene MxA mRNA. In contrast, a strong reduction in the expression of pro-inflammatory IL8 was discovered, even with low concentrations of ATR-002.

In order to see whether this effect is also seen in other cell types, the effect of MEK inhibition was studied in LPS induced primary human Peripheral blood mononuclear cells (PMBC) as described in **Example 6**. Again, it was found that ATR-002 reduces the pro-inflammatory cytokine/chemokine response in PMBCs. Specifically, the amounts of IP10, TNF-$\alpha$ and MCP-1 expression were studied and as can be seen from **Figure 12,** all three were reduced after treatment with ATR-002, indicating that 10$\mu$g/ml ATR-002 is sufficient to inhibit >90% of LPS induced MCP-1 in human PBMCs. Further studies on PMBC confirmed that ATR-002 shows significant, dose dependent inhibition of cytokine/chemokine stimulation with Anti-CD3 stimulation, ConA stimulation and PHA stimulation, as can be seen in **Figure 14.**

Finally, due to the urgency of providing a treatment for the COVID-19 pandemic and in view of the promising data from **Examples** described herein as well as of positive data from a Phase I safety study of ATR-002, a Phase II study is in progress. This phase II study is described in **Example 7.** The study is termed **RESPIRE** - A **R**andomized, Double-Blind, Placebo-Controlled, Clinical Study to Evaluate the **S**afety and Efficacy of ATR-002 in Adult Hospitalized **P**atients with Moderate Coronavirus Disease (COVID-19). The primary objective of the study is to demonstrate the efficacy of ATR-002 compared to placebo, each on standard of care, in the treatment of patients with COVID-19 assessed by the clinical severity status at day 15. As determined by **Examples 1** to **6**, ATR-002 has the potential to treat COVID-19 due to its Mode of Action with a dual benefit (effect) being (1) an antiviral agent, and (2) immunomodulation (to interfere with the cytokine storm). This clinical study will enroll a total of 200 adult hospitalized patients suffering from moderate coronavirus disease Stage II. A nasopharyngeal swab will be taken immediately prior to randomization to confirm presence of SARS-CoV-2 thereafter.

All randomized patients will be receiving standard of care as per local standards. 100 Patients will be randomized to receive ATR-002 900 mg orally once daily, 100 patients will be receiving matching Placebo. ATR-002 or placebo will be supplied in a controlled double-blind fashion for 5 days.

The primary objective of the study is to evaluate the efficacy of ATR-002 relative to placebo measured by the clinical severity status on a 7-point ordinal scale [1] Not hospitalized, without limitations, [2] Not hospitalized, with limitations, [3] Hospitalized, not requiring supplemental oxygen, [4] Hospitalized, requiring supplemental oxygen, [5] Hospitalized, on non-invasive ventilation or high flow oxygen devices, [6] Hospitalized, on invasive mechanical ventilation or ECMO, [7] Death.

Secondary outcomes will be measured by clinical signs and symptoms, patient reported outcomes, Treatment Emergent Adverse Events, Serious Adverse Events, derived clinical parameters, scores and study events, changes in laboratory values and ATR-002 plasma levels, as well as changes in quantitative SARS-CoV-2 samples.

All patients will be followed up after end of study drug treatment until day 90 for safety reasons and to determine survival status.

The patients selected for the study are adults 18 years of age or older at screening and requiring hospitalization, showing clinical signs of a COVID-19 infection defined as having fever, defined as temperature $\geq$ 37.3 °C (axilla), $\geq$ 38.0 °C (oral), or $\geq$ 38.6 °C (rectal or tympanic) and $SpO_2$ <=94% on room air or requiring supplemental oxygen to maintain $SpO_2$>94%. Based on the antiviral and cytokine/chemokine studies shown in the Examples and further data from the Phase I clinical study, an inhibition of >50% - 90% of MEK activity is required to reduce viral load and cytokine/chemokine expression by more than 50%. In the intended RESPIRE study described above and in **Example 5,** patients suffering from Stage II COVID-19 will be treated.

Prior to the initiation of the RESPIRE clinical trial described in **Example 7,** experiments were performed on the Syrian Hamster model, which is the preferred model organism for studying SARS-CoV-2 infections as described in **Example 8.** The antiviral efficacy of the human equivalent dose of ATR-002 was tested in the hamster model after SARS-CoV-2 challenge. Animals were treated on the day of infection or 24 hours after infection, and parameters like the percentage of lung lesions and viral load in throat swabs and nasal turbinate tissue were analysed on day 3 - 4 after challenge. ATR-002 treatment resulted in a significant reduction of virus titer and lung lesions in a SARS-CoV-2 Syrian hamster infection model as can be seen in **Figure 16.** The chosen dosage of 100mg/kg for the first treatment followed by 75mg/kg once daily represents the hamster equivalent dose for a 900mg followed by 600mg human dose, which is used for the ATR-002 phase 2 clinical trial. Thus, the present results perfectly support the dose justification of the ATR-002 clinical trial and support the in vivo effect of ATR-002 in the treatment of SARS-CoV-2. The reduction of damage in

the lungs as shown in **Example 8** and **Figure 23** further provides evidence that the MEK inhibitor ATR-002 of the present invention is able to reduce long-term tissue damage, which is one typical syndrome of the described long COVID syndromes. Therefore, **Example 8** provides evidence for the effect of the MEK inhibitor ATR-002 of the present invention in prevention and treatment of the long COVID syndrome.

Furthermore, to study the effect of ATR-002 on viral replication in genuine human cells of the respiratory tract, primary human Air-Liquid Interference (ALI) cultures were used, that were obtained from throat swabs of four healthy adults. Three of the four ALI cultures could be infected with SARS-CoV-2 and were treated with ATR-002 using two different concentrations as described in **Example 9**. **Figure 17** shows results of one experiment. Both concentrations used (50 $\mu$M and 100 $\mu$M) completely blocked the production of progeny viral particles in these three cultures, indicating that ATR-002 is very effective against a SARS-COV-2 infection in the primary human ALI cultures. This is a good indication that ATR-002 will prove effective in treating human coronavirus infections, and specifically SARS-CoV-2 stage II.

As can be seen from **Figure 15** which is from a paper from Hasan et al., entitled "COVID-19 Illness in Native and Immunosuppressed States: A Clinical-Therapeutic Staging Proposal" and summarized below, in stage II COVID-19 both the viral response phase and the host response phase are ongoing, so that this would be the stage at which the use of a MEK inhibitor would be most useful. The stages of COVID-19 as determined by Hasan et al are summarized again below.

The initial stage, termed Stage I, is a mild infection and occurs at the time of inoculation and early establishment of disease. For most people, this involves an incubation period associated with mild and often non-specific symptoms for some days such as malaise, fever, and a dry cough. In patients who can keep the virus limited to this stage of COVID-19, prognosis and recovery is excellent. Treatment at this stage is primarily targeted towards symptomatic relief. Should an antiviral therapy be proven beneficial, targeting selected patients during this stage may reduce duration of symptoms, minimize contagiousness, and prevent progression of severity.

In the second stage, termed Stage II, of an established pulmonary disease, viral multiplication and localized inflammation in the lung is the norm. Stage II includes pulmonary involvement, termed Stage IIa, without and Stage IIb with hypoxia. During this stage, patients develop a viral pneumonia, with cough, fever and possibly hypoxia. Over the course of the disease, dyspnea occurs after a median of 13 days after the first onset of symptoms (range 9-16.5 days). Dyspnea is a sign of serious disease of the airway, lungs, or heart and is characterized by difficult or labored breathing and shortness of breath. In the case of COVID-19, imaging with chest X-ray or computerized tomography reveals bilateral infiltrates or ground glass opacities. It is at this stage that most patients with COVID-19 need to be hospitalized for close observation and management. Treatment primarily consists of supportive measures and antiviral therapies, once available. It is possible that patients will nevertheless progress to Stage III to require mechanical ventilation in an intensive care unit (ICU).

In Stage II COVID-19, markers of systemic inflammation may be elevated, but not remarkably so. In early studies performed on the first group of patients in Wuhan, China, it was found that upon entry into the hospital, plasma IL1$\beta$, IL1RA, IL7, IL8, IL9, IL10, basic FGF, GCSF, GMCSF, IFN$\gamma$, IP10, MCP1, MIP1$\alpha$, MIP1$\beta$, PDGF, TNF-$\alpha$, and VEGF concentrations were higher than in healthy adults. In addition, it was found that patients in ICU had plasma concentrations of IL2, IL7, IL10, GCSF, IP10, MCP1, MIP1$\alpha$, and TNF-$\alpha$ were higher than in patients upon admission to the hospital (Huang et al.; The Lancet; Vol 395; pp.: 497-506 February 15, 2020), indicating that an increase in these cytokines marks the transition from COVID-19 stage II to stage III. This transition marked by sudden and rapidly progressing clinical deterioration that is called the "COVID-19 cytokine storm" (also termed "cytokine storm" herein). As described in detail in Jamilloux et al. (Autoimmunity Reviews 2020), here markers of systemic inflammation are elevated, such as IL-1$\beta$, IL-R$\alpha$, IL-6, TNF-$\alpha$ and sIL2R$\alpha$. This corresponds to what was shown by Huang et al. discussed above.

A minority of COVID-19 patients will experience a COVID-19 cytokine storm and transition into the third and most severe stage of illness, termed Stage III, which manifests as an extra-pulmonary systemic hyperinflammation syndrome. Overall, the prognosis and recovery from this critical stage of illness is poor.

For this reason, the dual mechanism provided by MEK inhibition would be critical to prevent progressing into Stage III and therefore decrease mortality.

In order to better understand the mechanisms of SARS-CoV2 infection, further studies on the role of MEK inhibitors in preventing SARS-CoV-2 infection were performed as described in Example 10. Specifically, it is known that SARS-CoV-2 can enter the cell via ACE2/TMPRSS2. A possible effect of the expression of ACE2 and TMPRSS2 on the activation of the Raf/MEK/ERK signaling pathway was therefore addressed. ACE2 and TMRPSS2 overexpressing A549 cells were infected with SARS-CoV-2 and the phosphorylation state of ERK was analyzed 1 h.p.i. Shown in **Figure 18** are results of one out of three independent experiments. It was found that the overexpression of ACE2 in A549 cells leads to an ERK activation during the SARS-CoV-2 infection.

After this, it was tested whether ACE2 expression is reduced in cells after MEK inhibition via ATR-002 treatment. Calu-3 cells were treated with ATR-002 for 24 hours and the results were analyzed via immunofluorescence microscopy and WES technology. Results can be seen in **Figure 19,** where it is clear that ATR-002 leads to a reduction of ACE2

on Calu-3 cells.

In a next step, it was tested whether ATR-002 can block the replication of SARS-CoV-2 in ACE2 overexpressing A549 cells. Therefore, A549-ACE2, A549-ACE2/TMPRSS2 or Calu-3 cells were infected with SARS-CoV-2 and treated with ATR-002 as described above.

ATR-002 reduced the production of progeny viral particles in Calu-3 and A549-ACE2/TMPRSS2 cells, if cells were infected with a MOI of 0.001. Surprisingly, the ATR-002 treatment did not affect the production of progeny viral particles in A549-ACE2/TMPRSS2 cells when the concentration of the viral inoculum was ten times higher (MOI: 0.01). SARS-CoV-2 can enter the cell via two different pathways. Either via the direct activation of the spike protein by TMPRSS2, resulting in the fusion of viral and cellular membrane, or by the endosomal internalization and activation of the spike protein by cathepsin L.

Finally, to analyze whether a treatment with a MEK-inhibitor can reduce the expression of ACE2, Vero cells were pre-incubated with the MEK inhibitor CI-1040 or ATR-002 and were infected with a VSV pseudotype virus that carries the spike protein of SARS-CoV-2 on its surface and expresses the reporter gene GFP upon successful infection and internalization. Positive cells were analyzed by light microscopy. Data shown in **Figures 21** and **22** represent means $\pm$ SD of three independent experiments and shows that pre-incubation with CI-1040 or ATR-002 leads to a reduction in the internalization of the VSV-S pseudotype virus. This effect was time dependent. The longer the pre-incubation with CI-1040 or ATR-002, the lower the amount of GFP positive cells, which is the readout for the infection. These results indicate that MEK inhibitors, such as CI-1040 and ATR-002 reduce the cellular expression of ACE2 and could therefore be useful to prevent entry of viruses into cells.

The experiments described above indicate that MEK inhibition may play a role in preventing SARS-CoV-2 infection by blocking ACE2 receptors and in preventing the progression of SARS-CoV-2 in patients that are asymptomatic but tested positive for a human coronavirus. This is significant as it could provide a preventive or prophylactic effect of MEK inhibition in the context of early human coronavirus infections.

The MEK inhibitors of the present invention can be used in a method for treating and/or prevention. As such the term "treating" or "treatment" includes administration of a MEK inhibitor preferably in the form of a pharmaceutical composition, to a subject suffering from a coronavirus infection for the purpose of ameliorating or improving symptoms. Similarly included is the administration of a MEK inhibitor, preferably in the form of a pharmaceutical, to a subject suffering from a COVID-19 cytokine storm for the purpose of ameliorating or improving symptoms. Further, included is the administration of a MEK inhibitor, preferably in the form of a pharmaceutical, to a subject suffering from a long COVID syndrome.

Furthermore, the term "prevent" as used herein, refers to a medical procedure whose purpose is to prevent a disease. As used herein, the terms "prevent", "prevention" and "preventing" refer to the reduction in the risk of acquiring or developing a given condition in a patient diagnosed with a coronavirus infection, such as a COVID-19 cytokine storm or a long COVID syndrome. Also meant by "prevention" is the reduction or inhibition of markers of systemic hyperinflammation, such as TNF-$\alpha$, IL-1$\beta$, IP-10, IL-8, MCP-1, and/or MIP-1$\beta$, in a subject diagnosed with a coronavirus infection, such as SARS-CoV-2 to reduce the risk of systemic hyperinflammation, such as a COVID-19 cytokine storm, in a subject.

"MEK inhibitors" inhibit the mitogenic signaling cascade Raf/MEK/ERK in cells or in a subject by inhibiting the MEK (mitogen-activated protein kinase kinase). This signaling cascade is hijacked by many viruses, in particular influenza viruses and corona viruses, to boost viral replication. Specific blockade of the Raf/MEK/ERK pathway at the bottleneck MEK therefore impairs growth of viruses, in particular corona viruses. Additionally, MEK inhibitors show low toxicity and little adverse side effects in humans. There is also no tendency to induce viral resistance (Ludwig, 2009). In the context of the invention, the MEK inhibitor is selected from the group consisting of PD-0184264, CI-1040, GSK-1120212, GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof. A particularly preferred inhibitor is PD-0184264.

The "disease" caused by a corona virus may be an acute respiratory disease caused by SARS-CoV, SARS-CoV-2 or MERS. Specifically, in a preferred embodiment the coronavirus is SARS-CoV-2 and the patient is suffering from COVID-19. Preferably, the subject is suffering from long COVID symptoms. It is envisioned that subjects predisposed - with risk factors for Long COVID, such as older people, women and those with a greater number of different symptoms in the first week of their illness are more likely to develop long COVID.

The "coronavirus" may be SARS-CoV, SARS-CoV-2 or MERS or a related new zoonotic or mutant coronavirus. In one specific embodiment, the coronavirus is resistant to previous antiviral treatment, such as Remdesivir.

The "subject", which may be treated by the inhibitors, in particular MEK inhibitors of the present invention is a human subject that has been diagnosed with a coronavirus infection. In one embodiment, the subject is hospitalized. In a further embodiment, the subject is in a stage of infection after the begin of the adaptive immune response. The subject may be of any age and may be a child between 0 to 10 years, a teenager between 10 and 18 years or an adult of 18 years and above. The subject may optionally be between the ages of 50 and 65, between the ages of 18 or 50, or older than 65 years of age. In other embodiments the subject is selected from the group consisting of subjects who are at

least 60 years old, subjects who reside in chronic care facilities, subjects who have chronic disorders of the pulmonary or cardiovascular system, subjects who required regular medical follow-up or hospitalization during the preceding year because of chronic metabolic diseases, renal dysfunction, hemoglobinopathies, or immunosuppression.

In one specific aspect, the subject may be treated with the MEK inhibitor in order to prevent or treat a "COVID-19 cytokine storm". As mentioned above, this term is used within its regular meaning as used in the art (see, in this respect, Jamilloux et al, 2020) to mean a cytokine storm that may occur in subjects that have been infected with a human-pathogenic coronavirus, in particular SARS-CoV-2. Such a cytokine storm is marked by rapid clinical deterioration and an increase in pro-inflammatory cytokines marks the transition from Stage II to Stage III COVID-19. Specifically, both Huang et al. and Jamilloux et al. note that a sudden increase in IL-1β and/or TNF-α was observed, possibly together with an increase in two or more, three or more, four or more, five or more or all six of TNF-α, IL-1β, IP-10, IL-8, MCP-1, and MIP-1β. In one aspect, the MEK inhibitor is used to reduce the level of IL-1β and/or TNF-α in the subject, preferably reducing the level of one of more, two or more, three or more, four or more, five or more or all six of TNF-α, IL-1β, IP-10, IL-6, IL-8, MCP-1, MIP-1α and MIP-1β in a subject.

The inflammatory cytokines and chemokines cited above are well known in the art. Specifically, the terms TNF-a, IL-1β, IP-10, IL-8, MCP-1, and MIP-1β refer to the human protein sequences known in UNIPROT and GENEBANK under the following accession numbers:

| Gene | GeneBank accession | Uniprot accession | species |
|---|---|---|---|
| TNF-α | NM _ 000594 | P01375 | Human |
| IL-1β | NM_000576 | P01584 | |
| IP-10 | NM_001565 | P02778 | |
| IL-8 | NM _ 000584 | P10145 | |
| MCP-1 | NM_002982 | P13500 | |
| MIP-1β | NM _ 002984 | P13236 | |

The pharmaceutical composition for the use of the invention and comprising a MEK inhibitor such as PD-0184264 is administered to a human patient that is hospitalized and is suffering from a disease caused by a coronavirus. In one aspect, the human patient is over 60 years of age or belongs to a high risk or very high risk group for corona virus infection. In the case of COVID-19, the very high risk group includes patients who:

- are over 70 years of age
- have had an organ transplant
- are undergoing active chemotherapy
- are having radical radiotherapy for lung cancer
- have cancers of the blood or bone marrow such as leukemia, lymphoma or myeloma who are at any stage of treatment
- are having immunotherapy or other continuing antibody treatments for cancer
- are having other targeted cancer treatments which can affect the immune system
- have had bone marrow or stem cell transplants in the last 6 months, or who are still taking immunosuppression drugs
- have severe respiratory conditions including cystic fibrosis, severe asthma, pulmonary fibrosis, lung fibrosis, interstitial lung disease and severe COPD
- have a condition that provide a very high risk of getting infections (such as SCID, homozygous sickle cell)
- are taking medicine that makes you much more likely to get infections (such as high doses of steroids or immunosuppression therapies)
- have a serious heart condition and are pregnant.

The high risk group includes people who:

- are over 60 years of age
- have a learning disability
- have a lung condition that's not severe (such as asthma, COPD, emphysema or bronchitis)
- have heart disease (such as heart failure)
- have high blood pressure (hypertension)
- have diabetes
- have chronic kidney disease
- have liver disease (such as hepatitis)
- have a medical condition that can affect your breathing

- have cancer
- have a weak immune system (immunosuppressed)
- have cerebrovascular disease
- have a condition affecting brain or nerves (such as Parkinson's disease, motor neuron disease, multiple sclerosis, or cerebral palsy)
- have a problem with their spleen or have the spleen removed
- have a condition that means you have a high risk of getting infections (such as HIV, lupus or scleroderma)
- are taking medicine that can affect the immune system (such as low doses of steroids)
- have obesity.

In the method of the invention, PD-0184264 may be administered orally, intravenously, intrapleurally, intramuscularly, topically or via inhalation. Preferably, PD-0184264 is administered via inhalation or orally. In preferred embodiment, PD-0184264 is administered once daily in an oral dosage between 100mg and 1000mg, preferably 300mg, 600mg or 900mg, for on 1 to 21 consecutive days, preferably 5 to 18 or 7 to 14 consecutive days after hospitalization.

Specifically, as described previously, in Phase I clinical studies PD-0184264 was administered with a starting dose of 100 mg and up to three escalation steps in seven arms, following a single ascending dose / multiple ascending dose (SAD / MAD) regime. Administration scheme was one dose PD-0184264, escalating 100 mg to 900 mg (SAD), followed by seven doses PD-0184264, escalating 100 mg to 600 mg QD over seven days (MAD). Each dose cohort was considered to be safe by the Safety Review Committee (SRC), with release allowed for the next higher dose (up to SAD 900 mg and MAD 600 mg, respectively). The observed pharmacokinetic profile supports the intended once-daily regime for the further clinical development, and a dosage of 900mg is intended for testing.

Only few adverse events in total and no serious adverse events were observed during the trial. PD-0184264 is thus considered to be safe and well tolerable. Pharmacokinetics exposure and determination of MEK inhibition were verified in the Phase I study and confirm the maintenance of clinically relevant blood levels.

The present invention also envisages different compositions, preferably pharmaceutical compositions. The present invention relates to a composition comprising PD-0184264 for use in a method for the treatment of a disease caused by a coronavirus, such as SARS-CoV-2.

As mentioned above, the composition comprising the MEK inhibitor may be a pharmaceutical composition. A preferred embodiment of the pharmaceutical composition comprises PD-0184264. Preferably, such compositions further comprise a carrier, preferably a pharmaceutically acceptable carrier. The composition can be in the form of orally administrable suspensions or tablets, nasal sprays, preparations for inhalation devices, sterile injectable preparations (intravenously, intrapleurally, intramuscularly), for example, as sterile injectable aqueous or oleaginous suspensions or suppositories.

The MEK inhibitor is preferably administered in a therapeutically effective amount. The "therapeutically effective amount" for PD-0184264 or each active compound/inhibitor can vary with factors including but not limited to the activity of the compound used, stability of the active compound in the patient's body, the severity of the conditions to be alleviated, the total weight of the patient treated, the route of administration, the ease of absorption, distribution, and excretion of the compound by the body, the age and sensitivity of the patient to be treated, adverse events, and the like, as will be apparent to a skilled artisan. The amount of administration can be adjusted as the various factors change over time.

The inhibitors, methods and uses described herein are applicable to human therapy. The compounds described herein, in particular, PD-0184264 may be administered in a physiologically acceptable carrier to a subject, as described herein. Depending upon the manner of introduction, the compounds may be formulated in a variety of ways as discussed below. The concentration of therapeutically active compound in the formulation may vary from about 0.1-100 wt. %. The agents may be administered alone or in combination with other treatments. For example, for the treatment of Stage II COVID-19, the PD-0184264 may be administered at a dosage in the range of 10 to 100 mg/kg PD-0184264, preferably in the range of 25 to 75 mg/kg PD-0184264. Preferred administration is as a once daily dosage between 100 and 1000 mg, including any dosage amount in between such as 200, 300, 400, 500, 600, 700, 800, and 900 mg. In a preferred embodiment, administration is once daily and the dosage is 600mg or 900mg administered orally. PD-0184264 can be administered for a time period of 1 to 21 consecutive days or more, preferably for 5 to 18 and most preferably for 7 to 14 days after hospitalization.

The pharmaceutical compounds in the method of present invention can be administered in any suitable unit dosage form. Suitable oral formulations can be in the form of tablets, capsules, suspension, syrup, chewing gum, wafer, elixir, and the like. Pharmaceutically acceptable carriers such as binders, excipients, lubricants, and sweetening or flavoring agents can be included in the oral pharmaceutical compositions. If desired, conventional agents for modifying tastes, colors, and shapes of the special forms can also be included.

For injectable formulations, the pharmaceutical compositions can be in lyophilized powder in admixture with suitable excipients in a suitable vial or tube. Before use in the clinic, the drugs may be reconstituted by dissolving the lyophilized powder in a suitable solvent system for form a composition suitable for intravenous or intramuscular injection.

In one embodiment, the reduction of the viral infection is a reduction in plaque forming units (PFU)/ml. The "plaque forming units" is a measure of the number of particles capable of forming plaques per unit volume, such as virus particles. It is a functional measurement rather than a measurement of the absolute quantity of particles: viral particles that are defective or which fail to infect their target cell will not produce a plaque and thus will not be counted. For example, a solution of coronavirus with a concentration of 1,000 PFU/μl indicates that 1 μl of the solution carries enough virus particles to produce 1000 infectious plaques in a cell monolayer. In the case of the present invention, a cell culture treated with an inhibitor shows a reduced number of plaque forming units in a culture after the treatment, when compared to a culture before the treatment with a MEK inhibitor, such as PD-0184264.

For the purpose of the invention the active compound as defined above also includes the pharmaceutically acceptable salt(s) thereof. The phrase "pharmaceutically acceptable salt(s)", as used herein, means those salts of compounds of the invention that are safe and effective for the desired administration form. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylaminoethanol, histidine, procaine, etc.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

All publications and patents cited in this disclosure are incorporated by reference in their entirety. To the extent the material incorporated by reference contradicts or is inconsistent with this specification, the specification will supersede any such material.

Unless otherwise indicated, the term "at least" preceding a series of elements is to be understood to refer to every element in the series. Those skilled in the art will recognize or be able to ascertain using no more than routine experimentation many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

## EXAMPLES

The following examples illustrate the invention. These examples should not be construed as to limit the scope of the invention. The examples are included for purposes of illustration and the present invention is limited only by the claims.

### MATERIALS:

### 1. CELL LINES

| Description | Species | Origin | Source |
|---|---|---|---|
| Caco-2 | Homo sapiens | Epithelial cells, Colon, Colorectal adenocarcinoma | University Hospital Tübingen Institute of Medical Virology and Epidemiology |

(continued)

| Description | Species | Origin | Source |
|---|---|---|---|
| Vero E6 | *Cercopithecus aethiops,* Kidney | Epithelial cells, Kidney, normal | ATCC (American Type Culture Collection) Catalogue No CRL-1586 |
| A549 | Homo sapiens | Epithelial cells, carcinomic alveolar basal epithelial cell | ATCC (American Type Culture Collection) Catalogue No. CCL-185 |
| Calu-3 | Homo sapiens | Epithelial cells, Lung adenocarcinoma cells | ATCC (American Type Culture Collection) Catalogue No. HTB-55; |

| **2. VIRUS** | | |
|---|---|---|
| **Description** | **Species** | **Source** |
| SARS-CoV-2 | Severe acute respiratory syndrome coronavirus | SARS-CoV-2 isolate hCoV-19/Germany/FI1103201/2020 (GSAID EPI-ISL_463008) "FI" |
| SARS-CoV-2 SA variant | Severe acute respiratory syndrome coronavirus | SARS-CoV-2 isolate hCoV-19/Germany B.1351 South African variant "SA" (210211_SAv) isolated in February 2021 and belongs to the B.1.351 SARS-CoV-2 lineage. The isolate was sequenced by NGS and the mutations N501Y, K417N, and E484K were detected by rtPCR |

**3. DRUG**

ATR-002 (PD0184264) [2-(2-chloro-4-iodophenylamino)-N-3,4-difluoro benzoic acid], (M = 409,55 g/mol) was synthesized at ChemCon GmbH (Freiburg, Germany). For all cell culture experiments, a 10 mM stock solution of ATR-002 was prepared in DMSO (Merck-Millipore, Darmstadt, Germany) and further diluted in the respective media.

**Example 1: SARS-CoV-2 uses the MEK pathway**

In this initial experiment, the inventors were interested in finding out whether inhibition of the MEK pathway leads to inhibition of viral propagation. ATR-002 targets the host cell factor MEK, however the dose-response relationship of ATR-002 does not relate to the virus, but to the inhibition of the kinase. In *in vitro* experiments the antiviral efficacy of ATR-002 strongly depends on the activation status of MEK in the cell line used for the experiment. CaCo2 Cell-lines used for SARS-CoV-2 investigations below show a high constitutive MEK-activity. As a comparison, experiments in Vero cells were also performed.

**Methods:**

**A. Virus Yield Reduction Assay (VYR)**

For the Virus yield reduction assay a 24-well plate (Greiner Bio-One, Cat. 662-160) with 95% confluent Caco-2 cells was prepared. The wells were washed once with Caco-2 infection medium DMEM (Gibco, Cat. 41965-039) supplemented with 5% FCS (Capricorn, Cat. FBS-12A), 1% Penicillin/Streptomycin, (Sigma Aldrich, Cat. P4333) and 1% Non-essential amino acids (NEAA) (Merck, Cat. K 0293). Cells were infected with SARS-CoV-2 (MOI 0.1) for 1h in 200μL Caco-2 infection medium per well. Afterwards the Virus inoculum was removed completely and cells were treated with 1mL per well with different concentrations of ATR-002 (100μM, 50μM, 25μM, 12.5μM, 6.25μM, 3.125μM, 1.56μM, 0.78μM, 0.39μM, 0.195μM, 0.098μM, 0μM) in Caco-2 infection medium with 1% DMSO for 24h at 37°C, 5% $CO_2$. Supernatants were harvested 24h post infection and centrifuged at max. speed, 5min, 4°C to remove cell debris. Aliquots a 200μL were prepared and stored at -80°C. The cell layer of each well was lysed by addition of 50μL modified RIPA Buffer supplemented with phosphatase and protease inhibitor cocktails, incubation 15min, 4°C. Lysates were centrifuged at max. speed, 5min, 4°C, to remove cell debris. Lysates were stored at -80°C.

### B. Plaque Assay Infection of Vero cells with SARS-CoV-2 upon MEK inhibition

To determine the virus titer a standard plaque assay was performed. Vero cells were infected with SARS-CoV-2 strain FI (MOI 0.01). 1 hour post infection, the cells were treated with 50 μM ATR-002 or the respective amount of DMSO as a control. The compounds were present in the culture medium during the complete infection time. After 24, 36 and 48 hpi, supernatants were collected and virus titers quantified by plaque assay below. Data of a single experiment with three biological replicates are shown in **Figure 2A.**

Vero E6 cells were seeded at 100% confluency in 6-well plates (Greiner Bio-One, Cat. 657-160). 6-fold dilutions of virus samples were prepared in Vero E6 infection medium (IMDM, Gibco, Cat. 12440-053) supplemented with 5% FCS (Capricorn, Cat. FBS-12A) and 1% Penicillin/Streptomycin (Sigma Aldrich Cat. P4333). The cells were washed once with Vero E6 infection medium before infection with 1mL per well of the virus dilutions for 1h at 37°C, 5% $CO_2$. Afterwards the Virus inoculum was removed completely, and the cells were overlaid with Avicel-Medium (1,25% Avicel (FMC BioPolymer, Cat. RC581), 10% MEM (Gibco, Cat. 21430-20),0,01% DEAE-Dextran (Sigma Aldrich, Cat.No.: D9885), 2,8% $NaHCO_3$ (Merck, Cat. No: 1.06329.1000), 1% Penicillin/Streptomycin (Sigma Aldrich, Cat. P4333), 0,2% BSA (Carl Roth, Cat. 9163.4), 1% L-Glutamine (Sigma Aldrich, Cat. G7513). Incubation at 37°C, 5% $CO_2$. After 72h the Avicel-Medium was removed. The cells were rinsed twice with PBS (Gibco, Cat. 14190-094), fixed with 4% Roti-Histofix (Carl Roth, Cat. A146.1) in PBS (Lonza, Cat. 17515Q) for 30min at 4°C and stained with Crystal violet solution (1% Crystal violet (Merck, Cat. 1408), 10% Ethanol (SAV-IP, Cat. ETO-5000-99-1) in $ddH_2O$). The virus titer was calculated based on the number of plaques per well and the respective dilution factor.

**C. Detection of the viral gene RdRP during infection with SARS-CoV-2 upon MEK inhibition.** Vero cells were infected with FI (MOI 0.01). 1 hour post infection, the cells were treated with 50 μM ATR-002 or the respective amount of DMSO as a control. The compounds were present in the culture medium during the complete infection time. After 24, 36 and 48 hpi, supernatants were collected and used for RNA extraction. Extrapolated genome copy number per ml of the RdRP gene based on a standard curve after 24, 36 and 48 hpi. Data of a single experiment with three biological replicates are shown in **Figure 2B.**

### D. Wes analysis

Wes™ capillary electrophoresis by ProteinSimple® was used to identify and quantify pERK1/2, ERK1/2 and SARS-CoV-2 Nucleoprotein. Cell lysates were diluted with 0.1X Sample Buffer (ProteinSimple, Abingdon, Oxford, UK) to approximately 50ng total protein per lane and analyzed using specific antibodies. The primary antibodies pERK1/2 (Phospho-p44/42 MAPK (Erk1/2) (Thr202/Tyr204) (D13.14.4E) XP® Rabbit mAb, Cat. 43702) and ERK1/2 (p44/42 MAPK (Erk1/2) (137F5) Rabbit mAb, Cat. 4695) were purchased from Cell Signaling Technology (Cell Signaling Technology, Danvers, Massachusetts, U.S.A.) and used at 1:50 (for pERK1/2) and 1:100 (ERK1/2) dilutions in antibody diluent (ProteinSimple, Abingdon, Oxford, UK). The SARS-CoV-2 Nucleoprotein Antibody, monoclonal mouse Ab was purchased from ProSci (Cat. 35.580) and used at 1μg/ml in antibody diluent (ProteinSimple, Abingdon, Oxford, UK). The anti-rabbit secondary antibody (ProteinSimple, Cat. DM-001) and anti-mouse secondary antibody (ProteinSimple, Cat. DM-002) and all other reagents for WES analysis were also purchased from ProteinSimple and were ready to use.

### Results:

The results of the above methods can be seen in **Figures 1** and **2. Figure 1** shows that when CaCo-2 cells infected SARS-CoV-2 (MOI 0.1) were treated with different concentrations of ATR-002, inhibition of ERK as well as SARS-CoV-2 and the nucleocapsid protein, which is a viral marker, was seen at concentrations of 50 and 100 μM ATR-002. This high amount of ATR-002 necessary for inhibition does not relate to the virus titer but is rather due to the choice of CaCo2 cells, which have constitutively active MEK, so that high amounts are necessary for inhibitor of MEK. These experiments show that SARS-CoV-2 uses the MEK pathway for virus export.

Additionally **Figure 2** shows that Vero cells infected with SARS-CoV-2 and treated with 50 μM ATR-002 have a lower viral load than control cells throughout the whole observation period of 24h, 36h and 48h, indicating that the inhibitor not just caused a delay in virus replication but inhibited propagation in a sustained manner. This was confirmed by measurement of the SARS-CoV-2 viral gene RdRP.

### Example 2: Replication of SARS-CoV-2 in Calu-3 cells and ERK activation in the SARS CoV-2 life cycle as well as inhibition with ATR-002

In order to verify the effects seen in CaCo2 and Vero cells, further tests were performed on the human bronchioepithelial cell line Calu-3. First it was tested whether SARS-CoV-2 can infect Calu-3 cells (A) and whether infection would lead to activation (phosphorylation) of ERK in Calu-3 cells (B). Furthermore, it was tested whether ATR-002 was

capable of inhibiting SARS-CoV-2 in Calu-3 cells (C).

**A. Infection of Calu-3 cells with SARS-CoV-2**

The human bronchioepithelial cell line Calu-3 was cultivated in Dulbecco's modified Eagle's medium (DMEM) supplemented with 10% standardized foetal bovine serum (FBS Advance; Capricorne), 2 mM L-glutamine, 100 U/mL penicillin, and 0.1 mg/mL streptomycin. All cells were cultured in a humidified incubator at 37°C and 5% CO2. Calu-3 cells were infected with the SARS-CoV-2 isolate hCoV-19/Germany/FI1103201/2020 (EPI-ISL_463008) in infection-PBS (containing 0.2% BSA, 1% CaCl2, 1% MgCl2, 100 U/mL penicillin and 0.1 mg/mL streptomycin) at a MOI of 2 or Mock infected. Titers of newly produced SARS-CoV-2 virus particles and supernatants were analyzed, beginning 4 h.p.i. Figure 3A shows the results of three independent experiments. Figure 3B shows Western Blot lysates that were prepared after the indicated time points. Figure 3 C and D show the quantification of SARS-CoV-2 N and S protein expression and kinase phosphorylation during the time course of infection. Shown are means ± SD of three independent experiments. Data passed a one-way ANOVA followed by Dunnett's multiple comparison test (* $p \leq 0.0332$; **** $p \leq 0.0001$). Dashed lines indicate the level of the 0 h infection. (C,D) Exemplary Western Blot analysis of the quantification shown in (B). In a further experiment shown in Figure 3 (E) Calu-3 cells were infected with SARS-CoV-2 (FI) using different MOIs. ERK-activation was analyzed 1 h.p.i.

As can be seen from Figure 3, the SARS-CoV-2 was able to replicate in the Calu-3 cells. Additionally, it was seen that SARS-CoV-2 infection leads to an ERK activation on the very early stage of the viral life cycle. An ERK activation was observed 1 h.p.i. No additional ERK-activation in the later stages of the viral life cycle could be observed, indicating a possible role of the pathway activation for early process during the viral infection.

**B. ERK activation by SARS CoV-2 in Calu-3 cells**

To analyze the importance of ERK, the direct target of MEK in the Raf/MEK/ERK pathway, during the SARS-CoV-2 life cycle, an ERK1/2 knockdown was introduced in Calu-3 cells. 72 h.p.t. the cells were infected with SARS-CoV-2 and the expression of the viral spike protein and the production of progeny viral particles was evaluated. It was found that the ERK-knockdown results in decreased production of progeny viral titers, as can be seen in Figure 4 (A-C: MOI: 1.0; D: MOI: 0.1). Figure 4 A shows the expression of the viral $S_0$ protein analyzed 8 h.p.i. Immunoblots were prepared and probed with anti-S, anti-ERK1/2 and anti-Tubulin antibodies. Shown are results of one out of three independent experiments. Figure 4 B shows Quantification of the $S_0$ protein expression and the knockdown efficacy. Shown are means ± SD of three independent experiments. Data passed an unpaired two-tailed *t*-test (* $p \leq 0.0332$, **** $p \leq 0.0001$). The titers of (A) are shown in Figure 4 C. Shown are means ± SD of three independent experiments, each performed in duplicates. PFU/ml: Data passed an unpaired two-tailed *t*-test with Welch-correction. Percentage: Data passed a paired two-tailed *t*-test (* $p \leq 0.0332$; *** $p \leq 0.0002$). Figure 4 D shows the Titer analysis 24 h.p.i. Shown are means ± SD of three independent experiments, each performed in duplicates. PFU/ml: Data passed an unpaired two-tailed *t*-test with Welch-correction. Percentage: Data passed a paired two-tailed *t*-test (* $p \leq 0.0332$; **** $p \leq 0.0001$).

Thus, it was shown that that a knockdown of ERK lead to a decrease in the expression of the spike protein and the production of progeny viral particles, not only confirming the role of ERK during the viral life cycle but also verifying the function of the MEK/ERK kinase module in SARS-CoV-2 propagation by genetic means.

**C. ATR-002 is capable of inhibiting SARS-CoV-2 in Calu-3 cells**

It was then tested whether a MEK inhibitor could decrease SARS-CoV-2 titers in Calu-3 cells in a comparable way to the decrease seen in CaCo2 and Vero cells in **Example 1**. Calu-3 cells were infected with SARS-CoV-2 and treated with 100μM, 75μM or 50μM ATR-002 using the same virus yield protocol as for **Example 1**. Specifically, for the virus yield reduction assay 24-well plates with 95% confluent Vero E6, CaCo2 and Calu-3 cells were prepared. The wells were washed once with Infection medium prior infection with SARS-CoV-2 SA (MOI 0.1, 1 and 10) for 1 hour in 200 μL infection medium per well. Afterwards the virus inoculum was removed completely, the wells were washed once with Infection medium and then treated with 1 mL per well with different concentrations of ATR-002 (100 μM, 75 μM, 50 μM, 0 μM) in infection medium with 1% DMSO for 24 hours at 37°C 5% $CO_2$. Three wells were prepared of each condition. The supernatants of the three wells per condition were pooled 24 hours post infection and centrifuged at max. speed, 5 minutes, 4°C to remove cell debris. Aliquots a 140 μL were prepared of the supernatants and stored at -80°C. The virus titer of the samples was determined via RT-qPCR (Real Time Quantitative Polymerase Chain Reaction) in technical triplicates. Therefore, the viral RNA was isolated and the probe used for detection of the viral RNA copies was directed against the SARS-CoV-2 N gene.

As can be seen from **Figure 5**, the effect seen on all cell types was comparable.

In a next step, Calu-3 cells were incubated with increasing amounts of ATR-002 over 24, 48 or 72 hours.

Specifically, Calu-3 cells were infected with SARS-CoV-2 (FI) using a MOI of 0.01. 1 h.p.i. cells were treated with ATR-002. Mock, SARS-CoV-2 and DMSO served as controls. Figure 6 A shows titer reduction of SARS-CoV-2 in Calu-3 cells after ATR-002 (10 - 150 μM) treatment. Untreated (SARS-CoV-2) and DMSO (0.1 %) treated cells served as negative controls. Data represents means ± SD of three independent experiments, each performed in triplicates. Data passed a one-way ANOVA followed by Dunnett's multiple comparison test (** $p \leq 0.0021$; *** $p \leq 0{,}0002$; **** $p \leq 0.0001$) for each time point separately. DMSO was used as reference. Figure 6 B shows the EC50 calculation of the values from (A). Data in combination with (C) was used to calculate the CC50 value and the selectivity index (SI). Figure 6C shows Cytotoxicity evaluation of ATR-002 in Calu-3 cells after 72 h treatment. Data represent means ± SD of three independent experiments. Figure 6D shows Light microscopy pictures of one out of three independent experiments of (A). We could observe a concentration dependent decrease in the production of progeny viral particles. In a non-toxic concentration range. This effect maintained over the total infection time of 72 hours indicating that the pathway inhibition with ATR-002 has a longtime effect on the viral life cycle.

**Example 3: In vitro efficacy of ATR-002 against SARS-CoV-2 SA variant B.1351**

The same experimental setup as in Examples 1 and 2 was used to see whether ATR-002 was effective against the South African SARS-CoV-2 variant B.1351. Vero cells and Calu-3 cells were infected with SARS-CoV-2 SA at a MOI of 0.1, 1 and 10 and treated with 20.48, 30.72 and 40.96 μg/ml (corresponding to 50, 75 and 100μM) ATR-002 as can be seen from **Figure 7** in comparison to **Figure 5.**

Specifically, for the virus yield reduction assay 24-well plates with 95% confluent Vero E6 and Calu-3 cells were prepared. The wells were washed once with Infection medium prior infection with SARS-CoV-2 SA (MOI 0.1, 1 and 10) for 1 hour in 200 μL infection medium per well. Afterwards the virus inoculum was removed completely, the wells were washed once with Infection medium and then treated with 1 mL per well with different concentrations of ATR-002 (100 μM, 75 μM, 50 μM, 0 μM) in infection medium with 1% DMSO for 24 hours at 37°C 5% $CO_2$. Three wells were prepared of each condition. The supernatants of the three wells per condition were pooled 24 hours post infection and centrifuged at max. speed, 5 minutes, 4°C to remove cell debris. Aliquots a 140 μL were prepared of the supernatants and stored at -80°C. The virus titer of the samples was determined via RT-qPCR (Real Time Quantitative Polymerase Chain Reaction) in technical triplicates. Therefore, the viral RNA was isolated and the probe used for detection of the viral RNA copies was directed against the SARS-CoV-2 N gene.

As can be seen in **Figure 7,** reduction of virus yield by ATR-002 could be seen in both Vero and Calu-3 for the SARS-CoV-2 SA variant.

**Example 4: ATR-002 reduces cytokine & chemokine gene expression in an acute lung injury (ALI) mouse model**

The ALI mouse model of LPS induced cytokine & chemokine expression allows to investigate the immunomodulatory efficacy of ATR-002 in the absence of a virus infection *in vivo.*

**Methods:**

Mice were anesthetized using ketamine (10 mg/kg) solution injected i.p. Both treatment groups were stimulated with 5mg/kg LPS prepared in PBS via i.p route. One hour post stimulation the treatment group was treated with 25 mg/kg ATR-002 via i.p route. Mice were euthanized 6h post treatment and lungs were preserved directly in RNAlater. RNA was isolated from lungs using RNeasy plus universal Midi kit (Qiagen, Hilden, Germany) according to the manufacturer instructions. RNA elution was performed in two rounds with RNase-free water and stored at -20 °C for further analysis. The RNA quality was determined using a NanoDrop spectrophotometer (ThermoFisher scientific) and was reverse transcribed using a $RT^2$ First Strand Kit (Qiagen, Hilden, Germany). Afterwards, the cDNA was used on the real-time $RT^2$ Profiler PCR Array in combination with $RT^2$ SYBR® Green qPCR Mastermix.

For optimal performance, the arrays were customized to include: 84 genes and 5 house-keeping genes to be used for data normalization. For quality control, mouse genomic DNA contamination test, 3 reverse transcription efficiency tests and 3 PCR array reproducibility test were included. Results for expression of genes coding for cytokines and chemokines involved in moderate and severe COVID-19 are presented.

The following sequences were used:

| Gene | GeneBank accession | Uniprot accession | species |
|---|---|---|---|
| TNF | NM_013693 | P06804 | |
| IL-1b | NM_008361 | P10749 | |
| IP-10 | NM_021274 | P17515 | |

(continued)

| | | | |
|---|---|---|---|
| CXCL1 (KC)(IL8) | NM_008176 | P12850 | Mouse |
| MCP-1 | NM_011333 | P10148 | |
| MIP-1β | NM_013652 | P14097 | |

**Results:**

**Figure 8** shows that ATR-002 treatment of ALI mice leads to a decrease in the cytokines TNF-alpha, IL-1beta, IP-10, IL-8, MCP-1, and MIP-1beta. As discussed in the background section above, according to Huang et al., all of these cytokines are increased in COVID-19 patients, and TNF-alpha, IP-10 and MIP-1beta are increased in Stage III COVID-19 patients as compared to the levels upon hospitalization with Stage II COVID-19. This indicates that, independent of SARS-CoV-2 infection, ATR-002 is able to reduce cytokine and chemokine gene expression in mammals.

**Example 5: ATR-002 reduces the pro-inflammatory cytokine/chemokine response after SARS-CoV-2 infection in CaCo2 cells and Calu-3 cells**

CaCo2 cells were infected with SARS-CoV-2 and treated with ATR-002 as described in Example 1. Amounts of MCP-1 were measured and results are shown in **Figure 9.** Specifically, ATR-002 is able to reduce MCP-1 expression in SARS-CoV-2 infected cells. However, a high amount of ATR-002 is necessary for this, as discussed in **Example 1,** this is due to the constitutive activation of the MEK pathway in CaCo2 cells. Due to somatic driver mutation, high amounts of ATR-002 are required to inhibit MCP-1 expression in SARS-CoV-2 infected CaCo2 cells. We further addressed the question, if ATR-002 can decrease the expression of pro-inflammatory cytokines in Calu-3 cells. Therefore, we infected Calu-3 cells with SARS-CoV-2 and treated them with increasing amounts of ATR-002 as described in **Example 2.** Calu-3 cells were infected with SARS-CoV-2 (FI) using a MOI of 0.01. 1 h.p.i. cells were treated with ATR-002. Mock, SARS-CoV-2 and DMSO served as controls. Expression analysis of IL-6, IL-8, MCP-1, IP-10 and CCL5 mRNA by quantitative real-time PCR. Results are depicted in **Figure 10** as n-fold mRNA expression of mock infected cells. Data represent means ± SD of three independent experiments, each performed in triplicates. Data passed a one-way ANOVA test followed by Dunnett's multiple comparison test (* $p \leq 0.0332$; ** $p \leq 0.0021$; *** $p \leq 0{,}0002$; **** $p \leq 0.0001$) for each time point separately. DMSO was used as reference. The results confirm that ATR-002 can not only decrease the production of progeny viral particles, as shown in the previous examples, but furthermore, reduce the expression of pro-inflammatory cytokines. This is an additional benefit of the ATR-002 treatment, which reduces the possibility of a cytokine storm during a viral infection.

In a second step, to investigate a general effect of the ATR-002 treatment on the expression of anti-viral interferon and pro-inflammatory cytokines, A549 cells were transfected with polyI:C for 24 h. In parallel, cells were treated with increasing amounts of ATR-002. DMSO served as control. It was found that MEK inhibition by ATR-002 does not affect the IFN response but reduces proinflammatory IL-8 expression after poly (I:C) stimulation.

Specifically, human lung epithelial A549 cells ($2 \times 10^5$/ml) were transfected with 100 ng/ml poly (I:C) via Lipofectamine 2000 and treated with the MEK inhibitor ATR-002 at indicated concentrations for 24 hours. Transfection medium was replaced 6 hours post transfection. Unstimulated controls were also exposed to transfection medium containing Lipofectamine at equivalent concentrations of stimulated cells. Results of these experiments are shown in **Figure 11. Figure 11A** shows cell lysates for Western Blotting which were taken after 24 hours of inhibitor treatment and analyzed by immunoblotting using primary antibodies against pERK/ERK, pSTAT/STAT and Tubulin. Protein expression was detected by HRP-linked secondary antibodies and enhanced chemiluminescence. In **Figure 11B** RNA was isolated after 24 hours of inhibitor treatment using Qiagen Rneasy mini kit according to the supplemented protocol. RT-qPCRs were performed using the synthesized cDNA, SYBR Green and the corresponding primer pairs in a LightCycler 480 (Roche) to determine MxA and IL8 mRNA expression levels. Data represent means ± SD of three independent experiments, each performed in triplicates. Data passed an one-way ANOVA test followed by Dunnett's multiple comparison test (** $p \leq 0.0021$; *** $p \leq 0{,}0002$) for each time point separately. Mock was used as reference.

It was found that the ATR-002 treatment did not affect the stimulation of the anti-viral interferon system, as shown for the constant expression of MxA mRNA. In contrast, a strong reduction in the expression of pro-inflammatory IL8 was discovered, even with low concentrations of ATR-002.

**Example 6: ATR-002 reduces the pro-inflammatory cytokine/chemokine response in Peripheral Blood Mononuclear cells (PMBC)**

In order to check what concentration of ATR-002 would be necessary for cytokine reduction, the amounts of IP10, TNF-alpha and MCP-1 expression was studied in LPS induced PMBC cells after ATR-002 treatment. As can be seen from **Figure 12,** all three were reduced after treatment of 10μg/ml of ATR-002, indicating that 10μg/ml ATR-002 is sufficient to inhibit >90% of LPS induced MCP-1 in human PBMCs.

In further studies, the ability of the compound ATR-002 to inhibit Human T-cell Inflammation, as measured by the release of specific cytokines was studied. The compound was tested at three (3) concentrations (100, 50, and 25 μM), in biological triplicates, using peripheral blood mononuclear cells (PBMC's) from three (3) human donors, and an exposure time of 24 hours. Anti-CD3, ConA, and PHA were used, individually, as T-cell stimulants.

**Methods:**

**T-cell Inflammation Inhibition Assay Protocol (Anti-CD3 Stimulation)**

Day 1

- High binding plates were coated with anti-CD3 (clone UCHT-1, 100 ng/well) and isotype IgG1 using PBS 1x and incubated overnight at 4°C.

Day 2

- Cryopreserved PBMCs were drip thawed and diluted to the appropriate density and seeded into U bottom 96-well polypropylene plates (1.2x105 cells per well) with 228 μL per well of culture medium (RPMI 1640, 10% heat-inactivated FBS, 1%penicillin/streptomycin, 2 mM L-glutamine).
- Cells were incubated at 37°C, 5% CO2 for 1 hour prior to the addition of test compounds.
- Test compound was solubilized in PBS and further diluted to 20X with cell culture medium.

Test compound was added to the PBMCs in volumes of 12 μL (1X) in triplicate and incubated for 1 hour at 37°C, 5% CO2.

- For reference compound, 12 μL of Dexamethasone (100 nM) was added to the control wells according to the plate map and incubated for 1 hour at 37°C, 5% CO2.
- After 1 hour incubation, 200 μL of cells (1x105) treated with test compound or controls were transferred to the anti-CD3 coated plates and incubated for 24 hours at 37°C, 5% CO2.
- For positive and negative controls, 200 μL (1x105) of cells was transferred to the coated plate with anti-CD3 or isotype IgG1.
- Plates were centrifuged at 200 x g for 10 minutes. Cell culture supernatants were collected and stored at -80°C until needed for analysis.

**T-cell Inflammation Inhibition Assay Protocol (ConA and PHA Stimulation)**

- Cryopreserved PBMCs were drip thawed and diluted to the appropriate density and seeded into 96-well polystyrene plates (2 ×105 cells per well) with 150 μL per well of culture medium (RPMI 1640, 10% heat-inactivated FBS, 1% penicillin/streptomycin, 2 mM L-glutamine).
- Cells were incubated at 37°C, 5% CO2 for 1 hour prior to the addition of test compound.
- Test compound was solubilized in DMSO and further diluted to 20X with cell culture medium.

Compound was added to the PBMCs in volumes of 10 μL (1X) in triplicate and incubated for 1 hour at 37°C, 5% CO2.

- For reference compound, 10 μL of Dexamethasone (100 nM) was added to the control wells according to the plate

map and incubated for 1 hour at 37°C, 5% CO2.

- After 1 hour incubation, 40 μL of ConA, (30 μg/mL) and PHA (10 μg/mL) were added to the test compound or controls wells to give final assay volume of 200 μL. The plates were incubated for 24 hours at 37°C, 5% CO2.
- Controls were added to the PBMCs in volumes of 10 μL of diluted working stock, followed by addition of 10 μL of appropriately diluted vehicle, to mimic compound addition, to give final assay volumes of 200 μL. The final DMSO vehicle concentration in the assay was 0.1%.
- Following 24-hour incubation, plates were centrifuged at 200 x g for 10 minutes. Cell culture supernatants were collected and stored at -80°C until needed for analysis.

**Data Analysis and Analyte Limits of Detection**

**Cytokines/Chemokines Measured**

**Anti-CD3 Stimulation**

GM-CSF, IFNγ, IL-1β, IL-2, IL-6, IL-8, IL-10, IL-17A, MIP-1α, and TNFα

**ConA Stimulation**

GM-CSF, IFNγ, IL-1β, IL-1RA, IL-6, MIP-1α, and TNFα

**PHA Stimulation**

IFNγ, IL-1β, IL-2, IL-4, IL-5, IL-6, IL-8, IL-10, IL-13, MIP-1α, and TNFα

Luminex assessment of cytokine levels in cell culture supernatants, all diluted 1:20 in Luminex assay buffer, were performed per manufacturer's protocol using the Human Cytokine/Chemokine Magnetic bead panel from Millipore Sigma (catalogue No. HCYTOMAG-60K) with standards range 3.2, 16, 80, 400, 2000, 10,000 pg/mL.

Levels of induction of each cytokine were interpolated from a standard curve, using a 5-point nonlinear regression analysis, where $y = (A+((B-A)/(1+(((B-E)/(E-A))*((x/C)^D)))))$. The raw data in the form of median fluorescence unit was interpolated into test concentrations (pg/mL).

**Results:**

P - Values compared to stimulation (summarized)

| | PHA | | | | ConA | | | | anti-CD3 | | | |
|---|---|---|---|---|---|---|---|---|---|---|---|---|
| | Dexamethasone | 40µg/ml | 20µg/ml | 10µg/ml | Dexamethasone | 40µg/ml | 20µg/ml | 10µg/ml | Dexamethasone | 40µg/ml | 20µg/ml | 10µg/ml |
| **GM-CSF** | | | | | 0,0002 | 0,0001 | 0,0001 | 0,0028 | 0,0001 | 0,0001 | 0,0001 | 0,0001 |
| **IFNg** | 0,0001 | 0,0001 | 0,0001 | 0,0001 | 0,0001 | 0,0001 | 0,0001 | 0,0001 | 0,0001 | 0,0001 | 0,0001 | 0,0001 |
| **IL-10** | 0,0151 | 0,0195 | 0,709 | 0,946 | | | | | 0,0001 | 0,0001 | 0,0001 | 0,0001 |
| **IL-13** | 0,0008 | 0,0001 | 0,0181 | 0,4586 | | | | | | | | |
| **IL-17** | | | | | | | | | 0,0001 | 0,0001 | 0,0001 | 0,0001 |
| **IL-1RA** | | | | | 0,0001 | 0,0001 | 0,7069 | 0,999 | | | | |
| **IL-1ß** | 0,0023 | 0,0001 | 0,0151 | 0,466 | 0,003 | 0,0001 | 0,0001 | 0,0012 | 0,0001 | 0,0001 | 0,0001 | 0,0001 |
| **IL-2** | 0,0001 | 0,0001 | 0,0001 | 0,0001 | | | | | 0,0001 | 0,0001 | 0,0001 | 0,0001 |
| **IL-4** | 0,0001 | 0,0001 | 0,0194 | 0,5395 | | | | | | | | |
| **IL-5** | 0,0001 | 0,0001 | 0,0023 | 0,3291 | | | | | | | | |
| **IL-6** | 0,0302 | 0,002 | 0,0031 | 0,0588 | 0,0002 | 0,0001 | 0,0001 | 0,0001 | 0,0001 | 0,0001 | 0,0001 | 0,0001 |
| **IL-8** | 0,002 | 0,0003 | 0,0027 | 0,0745 | | | | | 0,0001 | 0,0001 | 0,0001 | 0,0001 |
| **MIP-1a** | 0,001 | 0,0001 | 0,007 | 0,2204 | 0,0001 | 0,0001 | 0,0001 | 0,687 | 0,0001 | 0,0001 | 0,0001 | 0,0001 |
| **TNFa** | 0,0002 | 0,0001 | 0,0001 | 0,0028 | 0,0012 | 0,0001 | 0,0004 | 0,0336 | 0,0001 | 0,0001 | 0,0001 | 0,0001 |

**Summary:**

ATR-002 was found to show significant, dose dependent inhibition of cytokine/chemokine stimulation with Anti-CD3 stimulation, ConA stimulation and PHA stimulation, as can be seen in **Figure 14.** Dexamethasone inhibition controls decreased cytokine /chemokine secretion confirming performance of the assay.

**Example 7:**

**RESPIRE** - A Randomized, Double-Blind, Placebo-Controlled, Clinical Study to Evaluate the Safety and Efficacy of ATR-002 in Adult Hospitalized Patients with Moderate Coronavirus Disease (COVID-19). The primary objective of the study is to demonstrate the efficacy of ATR-002 compared to placebo, each on standard of care, in the treatment of patients with COVID-19 assessed by the clinical severity status at day 15. ATR-002 has the potential to treat COVID-19 due to its Mode of Action with a dual benefit (effect) being (1) an antiviral agent, and (2) immunomodulation (to interfere with the cytokine storm). This clinical study will enroll a total of 200 adult hospitalized patients suffering from moderate coronavirus disease Stage II. A nasopharyngeal swab will be taken immediately prior to randomization to confirm presence of SARS-CoV-2 thereafter.

All randomized patients will be receiving standard of care as per local standards. 100 Patients will be randomized to receive ATR-002 900 mg orally once daily, 100 patients will be receiving matching Placebo. ATR-002 or placebo will be supplied in a controlled double-blind fashion for 5 days.

The primary objective of the study is to evaluate the efficacy of ATR-002 relative to placebo measured by the clinical severity status on a 7-point ordinal scale [1] Not hospitalized, without limitations, [2] Not hospitalized, with limitations, [3] Hospitalized, not requiring supplemental oxygen, [4] Hospitalized, requiring supplemental oxygen, [5] Hospitalized, on non-invasive ventilation or high flow oxygen devices, [6] Hospitalized, on invasive mechanical ventilation or ECMO, [7] Death. Secondary outcomes will be measured by clinical signs and symptoms, patient reported outcomes, Treatment Emergent Adverse Events, Serious Adverse Events, derived clinical parameters, scores and study events, changes in laboratory values and ATR-002 plasma levels, as well as changes in quantitative SARS-CoV-2 samples.

All patients will be followed up after end of study drug treatment until day 90 for safety reasons and to determine survival status.

The patients selected for the study are adults 18 years of age or older at screening and requiring hospitalization, showing clinical signs of a COVID-19 infection defined as having fever, defined as temperature ≥ 37.3 °C (axilla), ≥ 38.0 °C (oral), or ≥ 38.6 °C (rectal or tympanic) and $SpO_2$ <=94% on room air or requiring supplemental oxygen to maintain $SpO_2$>94%. The first patient was treated on April 14, 2021, however the results of the treatment are not yet available. However, the inventors are convinced that ATR-002 will prove effective in treating stage II COVID-19 patients and in preventing cytokine storm associated with the transition from stage II to stage III COVID-19.

**Example 8:**

**Efficacy of ATR-002 against SARS-COV-2 in a Syrian Hamster infection model**

The objective of this study was the investigation of the therapeutic efficacy of the compound ATR-002 after SARS-CoV-2 challenge in the hamster model. Animals were treated with a 100 mg/kg loading dose followed by 75mg/kg once daily. This refers to the human equivalent dose of 900 mg loading followed by 600mg once daily which was used in Example 7 above.

**Methods**

Prior to investigating the efficacy of ATR-002 in treating SARS-COV-2 in the hamster model, three different doses were tested by the oral route for which it was anticipated that with these doses, levels in circulation would be reached that inhibit the Raf/MEK/ERK signalling pathway by >80% and consequently are effective against in vivo infection with SARS-CoV-2. The results from this study were used to determine the dosing level and regimen in the efficacy study. Animals were infected by intranasal infection with $1\times10^3$ $TCID_{50}$ SARS-CoV-2 (amount of replication competent virus). Treatment regimen was defined as follows:

| Group | Number of Animals per Group | Treatment Regimen | Treatment regimen and Dose | | | |
|---|---|---|---|---|---|---|
| | | | Day 0 | Day 1 | Day 2 | Day 3 |
| 1 | 6 | Therapeutic compound 4 hours p.i. | a.m 100mg/kg | a.m. 75mg/kg | a.m. 75mg/kg | a.m. 75mg/kg |
| 2 | 6* | Therapeutic compound 24 hours p.i. | None | a.m 100mg/kg | a.m. 75mg/kg | a.m. 75mg/kg |

(continued)

| Group | Number of Animals per Group | Treatment Regimen | Treatment regimen and Dose | | | |
|---|---|---|---|---|---|---|
| | | | Day 0 | Day 1 | Day 2 | Day 3 |
| 3 | 6 | Therapeutic vehicle 4 hours p.i. | a.m. vehicle | a.m. vehicle | a.m. vehicle | a.m. vehicle |
| *one animal died of aspiration pneumonia so only 5 animals are shown in the results. | | | | | | |

Treatment of SARS-CoV-2-infected hamsters started either four hours or 24 hours post infection (p.i.) with a 100mg/kg loading dose of ATR-002 followed by a daily treatment with 75mg/kg ATR-002 until day 3 p.i. At day 3 p.i. throat swabs were taken to determine the amount of replication competent virus ($TCID_{50}$) At day 4 p.i. the animals were euthanized, and nasal turbinates were collected for quantification of infectious virus titers and lung lobes were inspected for affected areas.

**Animals**

| | |
|---|---|
| Species | Syrian hamster (*Mesocricetus auratus)* |
| Supplier | Janvier |
| Microbiological status | SPF |
| Number | 36 |
| Sex | Male |
| Age | ~7-9 weeks old at the start of the experiment |
| Body weight range | Approximately 100-150 gram |
| Identification | Animals were uniquely identified before start of the experiment with animal markers. |

**Animal Handling**

**Anesthesia**

For all animal procedures, the animals were sedated with isoflurane (3-4%/$O_2$).

**Per oral administration**

Oral administration of test items was performed by use of a gavage needle with the doses as described in a volume of 500 μl/150g. Therefore, animals were weighed and the dose volume used was adjusted depending on recorded bodyweight. Animals were then monitored during recovery.

**Intranasal administration**

For intranasal administration the animals were held on their back and the inoculum (100ul) was equally divided over both nostrils using a pipet. Animals were held on their back until the complete inoculum was inhaled after which they were placed back in the cage to recover.

**Clinical observations**

Observations were conducted and noted daily by the animal facility technicians, and daily following challenge by the laboratory technicians (Animal welfare and clinical observations). These include ruffled fur, hunched back posture, accelerated breathing and lethargy, and were noted down when observed.
Animals were weighed on regular time points during the study using electronic scales (internal individual scale number and performance will be documented on appropriate forms). Body weight was recorded on appropriate forms.

**Precautions**

The precautions to be taken was that of handling of animals, manipulation of sharps and working under BSL(DM)3 conditions (External preclinical facility manual).

**Sampling post inoculation**

The respiratory tract was sampled on regular time points during the study. In short, throat swabs were collected in virus transport medium, aliquoted and stored. Upon necropsy, tissue samples were collected and stored in 10% formalin for histopathology and frozen for virological analysis. For virological analysis, tissue samples were weighed, homogenized in infection medium and centrifuged briefly before titration.

**Blinding/bias-reducing methods**

All personnel performing the clinical observations and laboratory analysis in which interpretation of the data is required were not aware of the Random Treatment Allocation Key at any time prior to completion of the study and were blinded by allocating a unique sample number to each sample collected.

**Detection of replication competent virus**

Quadruplicate 10-fold serial dilutions were used to determine the virus titers in confluent layers of Vero E6 cells as described above (SARS-CoV-2 titration on Vero E6 cells). To this end, serial dilutions of the samples (throat swabs and tissue homogenates) were made and incubated on Vero E6 monolayers for 1 hour at 37 degrees. Vero E6 monolayers were washed and incubated for 4-6 days at 37 degrees after which plates were scored using WST8 (colorimetric assessment). Plates were measured for optical density at 450 mn (OD450) using a micro plate reader. Viral titers ($TCID_{50}$) were calculated using the method of Spearman-Karber.

**Detection of viral RNA**

Throat swabs and homogenized tissue samples were used to detect viral RNA. To this end RNA was isolated and Taqman PCR was performed using specific primers (E_Sarbeco_F: ACAGGTACGTTAATAG TTAATAGCGT and E_Sarbeco_R: ATATTGCAGCAGTACGCACACA) and probe (E_Sarbeco_P1: ACACTAGCCATCCTTACTGCGCTTCG) as described by Corman et al (Detection of 2019 novel coronavirus (2019-nCoV) by real-time RT-PCR. Euro Surveill. 2020;25(3). The number of virus copies in the different samples was calculated.

**Gross-pathology**

At the time of necropsy for all animals (either found dead post infection, euthanised due to reaching humane endpoint or at experimental endpoint), gross pathology was be performed on each animal and all abnormalities were described. All lung lobes were inspected, an estimation of the percentage affected lung tissue from the dorsal view was described, in addition, any other abnormalities observed in other organs during full body gross-pathology were also recorded.

Left lung lobes and nasal turbinates were preserved in 10% neutral buffered formalin for histopathology with the right side of these tissues subsequently homogenised and subjected to Taqman PCR and virus titration.

**Results:**

Throat swabs were collected on day 3 p.i. and analysed for viral load in a $TCID_{50}$ assay on Vero E6 cells and in addition with qPCR. ATR-002 treatment resulted in a reduction of progeny virus in throat swabs. A 1.1 log10 (P=0.0134) and 1.3 log10 (P=0.0191) reduction was noted in the groups that had been treated 4 hours p.i (Fig. 16A) and 24 hours p.i. (Fig. 16A) respectively. These results were supported by PCR demonstrating a 1.1 log10 (P=0.0089) and 1.2 log10 (P=0.0090) reduction (Fig. 16B).

A similar pattern was observed for the infectious virus load in the nasal turbinates at day 4 p.i. whereas the treatment started at 4 hours p.i. (Fig. 16C), but not the treatment started 24 hours p.i., significantly reduced infectious virus titers (Fig. 16C). The reduction in infectious virus titers (TCID50/g tissue) in the nasal turbinates of hamsters with a treatment start 4 hours p.i. was 1.3 log10 (P=0.0335) (Fig. 16C). Again, these data were confirmed by PCR. A 1.1 log10 (P=0.0145) reduction (Fig. 16D; red dots) was found when treatment started 4 hours p.i.

On day 4 p.i., lung lesions of infected animals were inspected and the percentage of affected lung area was

estimated. Each treatment resulted in a significantly reduced affected lung tissue (P=0.0282, P=0.0314, respectively) (Fig. 16E).

The treatment of both groups was well tolerated without significant weight loss (Fig. 16F) or any obvious adverse effects compared to the group received the vehicle.

## Histopathology

Histopathological analysis from selected tissues (lung and nasal turbinates) was performed for all animals either found dead, euthanized due to reaching humane endpoint or at experimental endpoint. After fixation with 10% formalin, sections from left lung and left nasal turbinate were embedded in paraffin and the tissue sections were stained by H&E for histological examination. Parameters studies were alveolitis severity, bronchitis/bronchiolitis severity, rhinitis severity, tracheitis severity: 0 = no inflammatory cells, 1 = few inflammatory cells, 2 = moderate number of inflammatory cells, 3 = many inflammatory cells, alveolitis extent, 0 = 0%, 1 = <25%, 2 = 25-50%, 3 = >50%, alveolar oedema presence, alveolar haemorrhage presence, type II pneumocyte hyperplasia presence, 0 = no, 1 = yes and extent of peribronchial/perivascular cuffing, 0 = none, 1 = 1-2 cells thick, 2 = 3-10 cells thick, 3 = >10 cells thick.

The results of the histopathology are shown in **Figure 23.** Histopathologic changes in hamsters at 4 days post infection, intranasally infected with SARS-CoV-2 and treated with zapnometinib at days 1, 2, and 3 post infection, or treated with the therapeutic vehicle only are shown in **Figure 23.** Hamsters treated with the therapeutic vehicle only had a score 3 in alveolitis showing a large number of inflammatory cells including macrophages and neutrophils in the alveolar septa and lumina, haemorrhage (arrow heads) and oedema (*) in the alveolar lumina and type II pneumocyte hyperplasia (arrows) (A) and (C). Hamsters treated with zapnometinib had a score 1 in alveolitis with less cellular inflammatory infiltrate of few macrophages and neutrophils, no haemorrhage, oedema and type II pneumocyte hyperplasia (B) and (D). Haematoxylin and eosin staining, 40x magnification (A and C) and 200x magnification (B and D).

## Summary

The antiviral efficacy of the human equivalent dose of ATR-002 was tested in the hamster model after SARS-CoV-2 challenge. Animals were treated on the day of infection or 24 hours after infection, and parameters like the percentage of lung lesions and viral load in throat swabs and nasal turbinate tissue were analysed on day 3 - 4 after challenge. ATR-002 treatment resulted in a significant reduction of virus titer and lung lesions in a SARS-CoV-2 Syrian hamster infection model. The chosen dosage of 100mg/kg for the first treatment followed by 75mg/kg once daily represents the hamster equivalent dose for a 900mg followed by 600mg human dose, which is used for the ATR-002 phase 2 clinical trial described in Example 7. Thus, the present results perfectly support the dose justification of the ATR-002 clinical trial.

## Example 9: Effect of ATR-002 on viral replication in primary human Air-Liquid Interference (ALI) cultures

ALI cell cultures were obtained from throat swabs of four healthy adults. Cells from swabs were expanded and then further cultivated on the apical side of the porous membrane of a transwell chamber inlay. After submerged growth to near confluency, medium was removed from the apical compartment to allow cell differentiation upon exposure to air. Three of the four ALI cultures could be infected with SARS-CoV-2 and were treated with ATR-002 using two different concentrations. ALI cells were infected with SARS-CoV-2 (FI) using a MOI of 1.0. 1 h.p.i. cells were treated with ATR-002 (50 μM, 100 μM). Untreated (SARS-CoV-2) and DMSO (0.1 %) treated cells served as negative controls. **Figure 17** shows results of one experiment. Both concentrations (50 μM and 100 μM) completely blocked the production of progeny viral particles in these three cultures, indicating that ATR-002 is very effective against a SARS-COV-2 infection in the ALI cultures.

## Example 10: Further studies on the role of MEK inhibitors in preventing SARS-CoV-2 infection

It is known that SARS-CoV-2 can enter the cell via ACE2/TMPRSS2. A possible effect of the expression of ACE2 and TMPRSS2 on the activation of the Raf/MEK/ERK signaling pathway was addressed in the next set of experiments. ACE2 and TMRPSS2 overexpressing A549 cells were infected with SARS-CoV-2 and the phosphorylation state of ERK was analyzed 1 h.p.i. A549 cells and Calu-3 cells served as negative and positive control, respectively.

Specifically, Calu-3 cells were infected with SARS-CoV-2 (FI) using a MOI of 2.0. Immunoblots were prepared 1 h.p.i. and probed with anti-pERK1/2, anti-ERK1/2 and anti-Tubulin antibodies. Shown in **Figure 18** are results of one out of three independent experiments. Mock infected cells served as negative control. The overexpression of ACE2 in A549 cells leads to an ERK activation during the SARS-CoV-2 infection.

After this, it was tested whether ACE2 expression is reduced in cells after ATR-002 treatment. Calu-3 cells

were treated with ATR-002 for 24 hours and the results were analysed via immunofluorescence microscopy and WES technology. Specifically, Calu-3 cells were seeded on glass cover slips in 24-well plates, $1\times10^5$ cells per well and incubated for 48h at 37°C, 5% $CO_2$. After washing the cells with medium (IMDM with 10% FBS and 1% P/S), 1mL of 100μM ATR-002 in medium or 1mL 1% DMSO in medium (solvent control) were added to the wells and incubated for 24h at 37°C, 5% $CO_2$.

Then the wells were washed once with PBS and fixed with 300μL/well 4% PFA in PBS for 10 minutes at room temperature. After another wash step with PBS, the cells were blocked for 1 hour with 1% BSA in PBS at room temperature before incubation with 200μL/well of the primary antibody (Goat-Anti-ACE2 antibody (R&D systems; Cat.: AF933; Lot.:HOK0320061) (1:20) in 1% BSA in PBS for 1h at room temperature on a shaker (100 rpm). Afterwards, the cells were washed three times with PBS before incubation with 200μL/well of the secondary antibody Donkey anti-Goat IgG (H+L) Highly Cross-Absorbed Secondary Antibody, Alexa Fluor Plus 647 (Invitrogen; Ref.: A32849TR; Lot.:VE306231) (1:200) in 1% BSA in PBS for 45 minutes at room temperature on a shaker. After another three wash steps with PBS, 200μL Rhodamine Phalloidin (Invitrogen; Ref.: R415; Lot.: 2157163) (1:40 in 1% BSA in PBS) were added to each well and incubated for 30 minutes at room temperature on a shaker. After two final wash steps with PBS, the cover slips were removed from the wells and mounted on microscope slides with Roti®-Mount FluorCare DAPI (Roth; Art.-Nr.: HP20.1; Lot.: 080293945). The slides were let dry at 4°C in the dark and then analysed using a confocal laser scanning microscope (LSM800, Zeiss).

Results can be seen in **Figure 19,** where it is clear that ATR-002 leads to a reduction of ACE2 on Calu-3 cells.

In a next step, it was tested whether ATR-002 can block the replication of SARS-CoV-2 in ACE2 overexpressing A549 cells. Therefore, A549-ACE2, A549-ACE2/TMPRSS2 or Calu-3 cells were infected with SARS-CoV-2 and treated with ATR-002 as described above.

Cell lines were infected with SARS-CoV-2 (FI) using a MOI of 0.001 or 0.01. 1 h.p.i. cells were treated with ATR-002. Untreated and DMSO-treated cells served as controls. Titers were analyzed after 8, 24, 48 and 72 h.p.i. Titer reduction of SARS-CoV-2 in different cell lines after ATR-002 (100 μM) treatment. Untreated (SARS-CoV-2) and DMSO (0.1 %) treated cells served as negative controls.

ATR-002 reduced the production of progeny viral particles in Calu-3 and A549-ACE2/TMPRSS2 cells, if cells were infected with a MOI of 0.001. Surprisingly, the ATR-002 treatment did not affect the production of progeny viral particles in A549-ACE2/TMPRSS2 cells when the concentration of the viral inoculum was ten times higher (MOI: 0.01). SARS-CoV-2 can enter the cell via two different pathways. Either via the direct activation of the spike protein by TMPRSS2, resulting in the fusion of viral and cellular membrane, or by the endosomal internalization and activation of the spike protein by cathepsin L.

Finally, to analyze whether a treatment with a MEK-inhibitor can reduce the expression of ACE2, Vero cells were pre-incubated with the MEK inhibitor CI-1040 or ATR-002 and were infected with the VSV pseudotype virus system carrying the SARS-CoV-2 Spike protein. Positive cells were analyzed by light microscopy. Specifically, Vero cells were incubated with 20 μM CI-1040 or 100 μM ATR-002 for 1 h, 2 h, 4 h and 24 h prior to a 1 h infection with VSV ΔG/GFP-Luc+SΔ21 (MOI 0.01). GFP-positive cells were analyzed 24 h p.i.. DMSO served as negative control and was set to 100 %. Data shown in Figures 21 and 22 represent means ± SD of three independent experiments for CI-1040 and ATR-002 pre-incubation, respectively. Data passed a two-way ANOVA test followed by a Sidak test (* $p\leq0.05$; ** $p\leq0.001$).

It was found that pre-incubation with CI-1040 or ATR-002 leads to a reduction in the internalization of the VSV pseudotype virus. This effect was time dependent. The longer the pre-incubation with CI-1040 or ATR-002, the lower the amount of GFP positive cells, which is the readout for the infection. These results indicate that MEK inhibitors such as CI-1040 and ATR-002 reduce the cellular expression of ACE2.

**Example 11: Determination of immune-modulating effects of MEK inhibitor ATR-002 during reactivation of memory T cells**

The MEK inhibitor of the present invention ATR-002 targets the intracellular Raf/MEK/ERK signaling pathway. Based on published literature it is thought that inhibition of the pathway will have effects on T cell priming but not on clonal expansion of primed T cells. ATR-002 has the potential to modulate the pro-inflammatory cytokine response of the body.

ATR-002 has been tested in different concentrations and timepoints on its *in vitro* effect during memory T cell reactivation. Within the scope of this project (non-GLP) an *in vitro* pilot study has been performed to show general feasibility of the chosen method approach using antigens with known vast immunogenic prevalence in healthy populations.

It has been performed a 12 days FluoroSpot T cell clonal expansion assay as described by Nelde et al. 2021. In the pilot study, three healthy blood donors (buffy coat) were used to reactivate memory T cells with a suitable peptide mix of high immunogenic prevalence in healthy population (CEF peptide mix) during a reactivation culture period of 12 days. During the course of this reactivation period, the MEK inhibitor ATR-002 was added at 3 different timepoints each

in 2 different concentrations. Following the 12 days culture period, evaluation using FluoroSpot was tested to quantify (un-)stimulated T cells (IFN-y) against the chosen peptide mix.

**Table 1: Definitions / abbreviations**

| ***Abbreviation*** | ***Description*** |
|---|---|
| CEF | Peptide mix including sequences from human Cytomegalovirus, Epstein-Barr Virus and Influenza Virus |
| CMV | Cytomegalovirus |
| IFN-γ | Interferon-gamma |
| IL-2 | Interleukin 2 |
| PBMC | Peripheral Blood Mononuclear Cell |
| PHA | Phytohaemagglutinin |
| γ PWM | Pokeweed mitogen |
| SD | Standard deviation |
| SOP | Standard operating procedure |
| TNTC | Too numerous to count |

**Table 2: Summary 12-day *in vitro* expansion.**

| ***Assay characteristic*** | ***Description*** |
|---|---|
| Cell source (PBMC) | Buffy coat; Immune Analytics cryobank |
| Test antigen | CEF peptide mix (1 μg/ml) |
| Drug substance tested | ATR-002 (5, 10 μg/ml; timepoints d1, d5, d8) |
| Cell culture | 12 days prior to FluoroSpot assessment, addition of 20 U/ml IL-2 on days 2, 6, 9 |
| Negative control (NC) | Blank (medium), DMSO (0.1 %; d1, d5, d8) |
| Positive control (PC) | PHA (1 μg/ml) |

**Table 3: Summary IFN-γ FluoroSpot.**

| ***Assay characteristic*** | ***Description*** |
|---|---|
| Cells / well | $2 \times 10^4$ |
| Test antigen | CEF peptide mix (1 μg/ml), CMV pp65 peptide mix |
| Negative control (NC) | Blank (medium) |
| Positive control (PC) | Pokeweed mitogen |
| Stimulation time | 20 h |
| Unit of measurement | Spot counts (Reader: AID *i*Spot ELR08IFL (ELR08IFL1903298), Software V8.0, build 6782.15365) |

Samples were provided by Immune Analytics. For the pilot study, PBMC isolated from three buffy coats of healthy donors were used.

**Reagents and material**

**Table 4: Material**

| *Material* | *Description* | *Manufacturer* | *Cat. no.* |
|---|---|---|---|
| AIM-V® medium CTS™ | Therapeutic Grade, serum-free, L-glutamine, 50 μg/ml streptomycin and 10 μg/ml gentamicin sulfate | Gibco | 0870122-DK --- |
| ATR-002 | Manufactured: 01.03.2021 m(stock) = 4,13 mg Diluted (11.03.2021) with DMSO c(stock) = 10 mg/ml | ChemCon GmbH | |
| CEF | c(end) = 1 μg/ml | GenID GmbH | ELSP5935 |
| CMV pp65 | --- | GenID GmbH | ELSP5940 |
| DMSO (Dimethyl sulfoxide) | $C_2H_6OS$; M= 78,13 g/mol; ≥99, 5 % | Sigma | D8418 |
| Fetal calf serum (FCS) | FCS Superior, standardized, tested for virus and mycoplasma, endotoxin, for in vitro use only | Sigma Aldrich | S0615 |
| Human IL-2 IS | Premium grade, Activity: ≥5×$10^6$IU/mg, diluted in ultra pure water (26.02.2021) c(stock) = 0,1 mg/ml | Miltenyi Biotec | 130-097-744 |
| IFN-γ FluoroSpot | --- | GenID GmbH | ELSP5800 |
| PHA | c(end) = 1 μg/ml | GenID GmbH | ELSP5901 |
| PWM | --- | GenID GmbH | ELSP5904 |
| Sterile heat-inactivated fetal calf serum (S-HI-FCS) | SOP 6.3-2.1.3 Preparation of sterile-filtered heat-inactivated fetal calf serum | GenID GmbH | --- |
| Thawing medium | 80% AIM-V, 20% S-HI-FCS | GenID GmbH | --- |
| Trypan Blue Solution 0.4% | --- | Gibco | 15250-061 |
| Ultra-Pure Water | Sterile, low endotoxin (<0.25 EU/ml) free of RNAse and DNAse | Biochrome | L0020 |

**_In vitro_ culture for the expansion of antigen specific memory T cells**

PBMC (1.5 × $10^6$ cells/ml in 24-well plates) from three healthy donors (buffy coat) were thawed from cryostocks, resuspended in AIM-V medium and rested over night before cells were stimulated with CEF peptide mix (1μg/ml) and cultivated for 12 days in AIM-V medium. IL-2 (20 U/ml) was supplemented in fresh culture medium (100 μl) on days 2, 6 and 9.

Cells were treated with ATR-002 dissolved in fresh culture medium (100 μl) on days 1, 5 or 8 (10 μg/ml or 5 μg/ml; Table ).

ATR-002 stock solution was prepared at 10 mg/ml in DMSO to ensure final DMSO concentrations ≤ 0.1 %.

For each PBMC donor, 12 preparations were set up according to Table .

**Table 5: Preparations for *in vitro* culture.** DMSO: addition of DMSO, final concentration 0.1 % (equivalent to amount max. of solvent added with ATR-002).

| *#* | *stimulation* | *day 1* | *day 5* | *day 8* |
|---|---|---|---|---|
| | --- | --- | --- | --- |
| 2 | PHA | --- | --- | --- |
| 3 | CEF Peptide Mix | --- | --- | --- |
| 4 | CEF Peptide Mix | DMSO | --- | --- |
| 5 | CEF Peptide Mix | --- | DMSO | |

(continued)

| # | *stimulation* | *day 1* | *day 5* | *day 8* |
|---|---|---|---|---|
| 6 | CEF Peptide Mix | --- | --- | DMSO |
| 7 | CEF Peptide Mix | 5 µg/ml ATR-002 | --- | --- |
| 8 | CEF Peptide Mix | --- | 5 µg/ml ATR-002 | |
| 9 | CEF Peptide Mix | --- | --- | 5 µg/ml ATR-002 |
| 10 | CEF Peptide Mix | 10 µg/ml ATR-002 | --- | --- |
| 11 | CEF Peptide Mix | --- | 10 µg/ml ATR-002 | --- |
| 12 | CEF Peptide Mix | --- | --- | 10 µg/ml ATR-002 |

On day 12, cells were washed twice with AIM-V medium prior to FluoroSpot analysis.

**Quantitative IFN-γ FluoroSpot for the detection of antigen specific T cells**

IFN-γ FluoroSpot was performed according to the manufacturer's manual. Samples were analyzed in triplicates (including negative control (medium) and positive control (pokeweed mitogen).

$2 \times 10^4$ cells/well were seeded in 96-well FluoroSpot plates for analysis and stimulated with 1 µg/ml CEF peptide mix for 20 h. In addition, cells were stimulated with CMV pp65 peptide mix in order to account for preparations were CEF stimulation results in a FluoroSpot signal that cannot be evaluated

(TNTC).

**DATA CAPTURE**

FluoroSpot plates were read by an AID *i*Spot Spectrum reader system (Software V8.0) and spots were counted using project-specific count settings according to Immune Analytics general SOPs. Results are expressed as mean of absolute spot counts.

The formulas used for the calculation of parameters are described in Table .

**Table 6: Formulas.**

| *Parameter* | *Formula* |
|---|---|
| Mean | |
| Standard deviation (SD) | $\sqrt{\frac{\sum(x_i - x_{mean})^2}{n-1}}$ |

**Evaluation of the *in vitro* culture by means of a microscope**

ATR-002 (or DMSO alone) had no visible effect on cell proliferation/density as far as this was observable by the microscope.

**Reference**

Nelde, A., Bilich, T., heimann, J.S. et al. SARS-CoV-2-derived peptides define heterologous and COVID-19-induced T cell recognition. Nat Immunol 22, 74-85 (2021).

The results are shown in **Figure 24.** Spot counts from three different donors evaluated in an IFN-y Fluorospot assay after a 12-day T cell reactivation period (clonal expansion) with CEF peptide stimulation. Application of ATR-002 (or DMSO solvent control) was tested with 5 or 10 pg/mL on three different time points followed by induction of clonal expansion with IL-2 24 hours after each treatment. **(A-C)** Average spot counts from each individual donor - mean values of three replicates are shown from which the spot counts from the blank negative control are subtracted. **(D)** Mean % clonal expansion summarized from all three donors, compared to DMSO solvent control set as 100%. Spot counts,

representing the number of activated T cells, are not reduced after treatment of stimulated PBMCs with ATR-002, regardless of the day of treatment or concentration.

## REFERENCES


Greenhalgh, T., Knight, M., Court, C., Buxton, M., & Husain, L. (2020). Management of post-acute Covid-19 in primary care. BMJ, 370:m3026. DOI: 10.1136/bmj.m3026

Haasbach, E. et al (2017). The MEK-inhibitor CI-1040 displays a broad anti-influenza virus activity in vitro and provides a prolonged treatment window compared to standard of care in vivo. Antiviral research 142, 178-184.

Hasan et al (2020). COVID-19 Illness in Native and Immunosuppressed States: A Clinical-Therapeutic Staging Proposal. J Heart Lung Transplant. 2020 Mar 20

Huang et al.; The Lancet; Vol 395; pp.: 497-506 February 15, 2020

Huang, C., Huang, L., Wang, Y., Li, X., Ren, L., Gu, X.,& Cao, B. (2021). 6-month consequences of Covid-19 patients discharged from hospital: a cohort study. Lancet, 397, 220-232. DOI: doi.org/10.1016/ S0140-6736(20)32656-8

Jamilloux et al.; Should we stimulate or suppress immune response in COVID-19? Cytokine and anti- cytokine interventions, Autoimmunity Reviews, AUTREV 102567 accepted 28 April 2020. Lopez-Leon, S., Wegman-Ostrosky, T., Perelman, C., Sepulveda, R., Rebolledo, P.A., Cuapio, A., & Villapol, S. (2021). More than 50 long-term effects of Covid-19: a systematic review and meta- analysis. medRxiv, 2021.01.27.21250617. DOI: https://doi.org/10.1101/2021.01.27.21250617 LoRusso, P., Adjei, A., Varterasian, M., Gadgeel, S., Reid, J., Mitchell, D., et al. (2005). Phase I and Pharmacodynamic Study of the Oral MEK Inhibitor CI-1040 in Patients With Advanced Malignancies. Journal of Clinical Oncology 23(23), 5281-5293.

Ludwig, S. (2009). Targeting cell signaling pathways to fight the flu: towards a paradigm change in anti-influenza therapy. Journal of Antimicrobial Chemotherapy, 64, 1-4.

Mackey, T.K., Liang, B.A., 2012. Lessons from SARS and H1N1/A: employing a WHO-WTO forum to promote optimal economic-public health pandemic response. J Public Health Policy 33, 119-130. Mahmud, R., Rahman, M., Rassel, M., Monayem, F., Sayeed, S., Islam, M. S., & Islam, M. M. (2021). Post-Covid-19 syndrom among symptomatic Covid-19 patients. A prospective cohort study in a tertiary care center of Bangladesh. PLOS ONE, 16(4). DOI: https://doi.org/10.1371/journal.pone.0249644

Pleschka, S., Wolff, T., Ehrhardt, C., Hobom, G., Planz, O., Rapp, U.R., Ludwig, S., 2001. Influenza virus propagation is impaired by inhibition of the Raf/MEK/ERK signalling cascade. Nat Cell Biol 3, 301-305. Townsend, L., Dowds, J., O'Brien, K., Sheill, G., Dyer, A., O'Kelly, B., ... & Bannan, C. (2021). Persistent poor health post-Covid-19 is not associated with respiratory complications or initial severity. AnnalsATS. DOI: https://doi.org/10.1513/AnnalsATS.202009-11750C

Wabnitz, A., Mitchell, D, and Wabnitz, D. (2004). In Vitro and in Vivo Metabolism of the Anti-Cancer Agent CI-1040, a MEK Inhibitor, in Rat, Monkey, and Human. Pharmaceutical Research 21(9), 1670-1679.


## Claims

1. MEK inhibitor for use in a method of treatment or prevention of a disease caused by a coronavirus in a human subject, wherein the disease comprises a long COVID syndrome.

2. The MEK inhibitor for the use of claim 1, wherein the coronavirus is SARS-CoV, SARS-CoV-2 or MERS.

3. The MEK inhibitor for the use of claim 2, wherein the coronavirus is SARS-CoV-2 and the human subject is suffering from long COVID syndrome associated with COVID-19.

4. The MEK inhibitor for the use of any one of claims 1 to 3, wherein the MEK inhibitor is selected from the group consisting of PD-0184264, CI-1040, GSK-1120212, GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof.

5. The MEK inhibitor for the use of claim 4, wherein the MEK inhibitor is PD-0184264 or a pharmaceutically acceptable salt thereof, wherein PD-0184264 or pharmaceutically acceptable salt thereof is preferably administered to the human subject once daily in a dose of 100 to 1000 mg, preferably 300 to 900 mg, most preferably 300, 600 or 900 mg.

6. The MEK inhibitor for the use of any one of claims 1 to 5, wherein the human subject is suffering from long COVID syndrome associated with COVID-19 caused by SARS-CoV-2.

7. The MEK inhibitor for the use of claim 6, wherein the SARS-CoV-2 is a variant, preferably selected from the group consisting of D614G, B.1.1.7, B.1.351, P1, P2, B.1.617, B.1.427, B.1.429, B.1.525 or B.1.526.

8. The MEK inhibitor for the use of any one of claims 5 to 7, wherein the MEK inhibitor PD-0184264 is administered to the human subject after a period of about four weeks after an acute, symptomatic or asymptomatic coronavirus infection on 1 to 28 consecutive days, preferably 5 to 18 or 7 to 14 consecutive days, starting with onset of clinical symptoms.

9. The MEK inhibitor for the use of any one of claims 5 to 8, wherein the MEK inhibitor PD-0184264 is administered to the human subject in an oral dosage form.

10. The MEK inhibitor for the use of any one of claims 1 to 9, wherein the human subject is suffering from a long COVID syndrome comprising any one or more of the following symptoms fatigue, headaches, lung damage, shortness of breath, cough, inflammatory reactions, neurological disorders, heart palpitations, vertigo, anosmia (loss of smell), parosmia (distorted smell), insomnia, muscle weakness, low fever and/or cognitive dysfunction.

11. A MEK inhibitor for use in treating or preventing long COVID syndrome associated with COVID-19 in a subject infected by SARS-CoV-2 by improving disease progression.

12. The MEK inhibitor for use of claim 11, wherein improving disease progression comprises one or more, two or more, three or more, four or more, five or more, six or more, or all seven of the following reducing inflammation, reducing tissue damage, achieving immune modulation, reducing viral persistence, improving T cell responses, enhancing clonal expansion of T cells and enhancing IFN-gamma producing T cells.

13. The MEK inhibitor for use of claim 11 or 12, wherein the MEK inhibitor is preferably selected from the group consisting of PD-0184264, CI-1040, GSK-1120212, GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof.

14. The MEK inhibitor for use of any one of claims 11 to 13, wherein the SARS-CoV-2 is a variant, preferably selected from the group consisting of D614G, B.1.351, B.1.1.7, P1, P2, B.1.617, B.1.427, B.1.429, B.1.525 and B.1.526.

15. A pharmaceutical composition comprising the MEK inhibitor for the use of any one of claims 1 to 14.

Figure 1

A

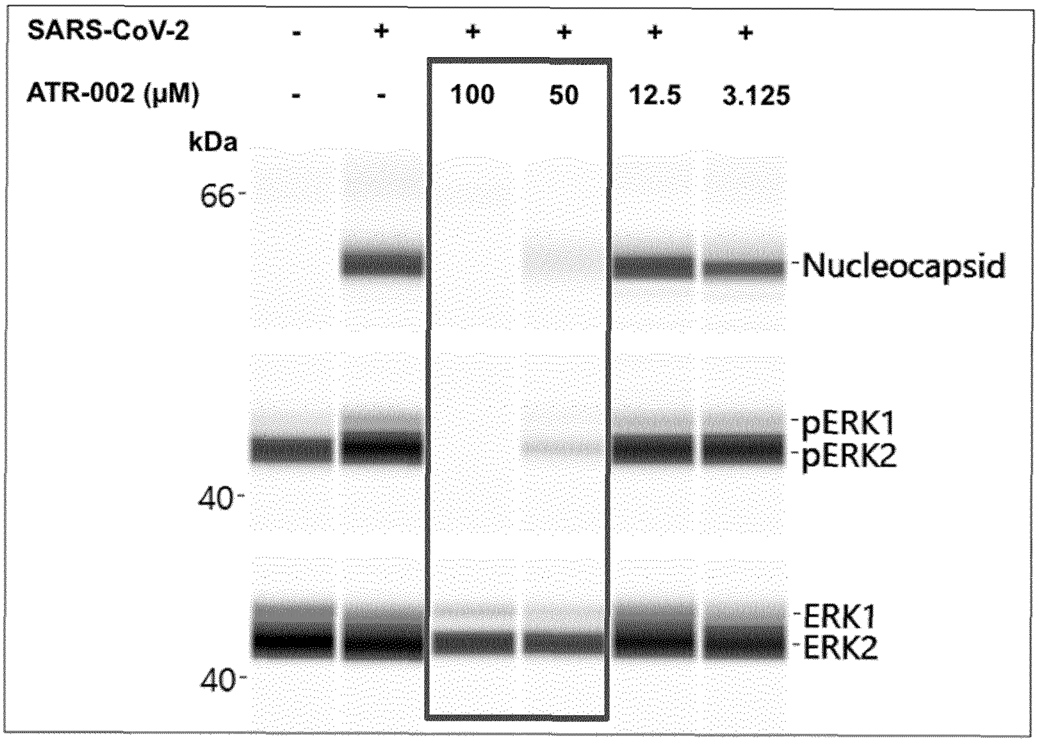
SARS-CoV-2
-
+
+
+
+
+
ATR-002 (µM)
-
-
100
50
12.5
3.125
kDa
66
40
40
Nucleocapsid
pERK1
pERK2
ERK1
ERK2

B

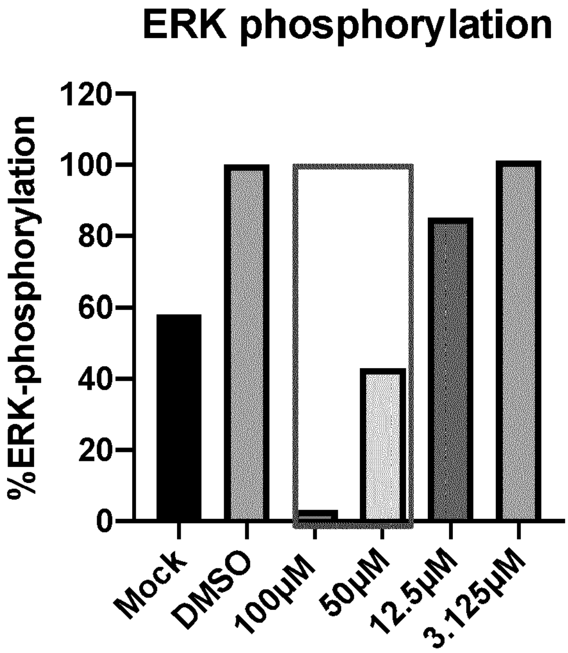
ERK phosphorylation
%ERK-phosphorylation
120
100
80
60
40
20
0
Mock
DMSO
100µM
50µM
12.5µM
3.125µM

C

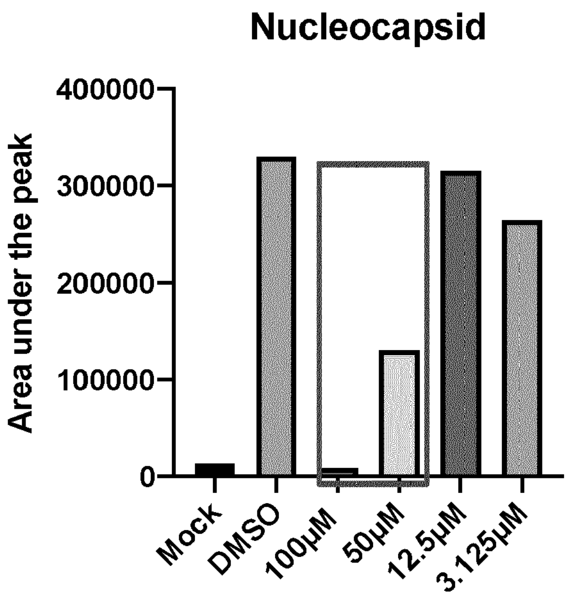
Nucleocapsid
Area under the peak
400000
300000
200000
100000
0
Mock
DMSO
100µM
50µM
12.5µM
3.125µM

Figure 1 continued

D

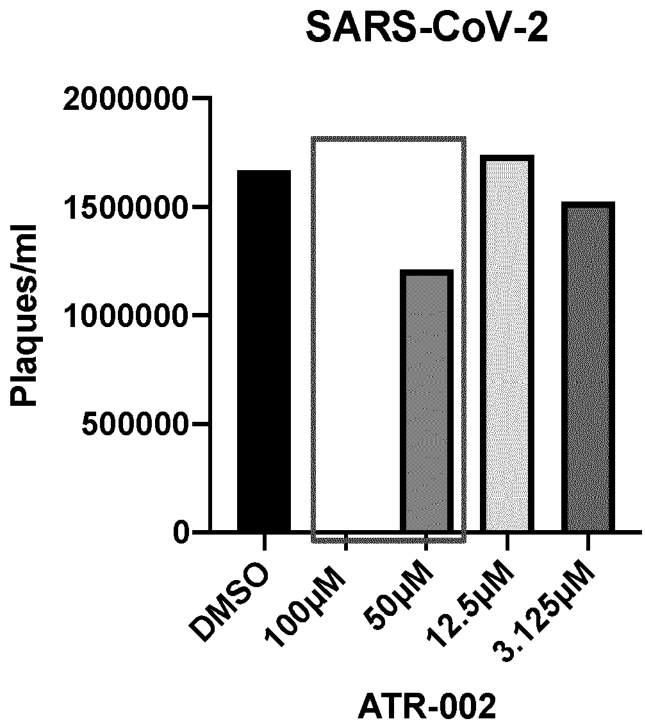

Figure 2

A

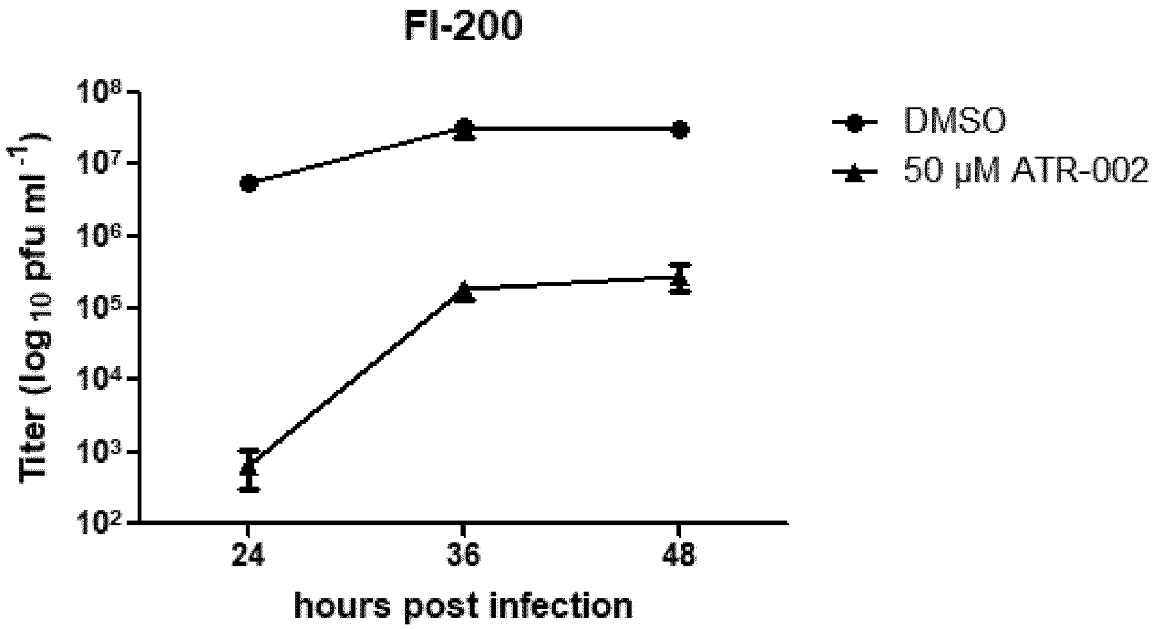
FI-200
$10^8$
$10^7$
$10^6$
$10^5$
$10^4$
$10^3$
$10^2$
Titer ($\log_{10}$ pfu $ml^{-1}$)
24
36
48
hours post infection
DMSO
50 µM ATR-002

B

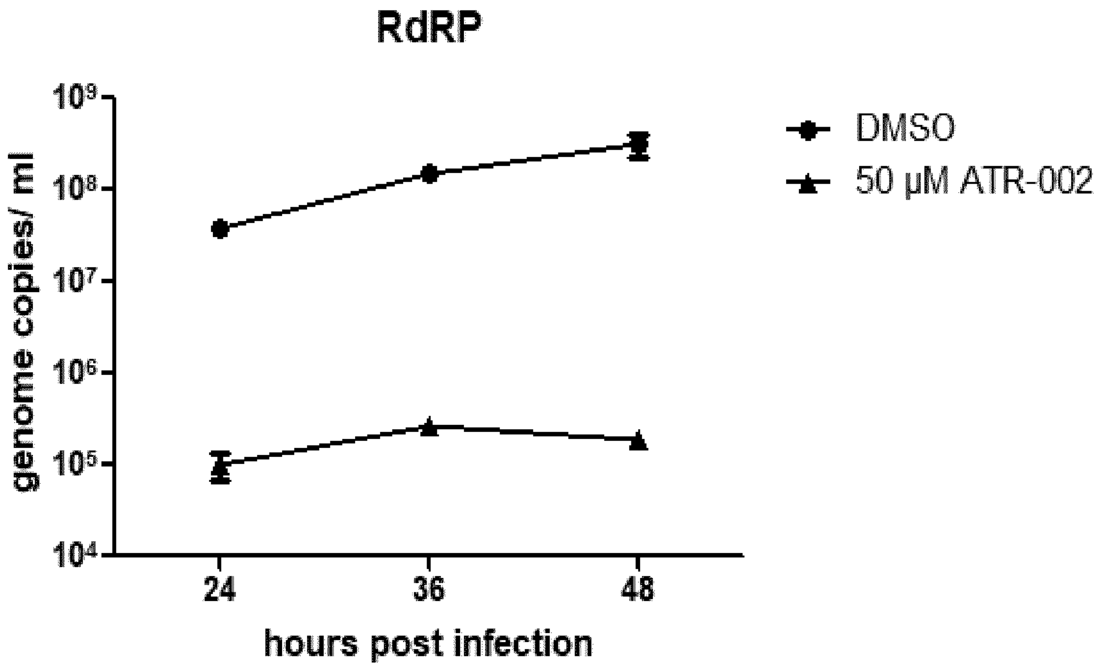
RdRP
$10^9$
$10^8$
$10^7$
$10^6$
$10^5$
$10^4$
genome copies/ ml
24
36
48
hours post infection
DMSO
50 µM ATR-002

Figure 3

A

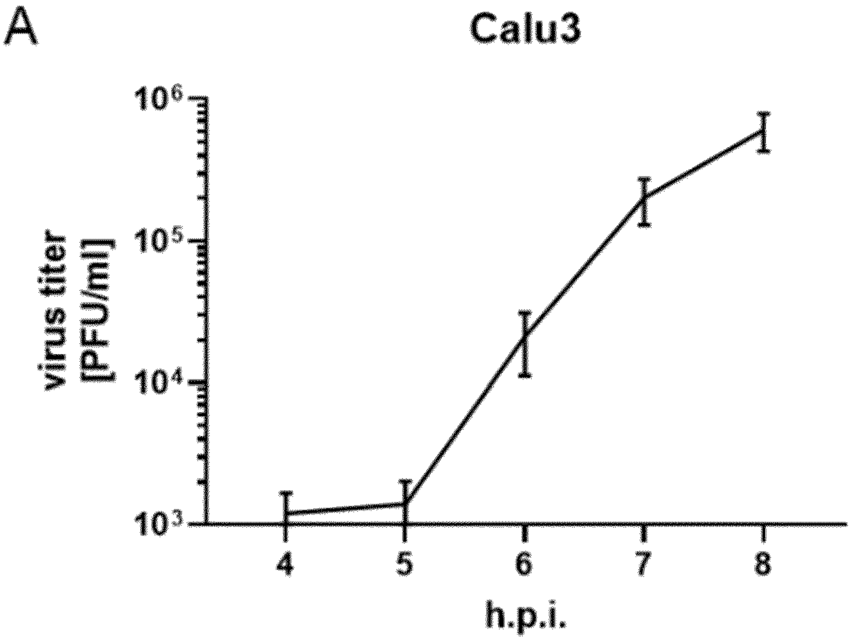

Figure 3 Continued

B

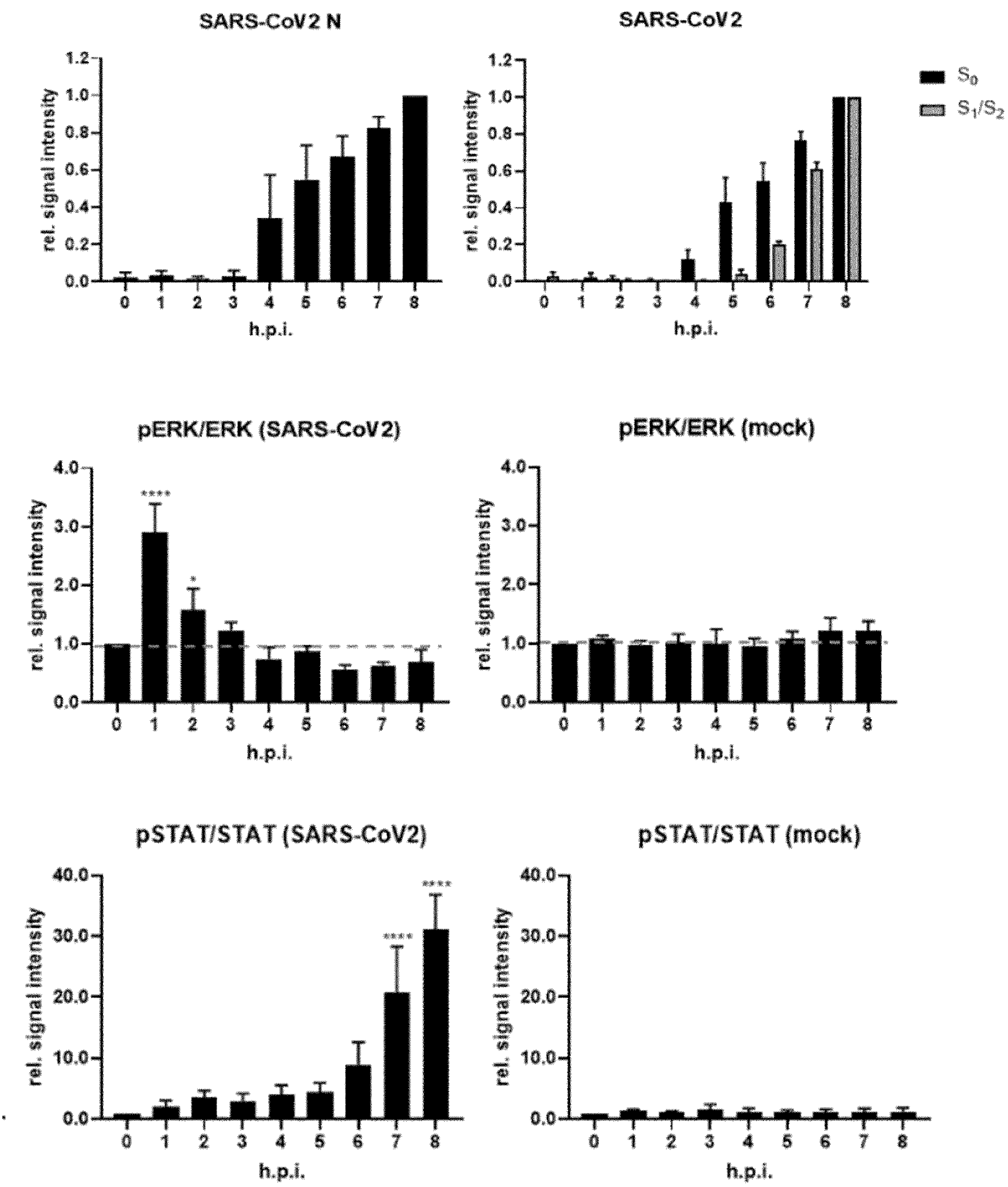

**Figure 3 continued**

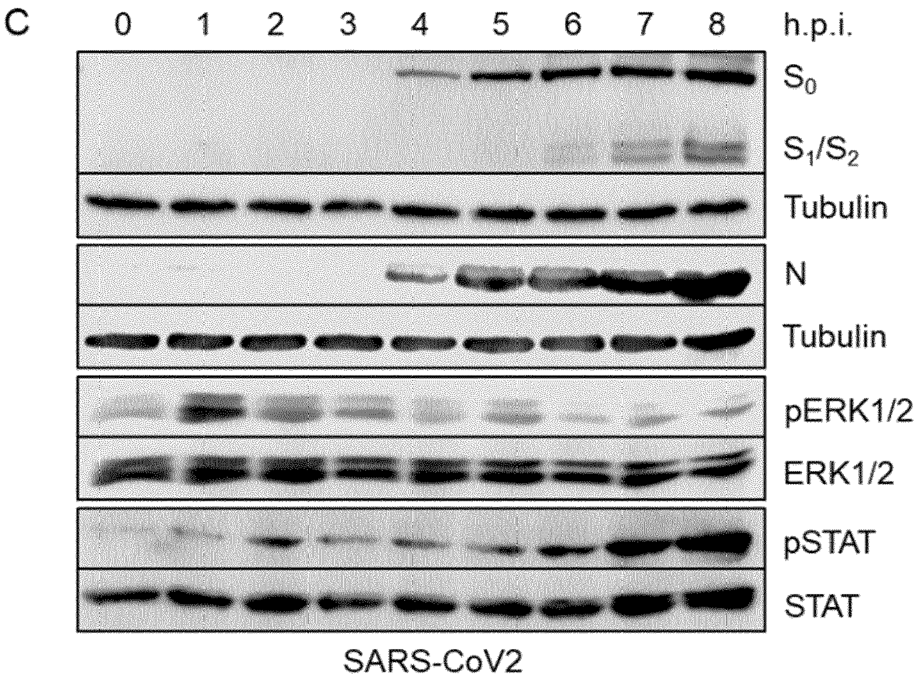


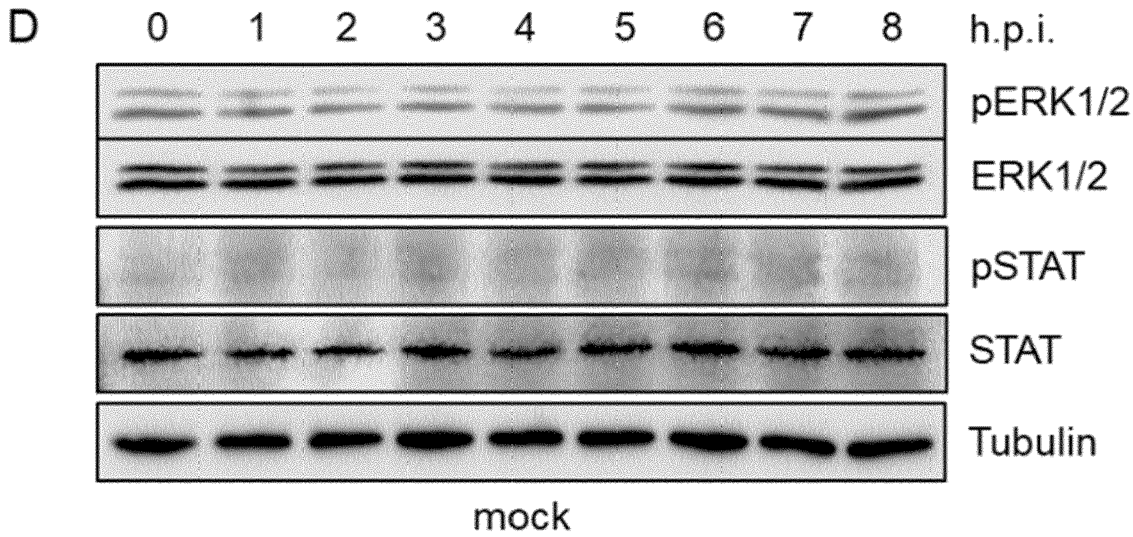

Figure 3 continued

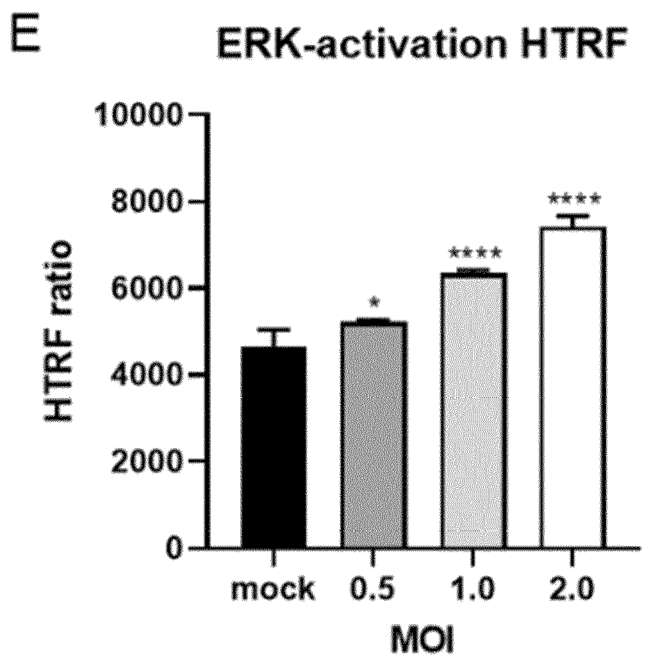

Figure 4

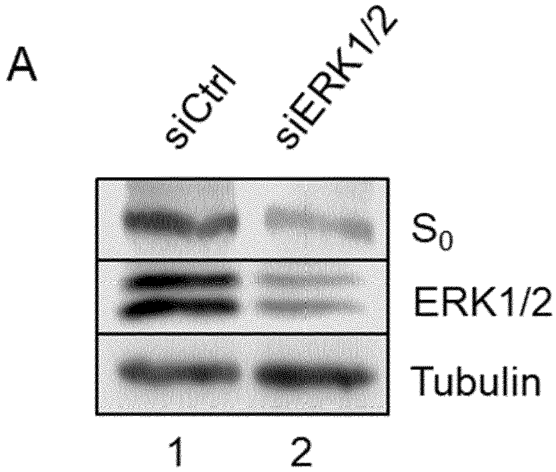
A
siCtrl
siERK1/2
$S_0$
ERK1/2
Tubulin
1
2

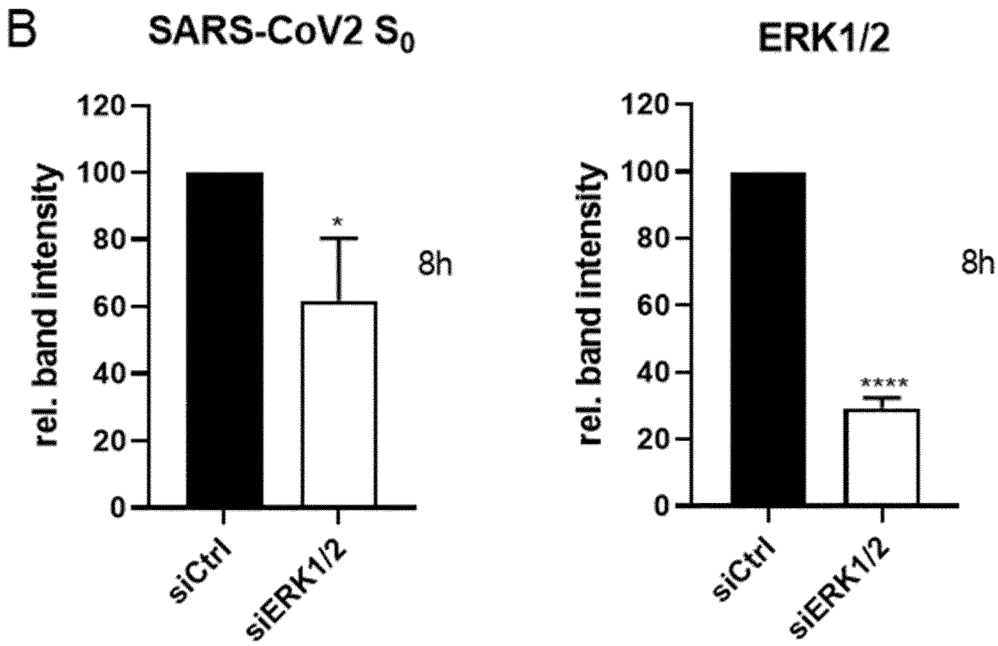
B
SARS-CoV2 $S_0$
rel. band intensity
120
100
80
60
40
20
0
*
8h
siCtrl
siERK1/2
ERK1/2
rel. band intensity
120
100
80
60
40
20
0
****
8h
siCtrl
siERK1/2

**Figure 4 Continued**

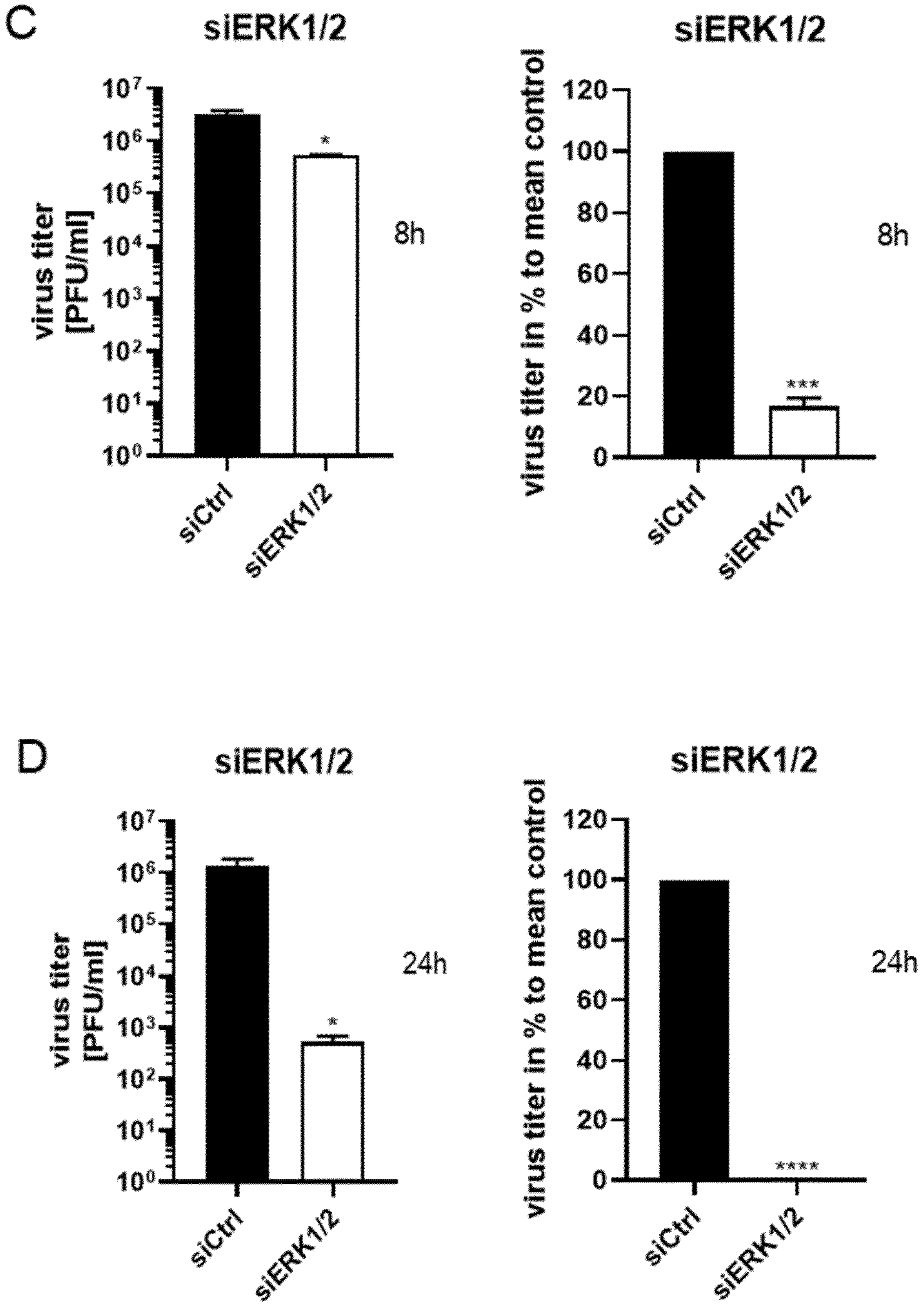

Figure 5

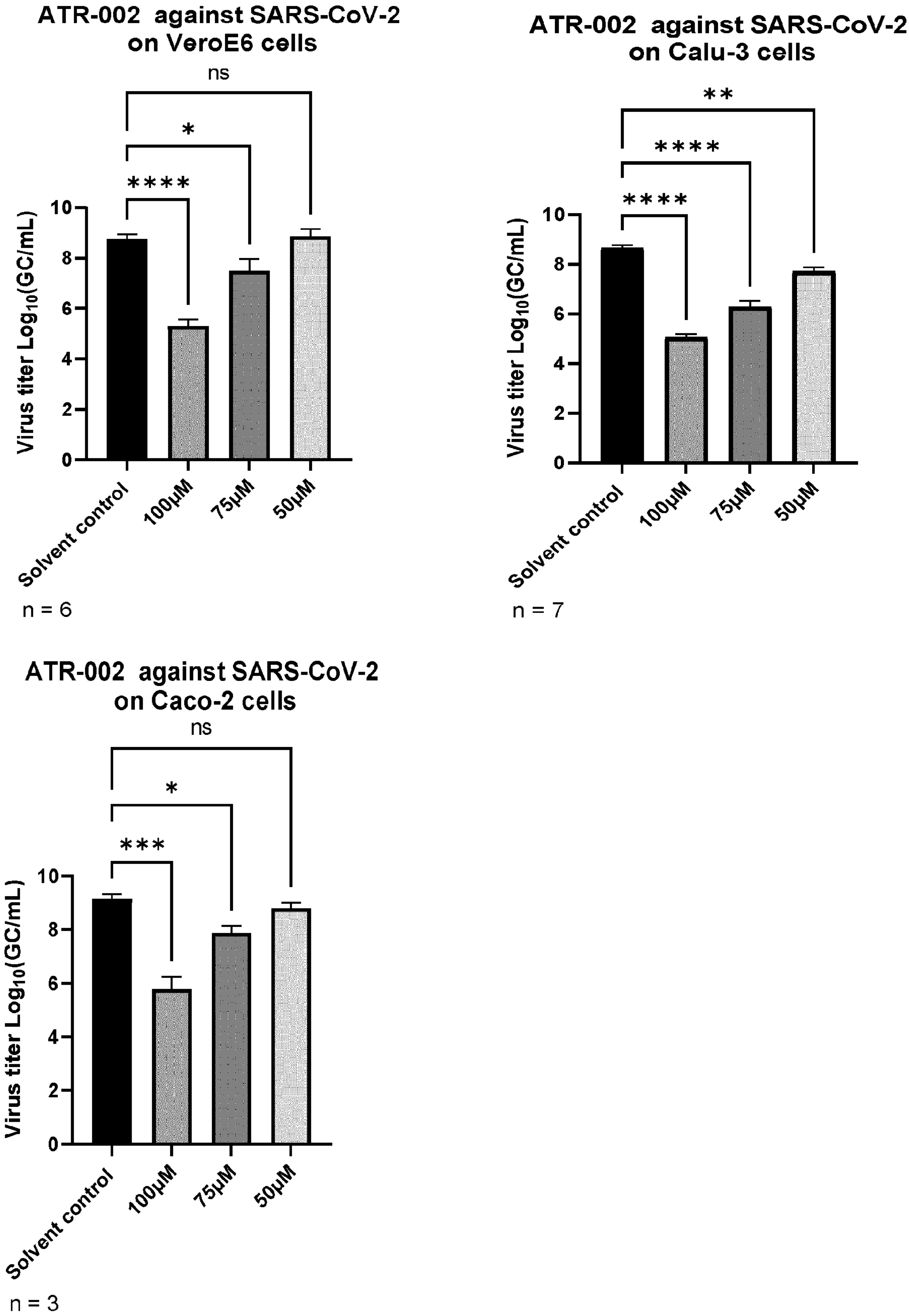
ATR-002 against SARS-CoV-2
on VeroE6 cells
ns
*
****
Virus titer $Log_{10}$(GC/mL)
10
8
6
4
2
0
Solvent control
100µM
75µM
50µM
n = 6
ATR-002 against SARS-CoV-2
on Calu-3 cells
**
****
****
Virus titer $Log_{10}$(GC/mL)
10
8
6
4
2
0
Solvent control
100µM
75µM
50µM
n = 7
ATR-002 against SARS-CoV-2
on Caco-2 cells
ns
*
***
Virus titer $Log_{10}$(GC/mL)
10
8
6
4
2
0
Solvent control
100µM
75µM
50µM
n = 3

Figure 6

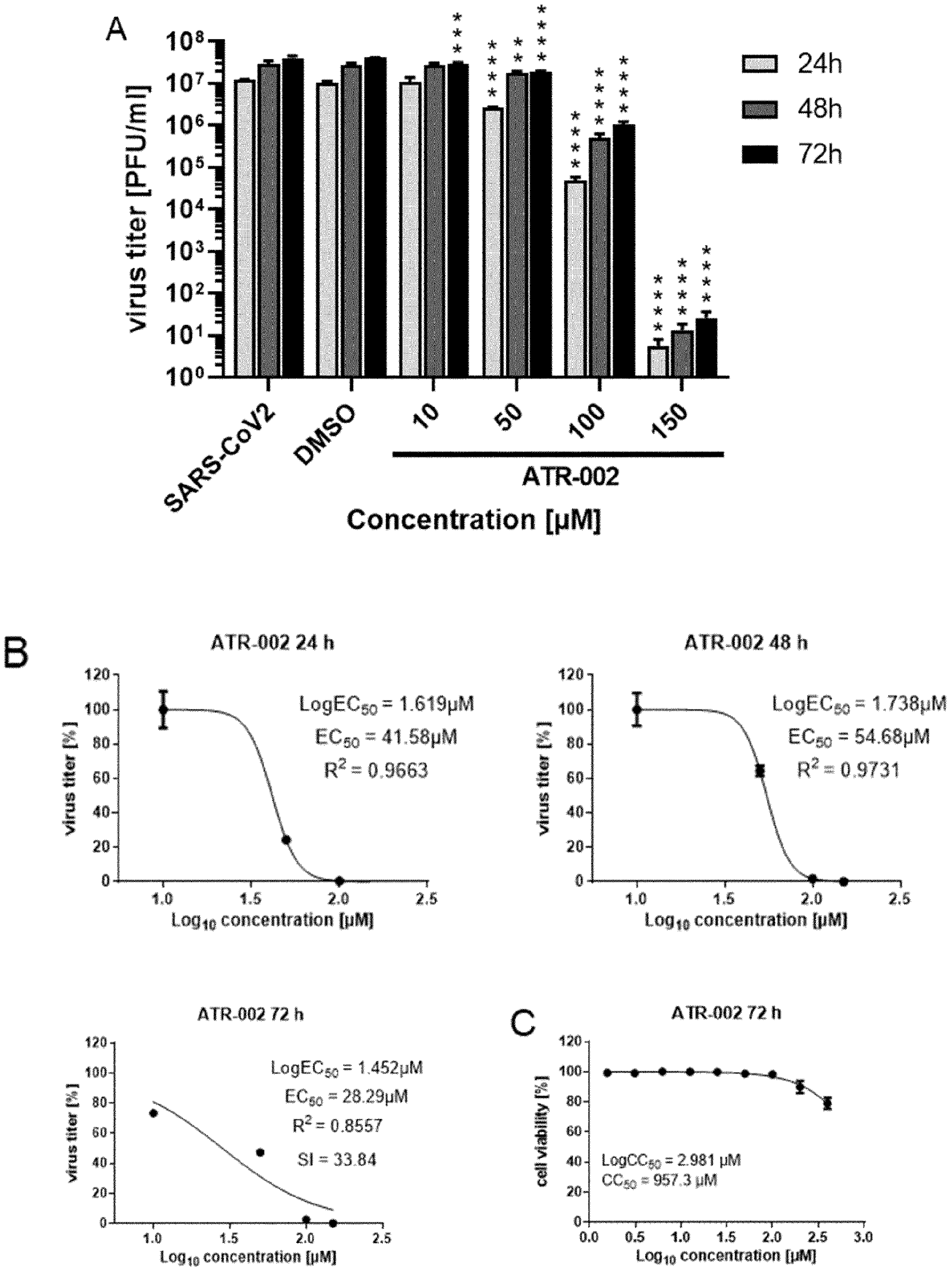
A
virus titer [PFU/ml]
24h
48h
72h
SARS-CoV2
DMSO
10
50
100
150
ATR-002
Concentration [µM]
B
ATR-002 24 h
LogEC50 = 1.619µM
EC50 = 41.58µM
R2 = 0.9663
virus titer [%]
Log10 concentration [µM]
ATR-002 48 h
LogEC50 = 1.738µM
EC50 = 54.68µM
R2 = 0.9731
ATR-002 72 h
LogEC50 = 1.452µM
EC50 = 28.29µM
R2 = 0.8557
SI = 33.84
C
ATR-002 72 h
cell viability [%]
LogCC50 = 2.981 µM
CC50 = 957.3 µM
Log10 concentration [µM]

Figure 6 continued

D

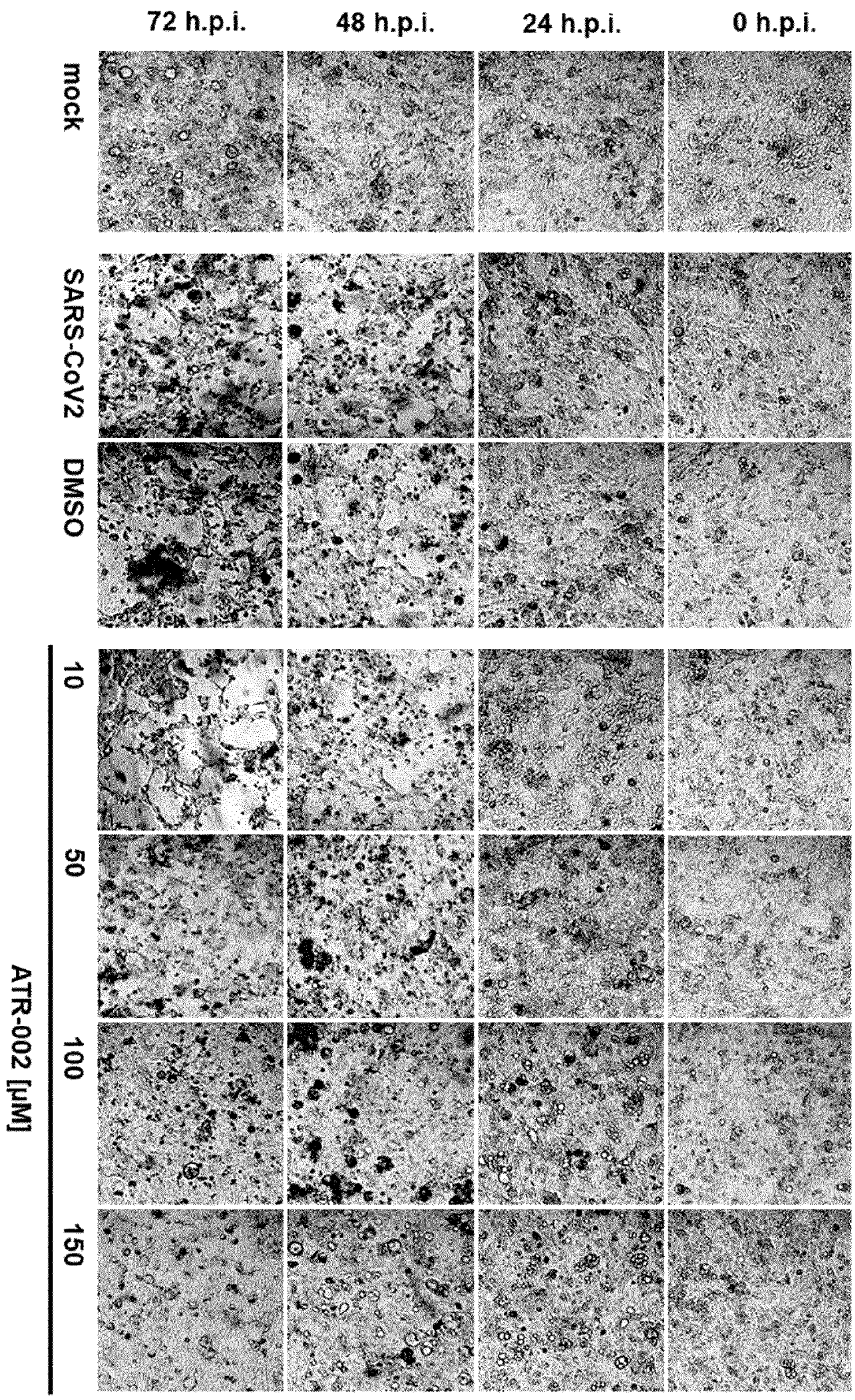

Figure 7

**ATR-002 Treatment of Vero E6 cells infected with SARS-CoV-2 SA MOI 0.1**

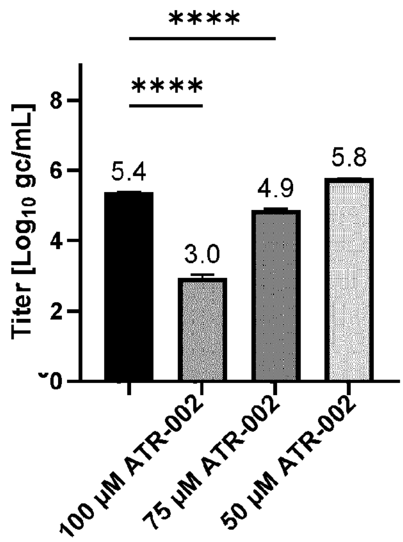


**ATR-002 Treatment of Vero E6 cells infected with SARS-CoV-2 SA MOI 1**

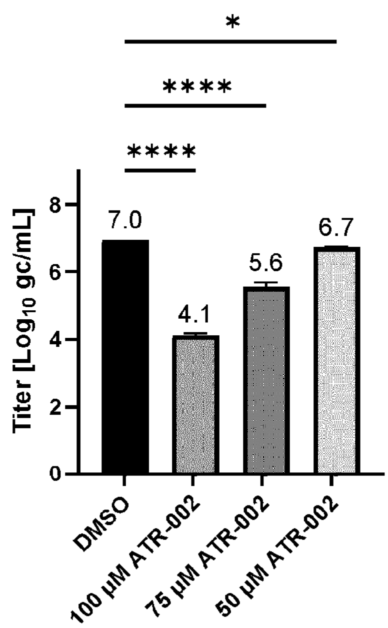

Figure 7 continued

ATR-002 Treatment of Vero E6 cells infected with SARS-CoV-2 SA MOI 10

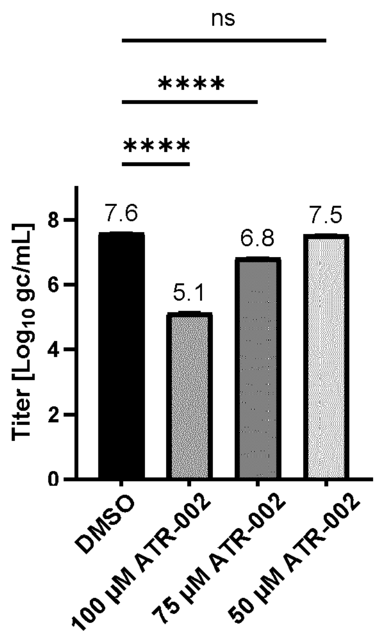


ATR-002 Treatment of Calu-3 cells infected with SARS-CoV-2 SA MOI 0.1

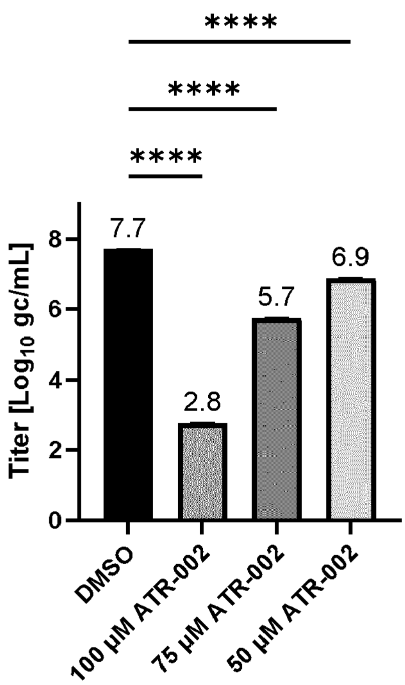

Figure 7 continued

## ATR-002 Treatment of Calu-3 cells infected with SARS-CoV-2 SA MOI 1

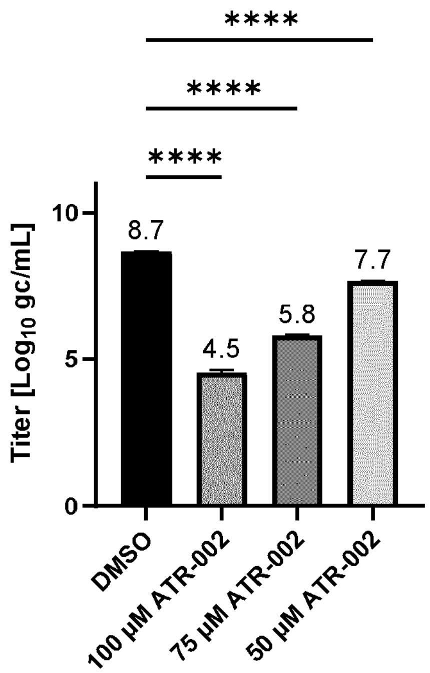


## ATR-002 Treatment of Calu-3 cells infected with SARS-CoV-2 SA MOI 10

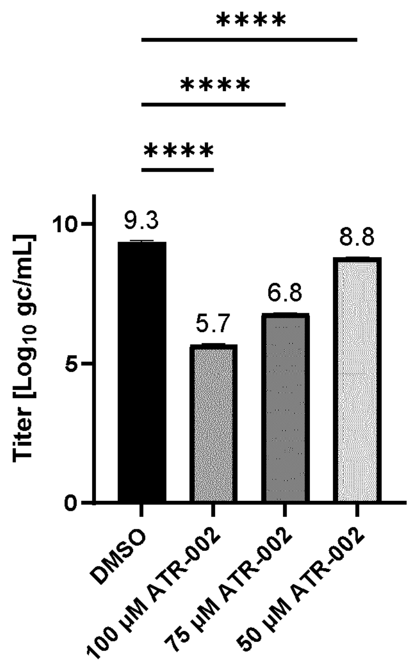

Figure 8

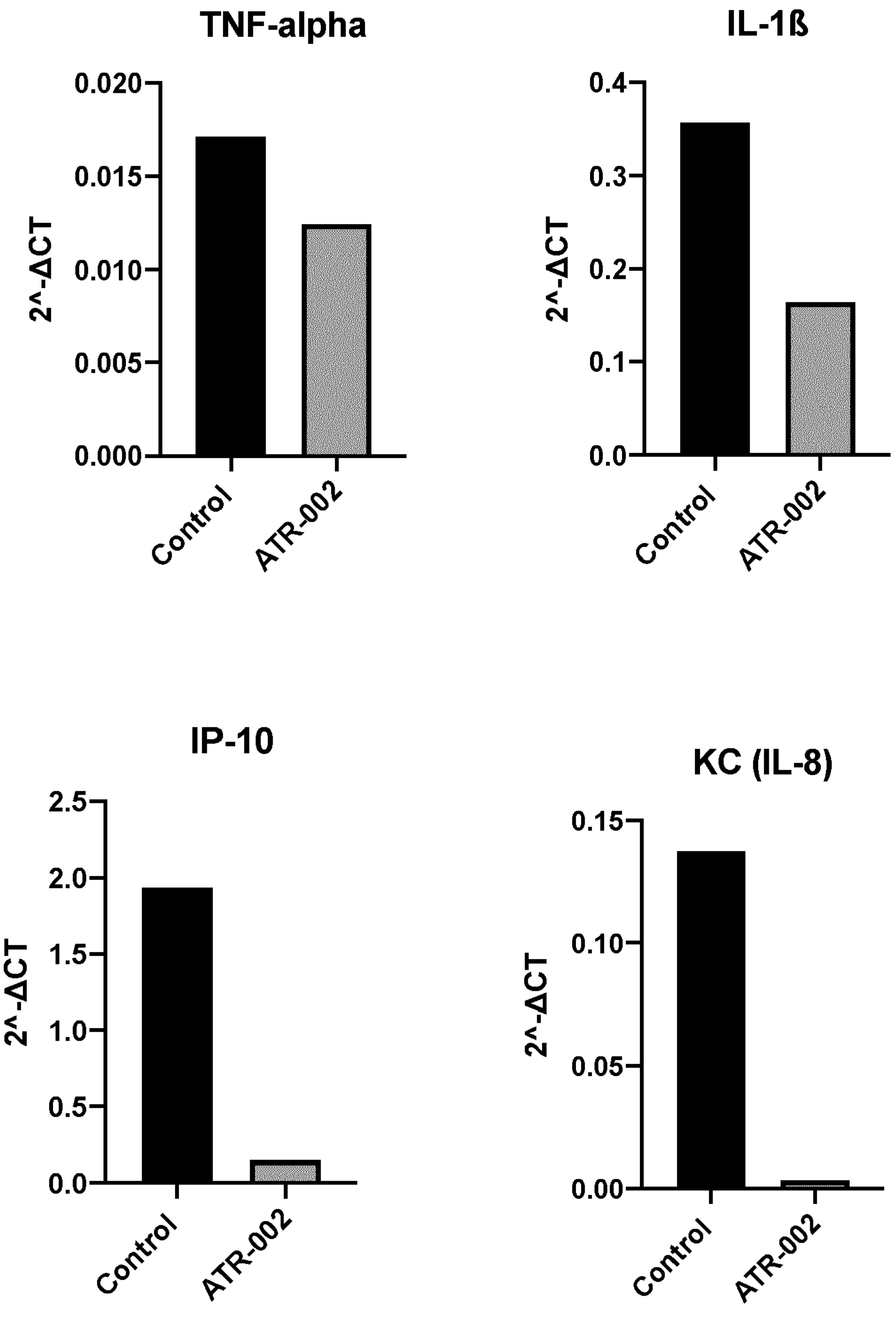
TNF-alpha
2^-ΔCT
0.020
0.015
0.010
0.005
0.000
Control
ATR-002
IL-1ß
2^-ΔCT
0.4
0.3
0.2
0.1
0.0
Control
ATR-002
IP-10
2^-ΔCT
2.5
2.0
1.5
1.0
0.5
0.0
Control
ATR-002
KC (IL-8)
2^-ΔCT
0.15
0.10
0.05
0.00
Control
ATR-002

Figure 8 continued

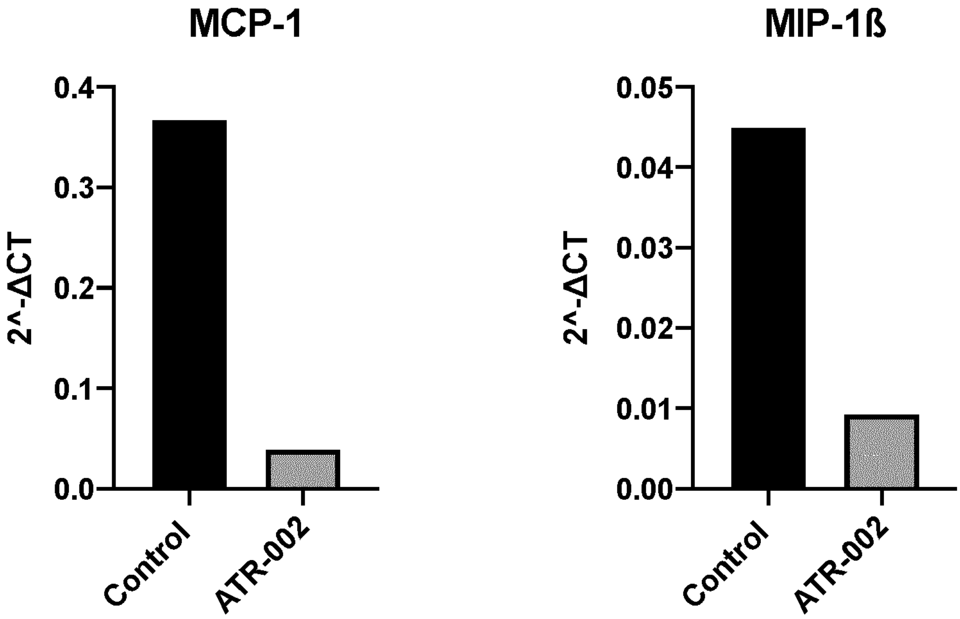

Figure 9

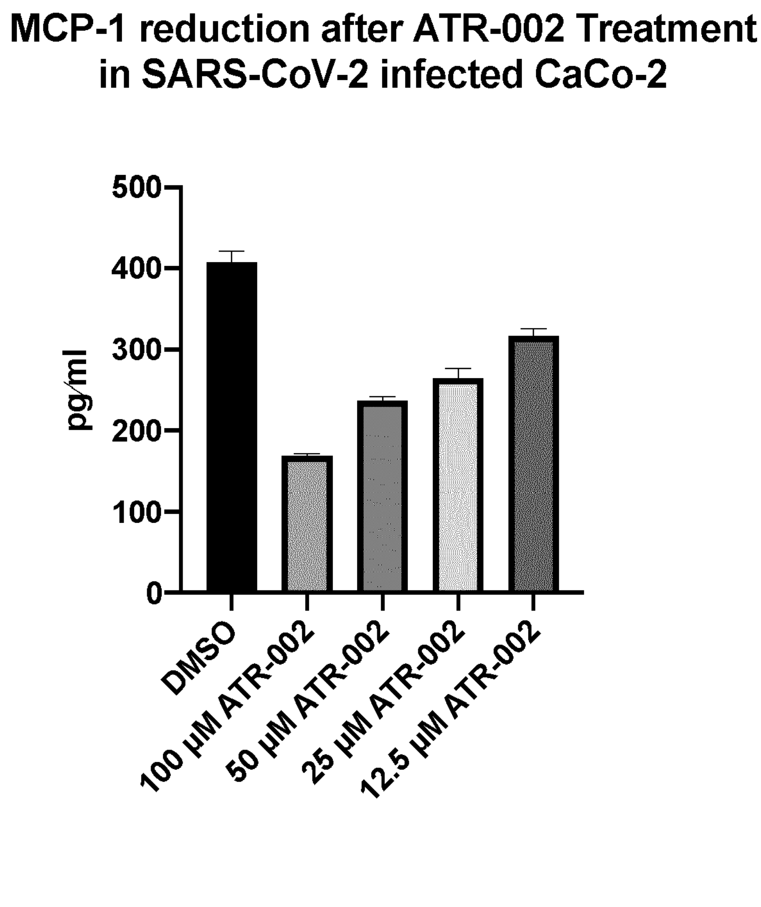
MCP-1 reduction after ATR-002 Treatment
in SARS-CoV-2 infected CaCo-2
pg/ml
500
400
300
200
100
0
DMSO
100 µM ATR-002
50 µM ATR-002
25 µM ATR-002
12.5 µM ATR-002

Figure 10

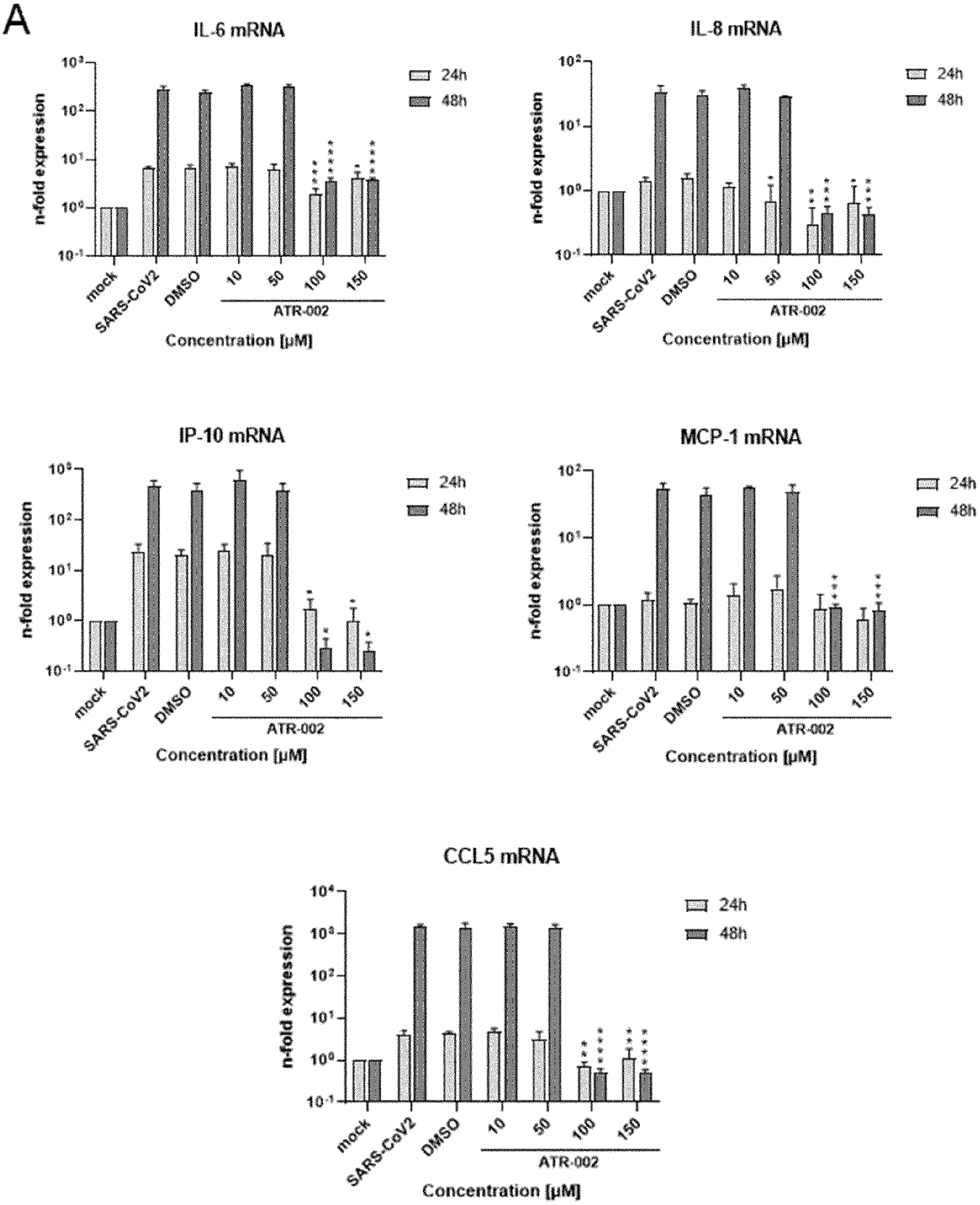
A
IL-6 mRNA
IL-8 mRNA
IP-10 mRNA
MCP-1 mRNA
CCL5 mRNA
n-fold expression
24h
48h
mock
SARS-CoV2
DMSO
10
50
100
150
ATR-002
Concentration [µM]

**Figure 10 continued**

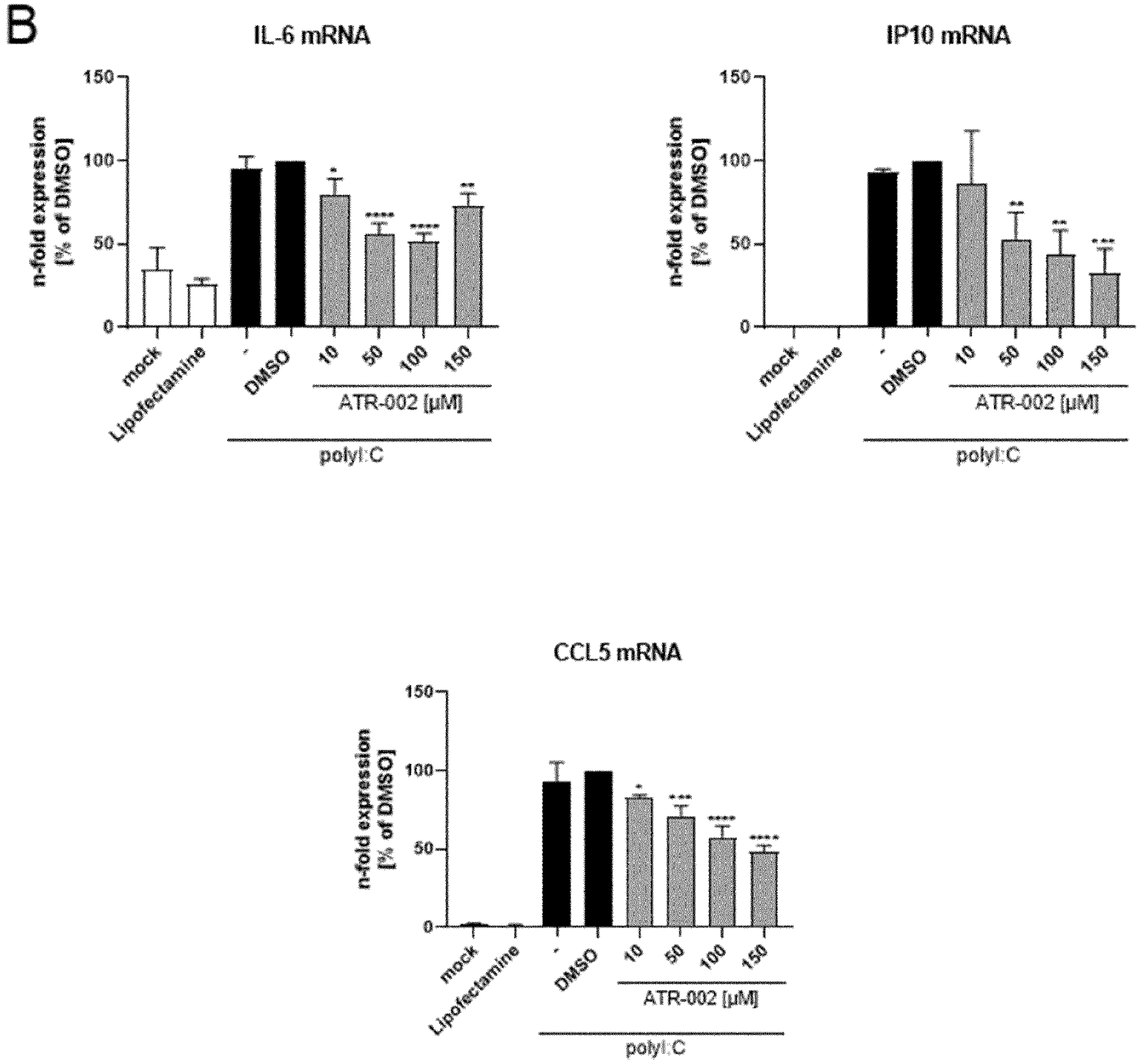

Figure 11

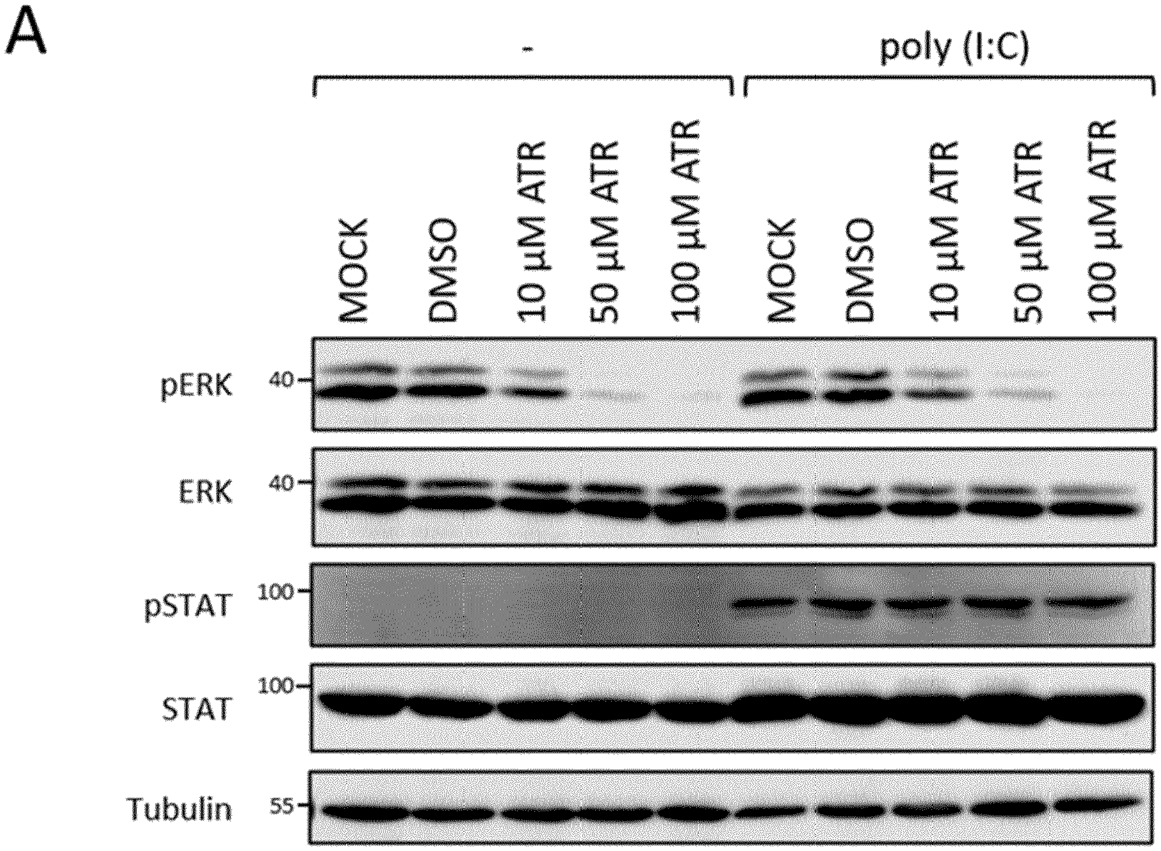
A
-
poly (I:C)
MOCK
DMSO
10 µM ATR
50 µM ATR
100 µM ATR
MOCK
DMSO
10 µM ATR
50 µM ATR
100 µM ATR
pERK
40
ERK
40
pSTAT
100
STAT
100
Tubulin
55

Figure 11 continued

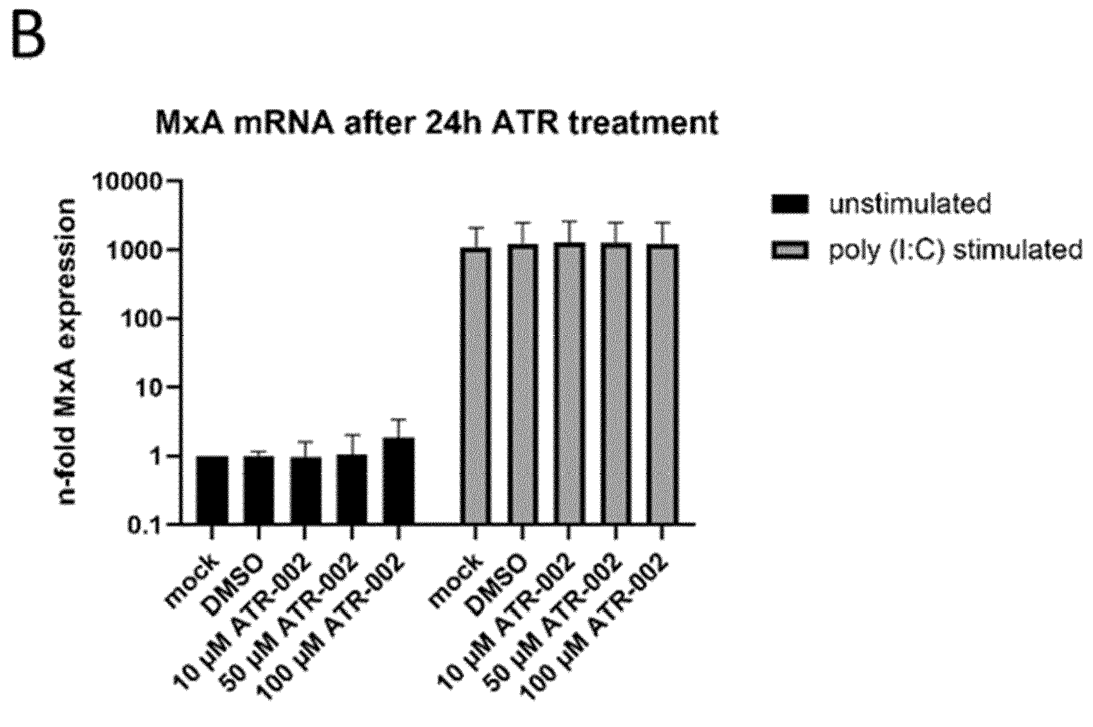


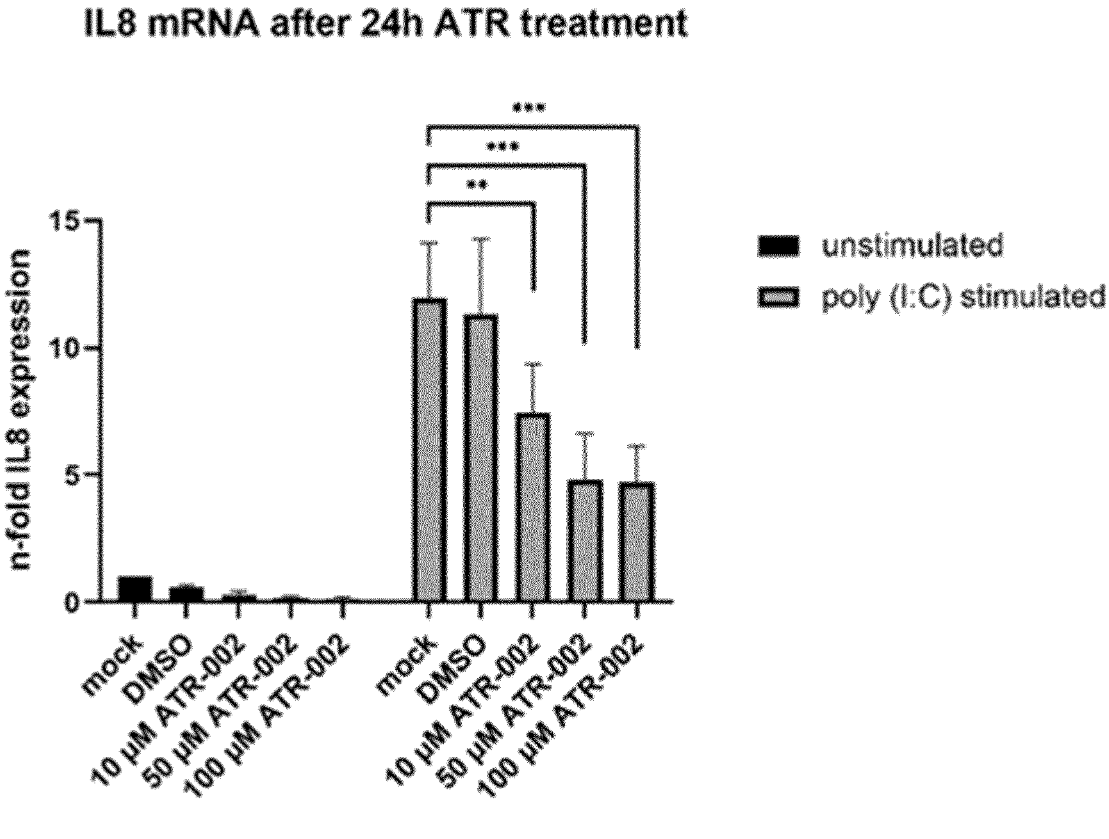

Figure 12

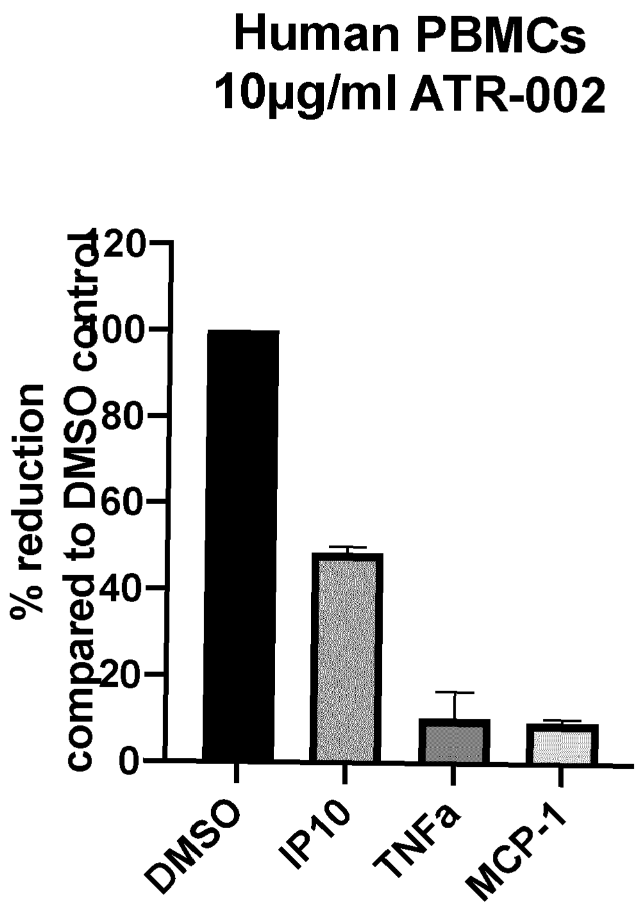
Human PBMCs
10µg/ml ATR-002
% reduction
compared to DMSO control
120
100
80
60
40
20
0
DMSO
IP10
TNFa
MCP-1

Figure 13

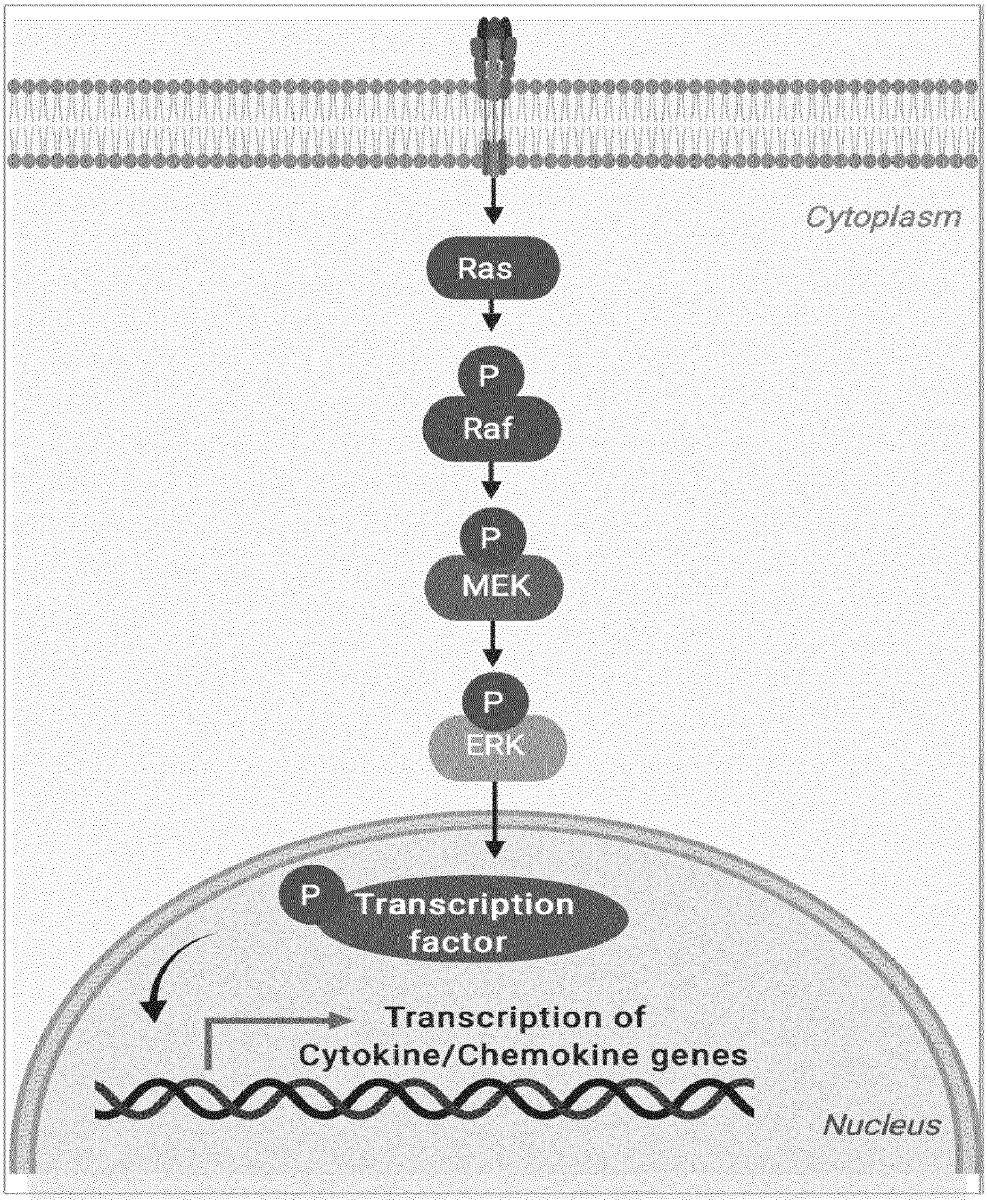
Cytoplasm
Ras
P
Raf
P
MEK
P
ERK
P
Transcription factor
Transcription of Cytokine/Chemokine genes
Nucleus

Figure 13 continued

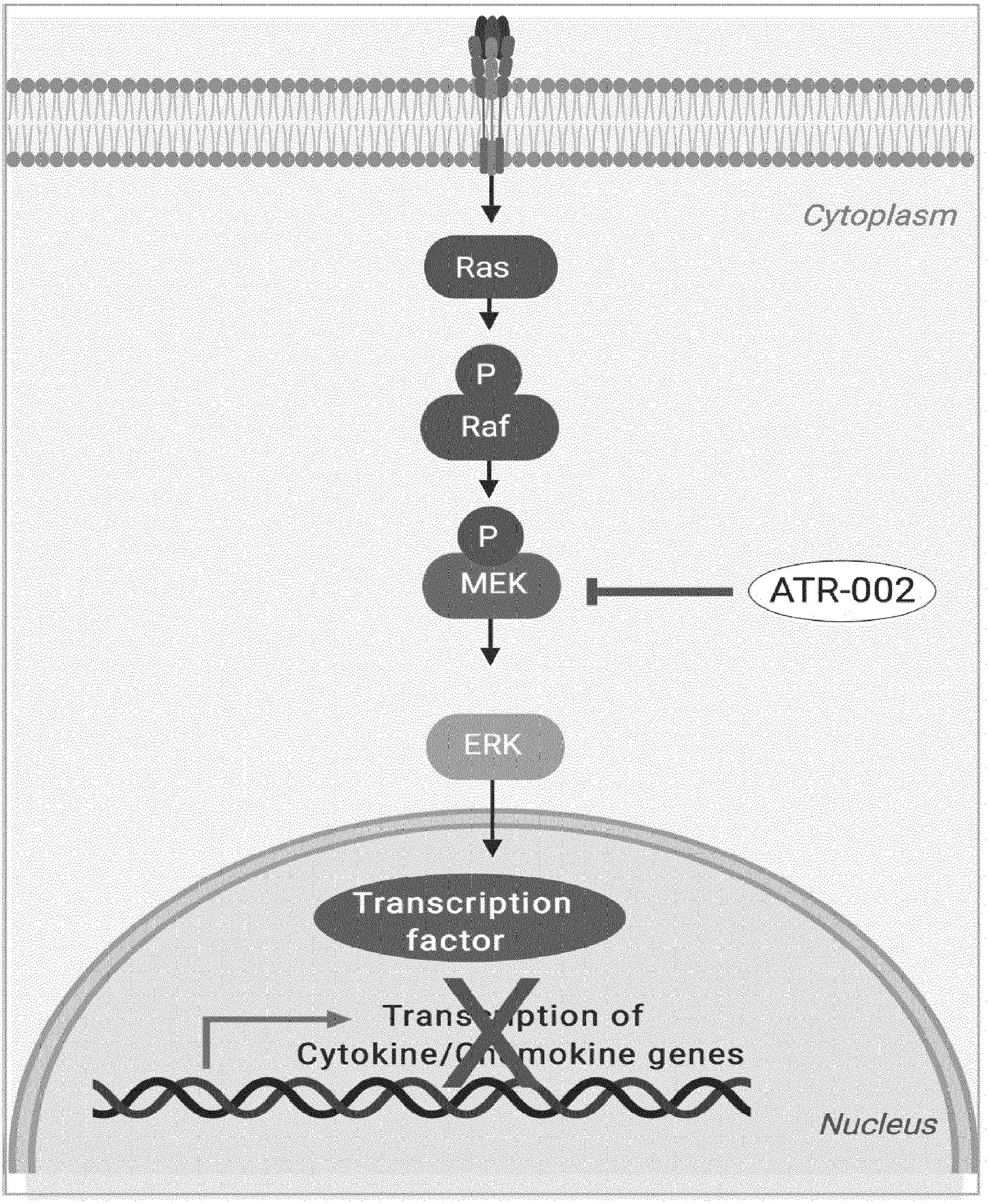

Figure 14

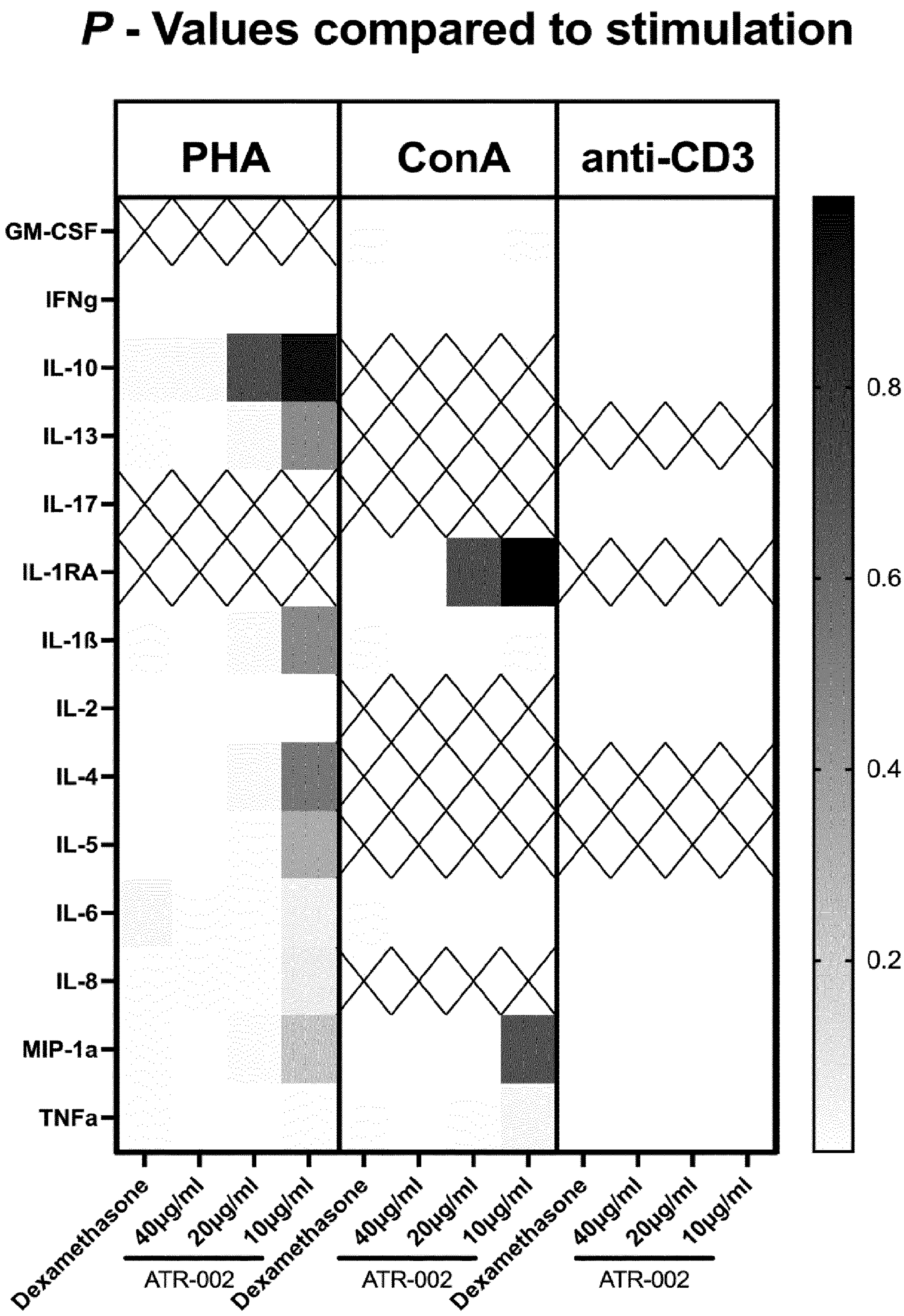
P - Values compared to stimulation
PHA
ConA
anti-CD3
GM-CSF
IFNg
IL-10
IL-13
IL-17
IL-1RA
IL-1ß
IL-2
IL-4
IL-5
IL-6
IL-8
MIP-1a
TNFa
0.8
0.6
0.4
0.2
Dexamethasone
40µg/ml
20µg/ml
10µg/ml
ATR-002
Dexamethasone
40µg/ml
20µg/ml
10µg/ml
ATR-002
Dexamethasone
40µg/ml
20µg/ml
10µg/ml
ATR-002

Figure 15

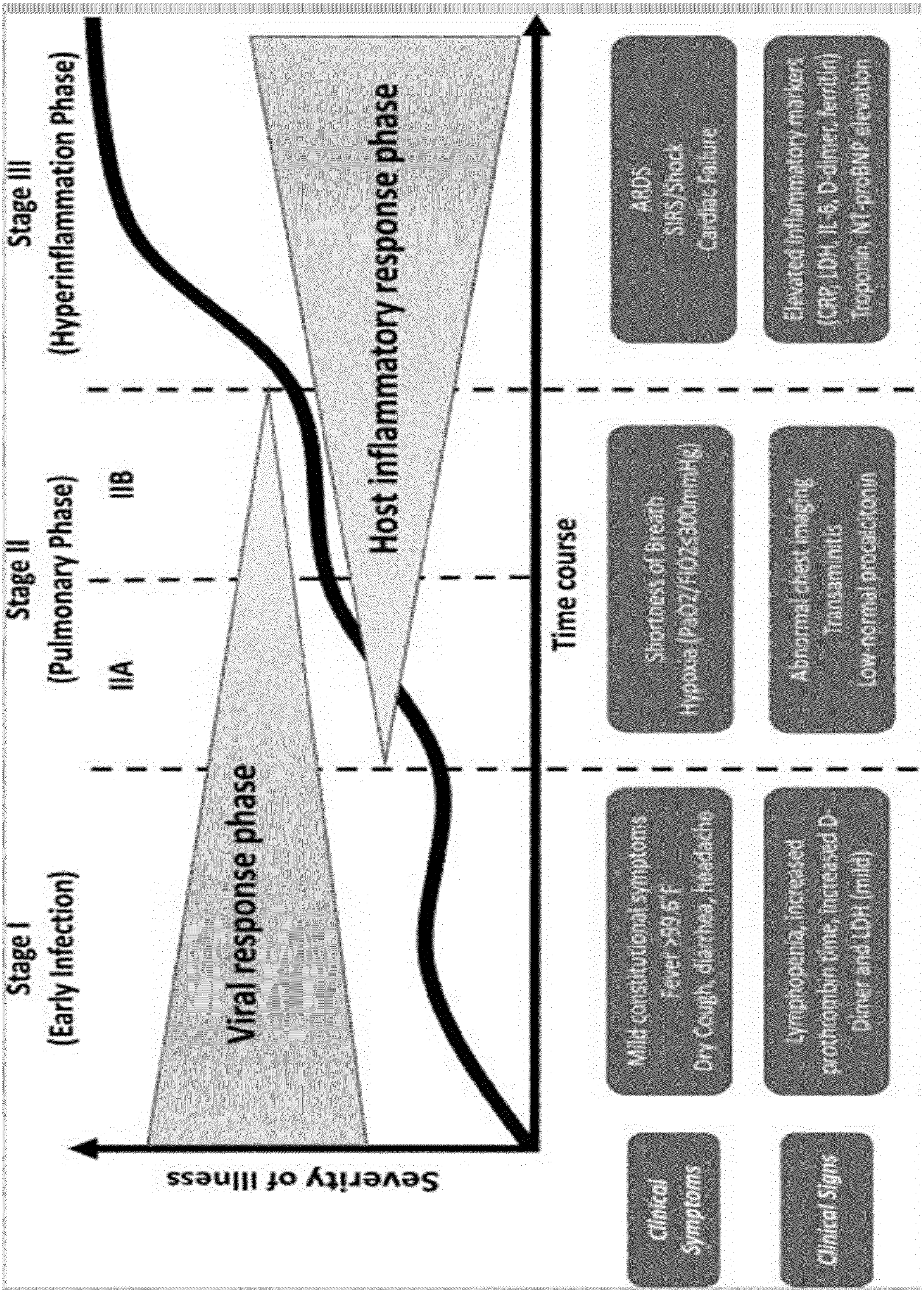
Stage I
(Early Infection)
Stage II
(Pulmonary Phase)
IIA
IIB
Stage III
(Hyperinflammation Phase)
Viral response phase
Host inflammatory response phase
Severity of Illness
Time course
Clinical Symptoms
Clinical Signs
Mild constitutional symptoms
Fever >99.6°F
Dry Cough, diarrhea, headache
Lymphopenia, increased prothrombin time, increased D-Dimer and LDH (mild)
Shortness of Breath
Hypoxia (PaO2/FiO2≤300mmHg)
Abnormal chest imaging
Transaminitis
Low-normal procalcitonin
ARDS
SIRS/Shock
Cardiac Failure
Elevated inflammatory markers
(CRP, LDH, IL-6, D-dimer, ferritin)
Troponin, NT-proBNP elevation

Figure 16

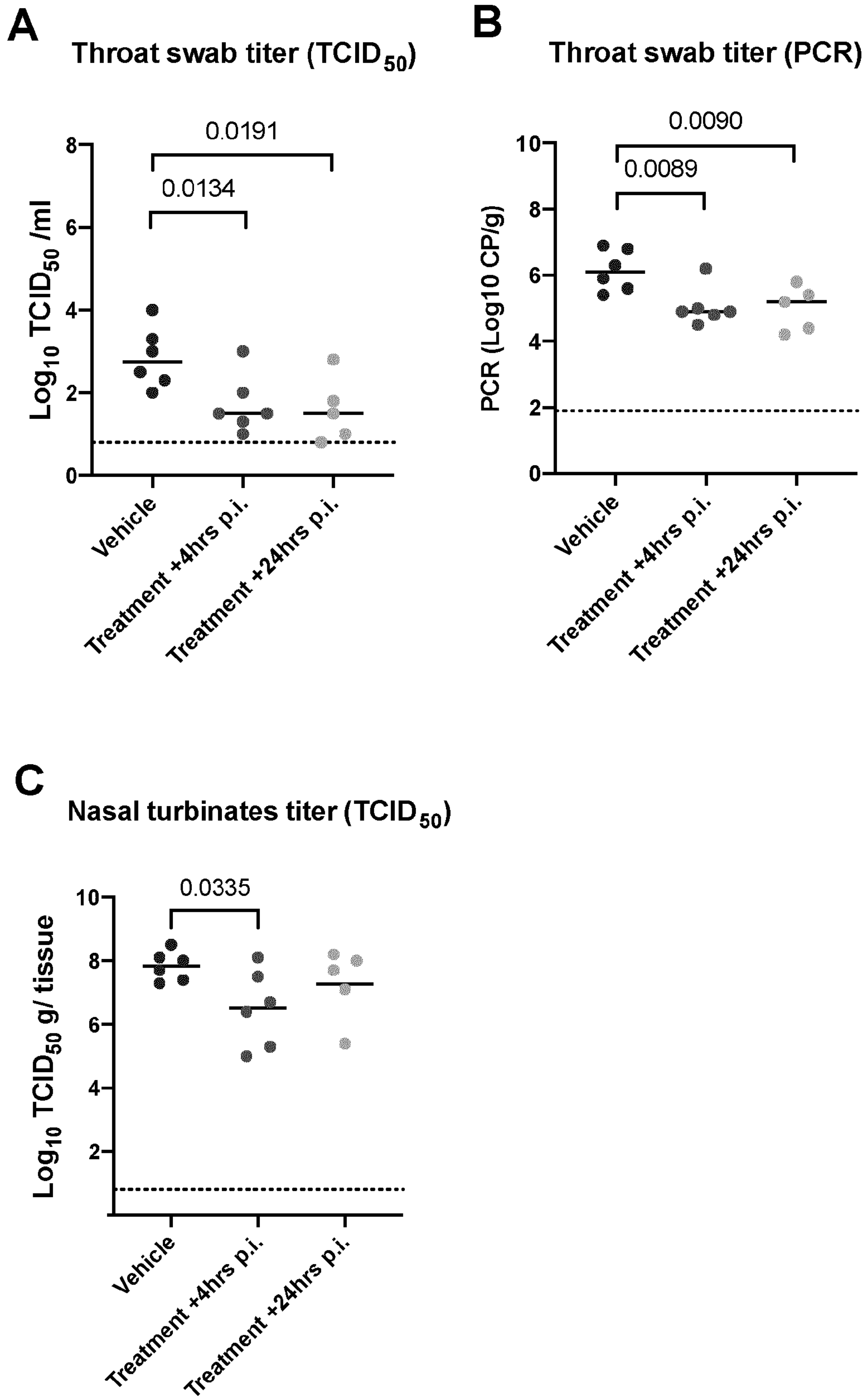
A
Throat swab titer (TCID50)
Log10 TCID50 /ml
0.0191
0.0134
Vehicle
Treatment +4hrs p.i.
Treatment +24hrs p.i.
B
Throat swab titer (PCR)
PCR (Log10 CP/g)
0.0090
0.0089
Vehicle
Treatment +4hrs p.i.
Treatment +24hrs p.i.
C
Nasal turbinates titer (TCID50)
Log10 TCID50 g/ tissue
0.0335
Vehicle
Treatment +4hrs p.i.
Treatment +24hrs p.i.

Figure 16 continued

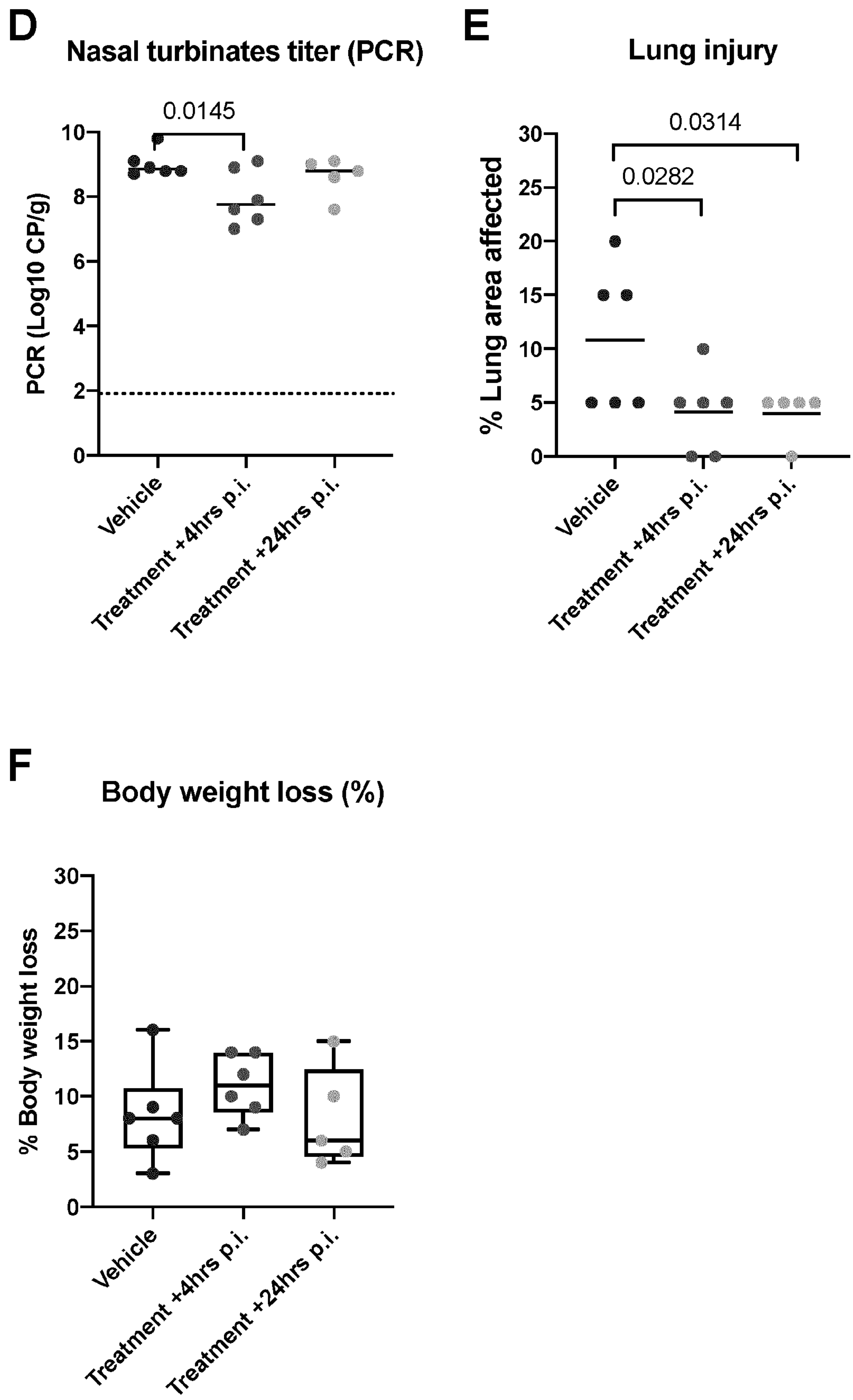

Figure 17

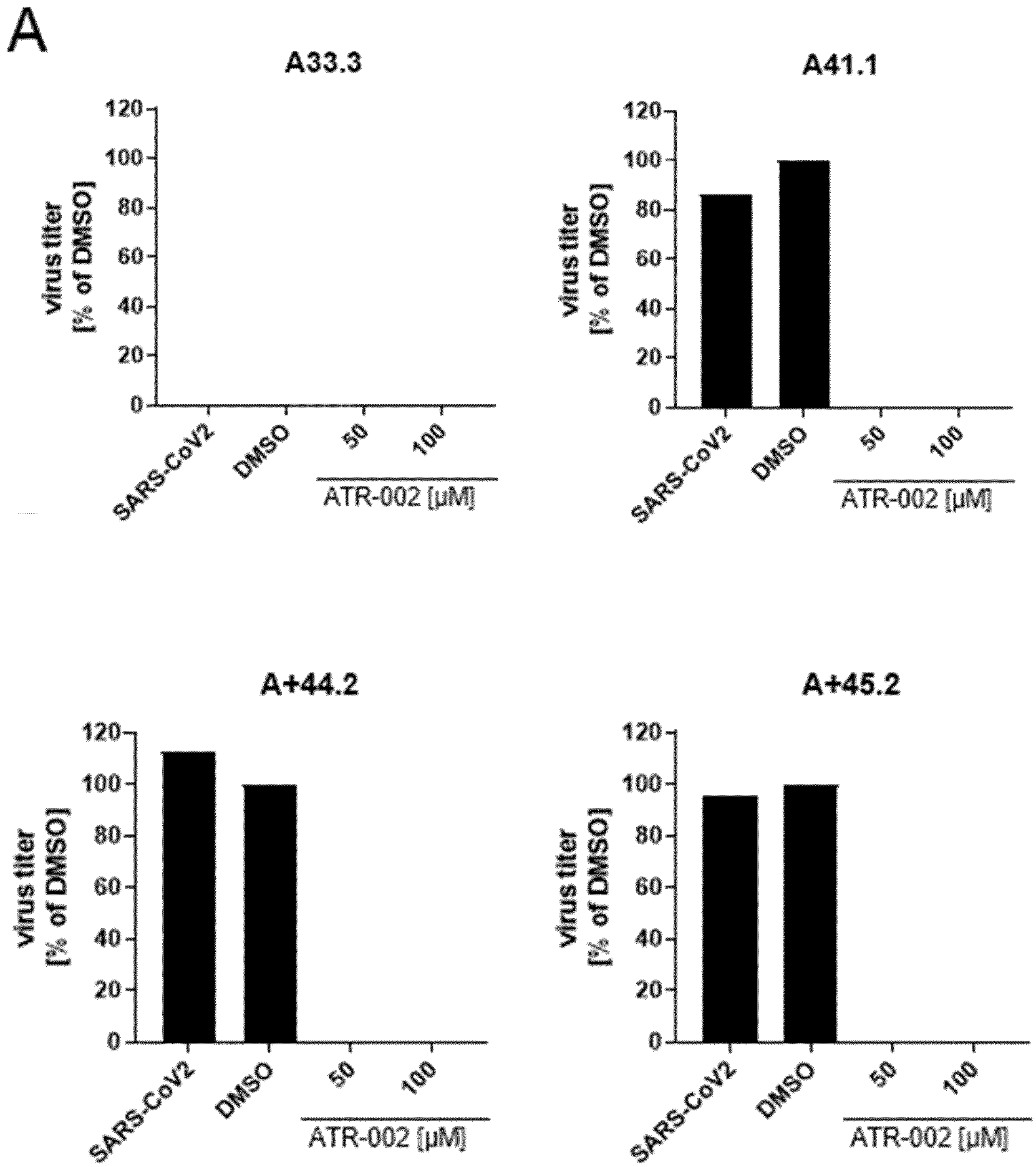
A
A33.3
120
100
80
60
40
20
0
virus titer
[% of DMSO]
SARS-CoV2
DMSO
50
100
ATR-002 [µM]
A41.1
120
100
80
60
40
20
0
virus titer
[% of DMSO]
SARS-CoV2
DMSO
50
100
ATR-002 [µM]
A+44.2
120
100
80
60
40
20
0
virus titer
[% of DMSO]
SARS-CoV2
DMSO
50
100
ATR-002 [µM]
A+45.2
120
100
80
60
40
20
0
virus titer
[% of DMSO]
SARS-CoV2
DMSO
50
100
ATR-002 [µM]

Figure 17 continued

B

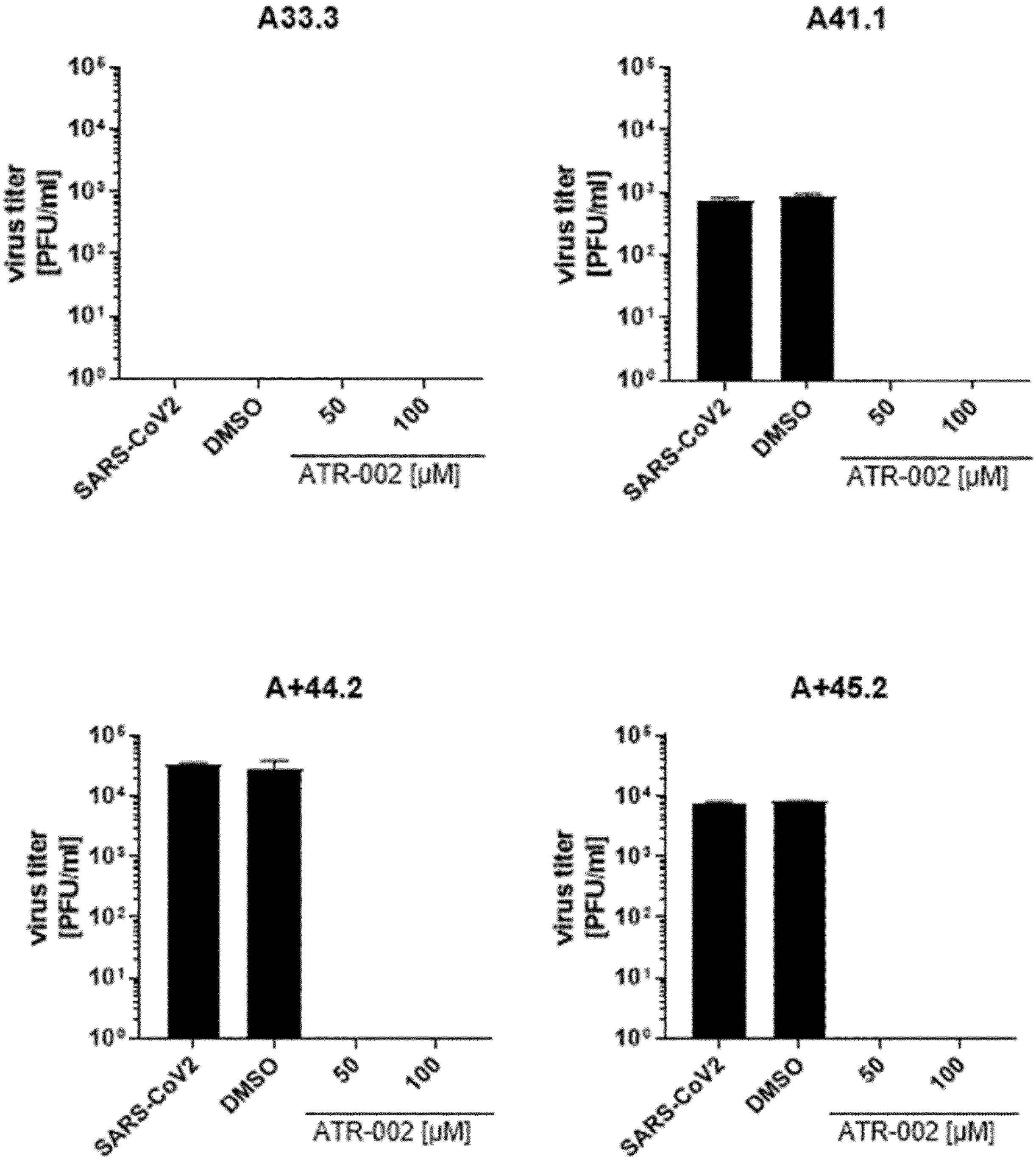

Figure 18

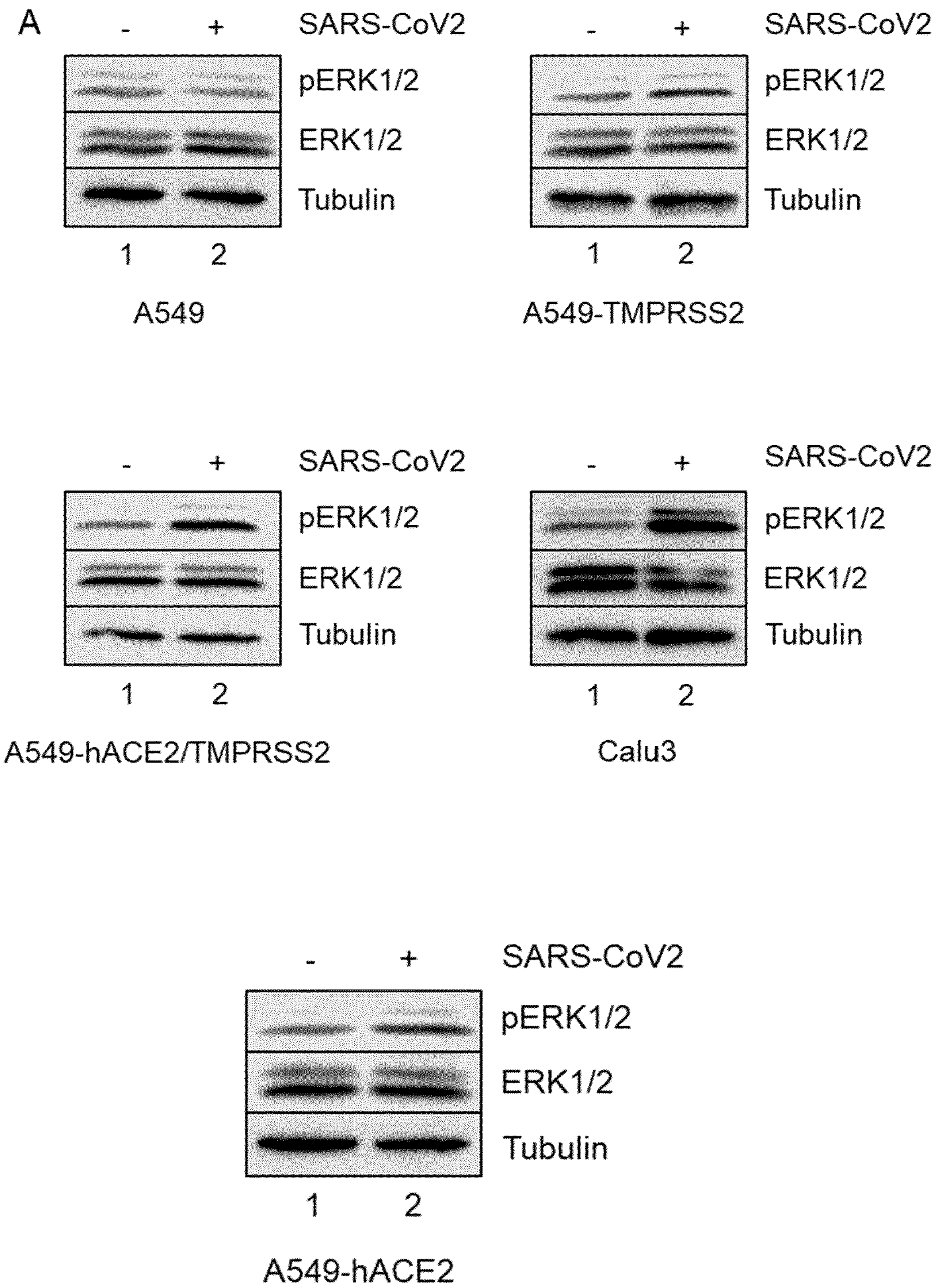
A
- + SARS-CoV2
pERK1/2
ERK1/2
Tubulin
1 2
A549
- + SARS-CoV2
pERK1/2
ERK1/2
Tubulin
1 2
A549-TMPRSS2
- + SARS-CoV2
pERK1/2
ERK1/2
Tubulin
1 2
A549-hACE2/TMPRSS2
- + SARS-CoV2
pERK1/2
ERK1/2
Tubulin
1 2
Calu3
- + SARS-CoV2
pERK1/2
ERK1/2
Tubulin
1 2
A549-hACE2

**Figure 18 continued**

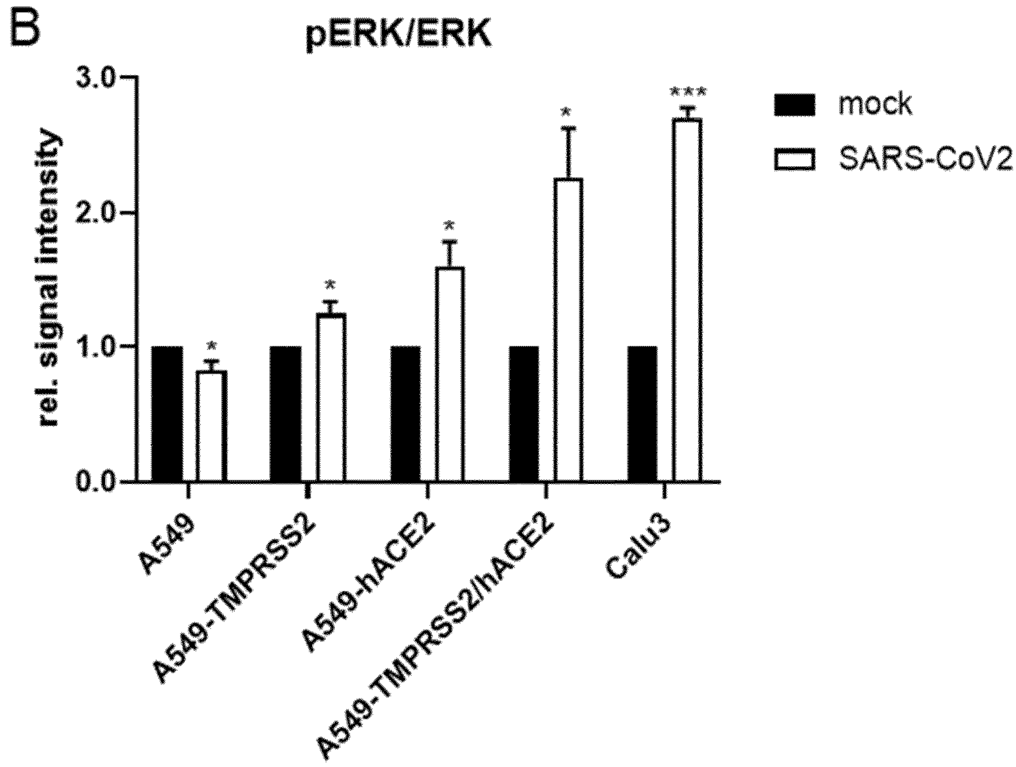

Figure 19

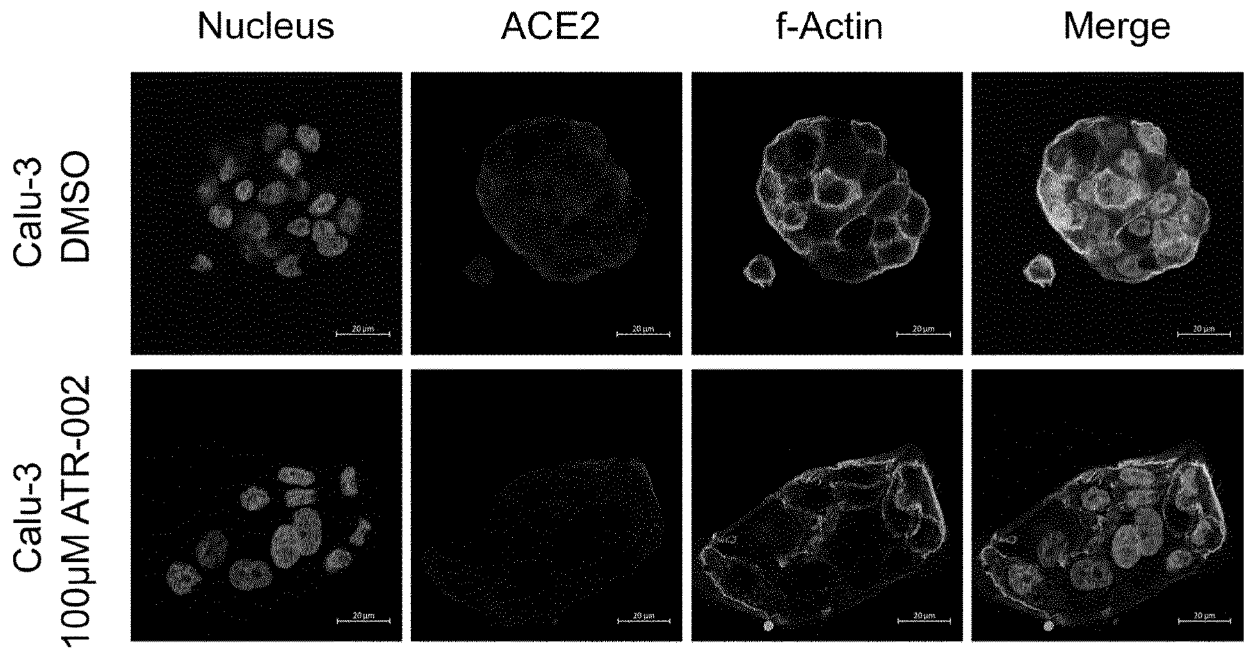
Nucleus
ACE2
f-Actin
Merge
Calu-3
DMSO
Calu-3
100µM ATR-002

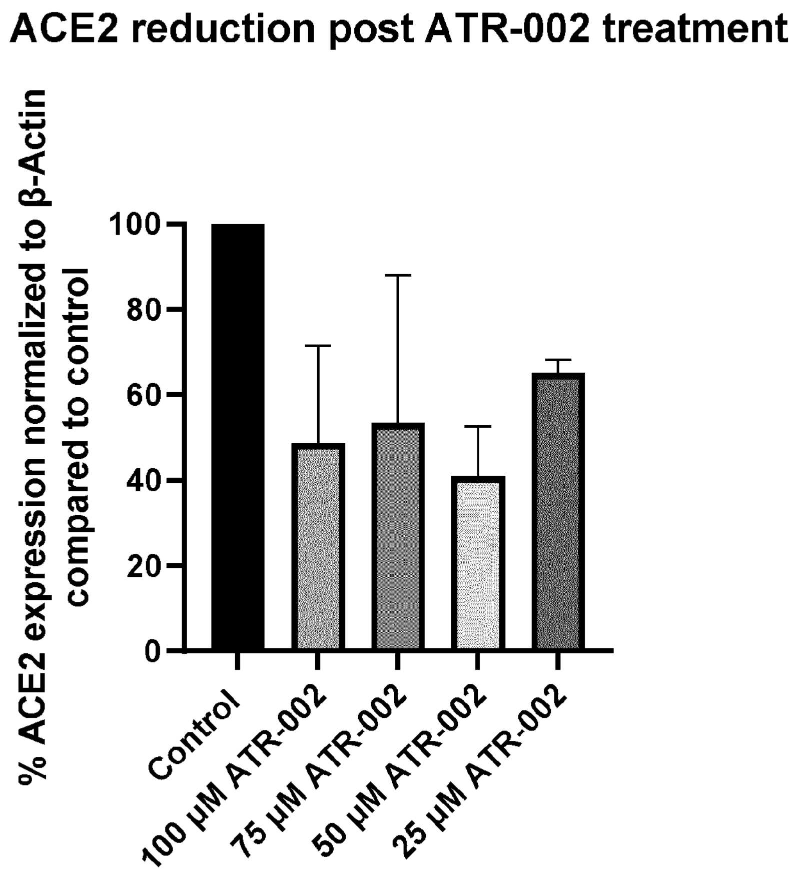
ACE2 reduction post ATR-002 treatment
% ACE2 expression normalized to β-Actin
compared to control
100
80
60
40
20
0
Control
100 µM ATR-002
75 µM ATR-002
50 µM ATR-002
25 µM ATR-002

Figure 20

A

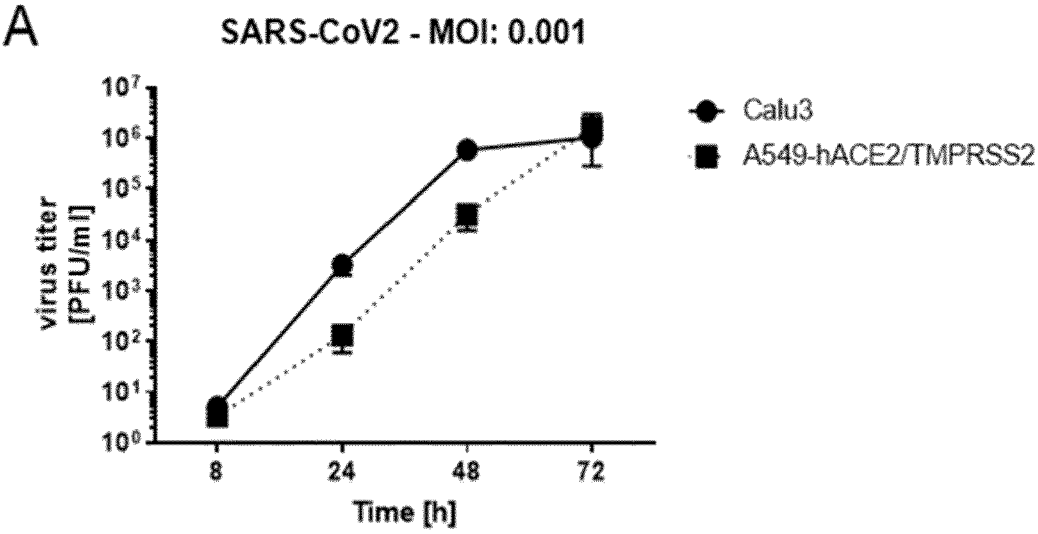


B

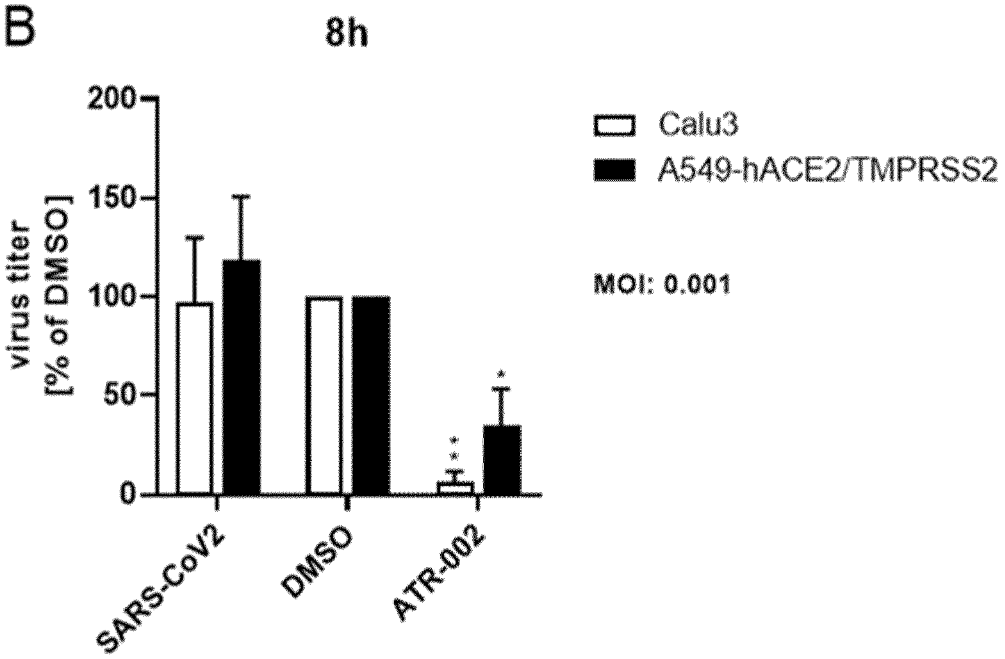


24h

Figure 20B continued

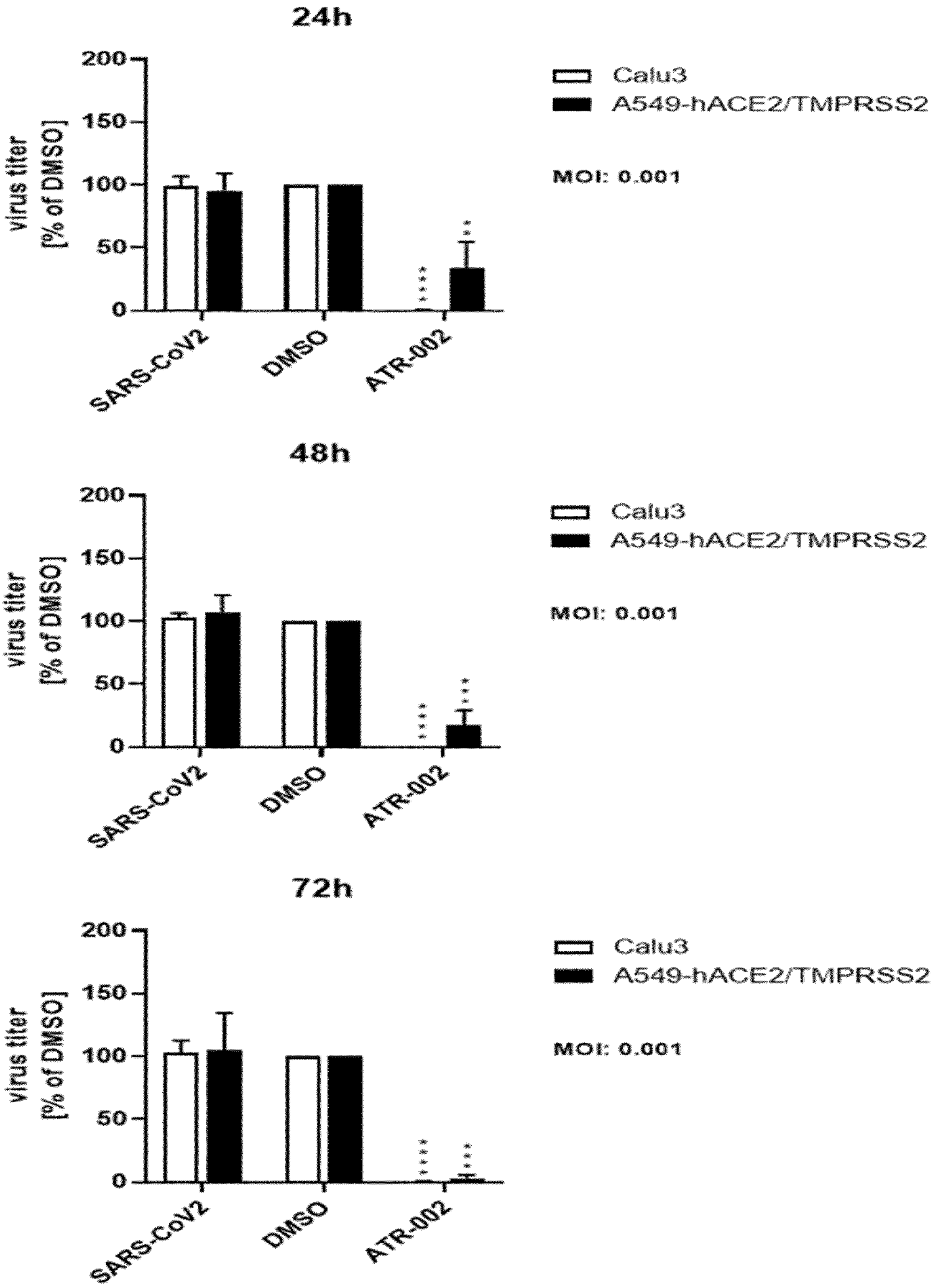

Figure 21 continued

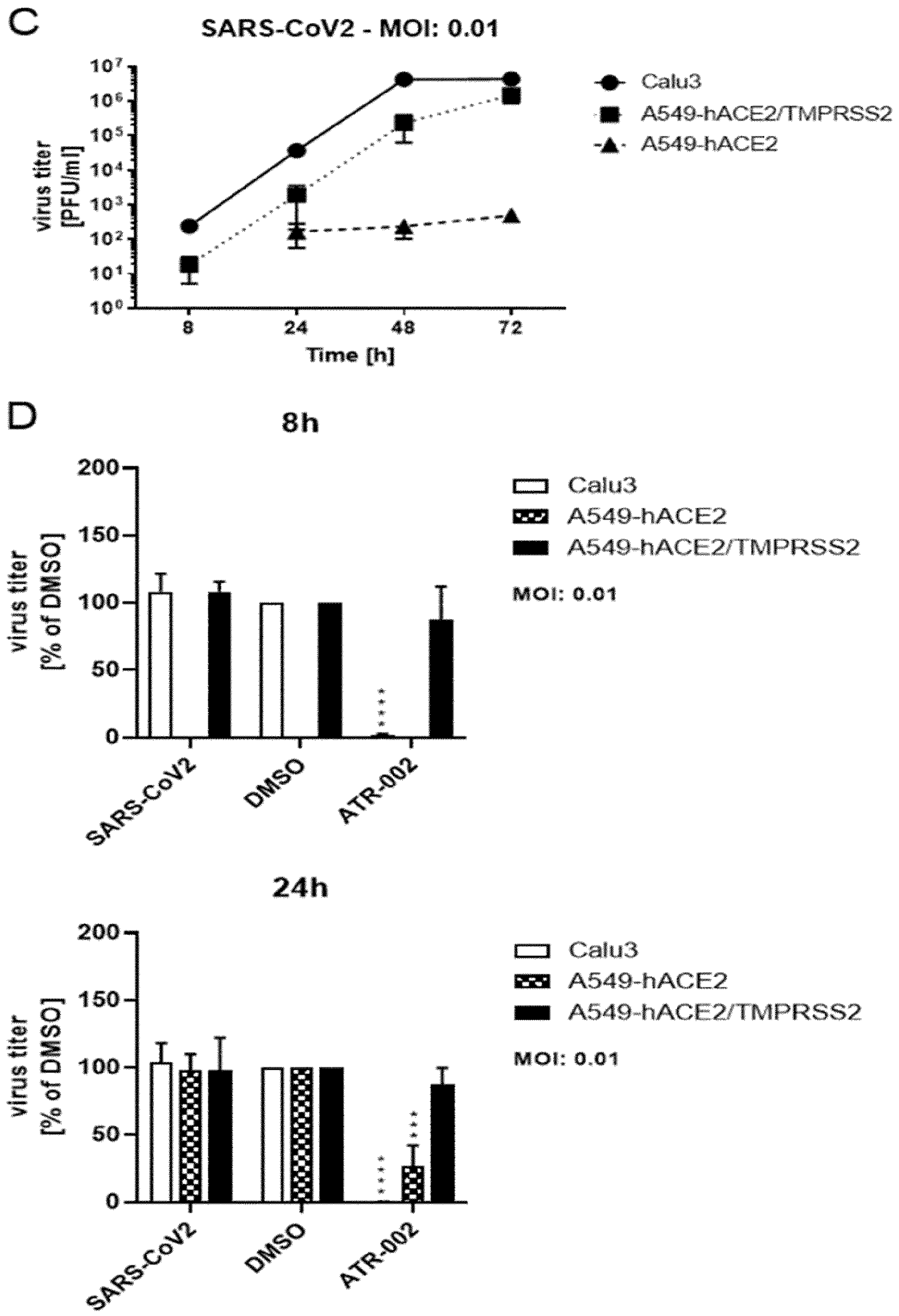

Figure 21D continued

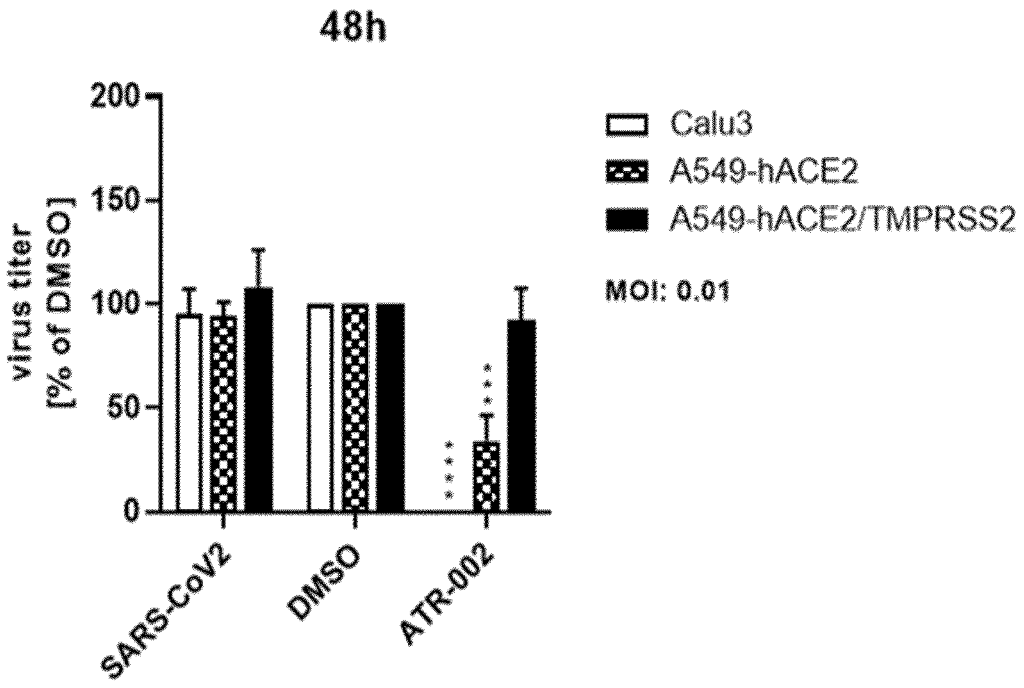


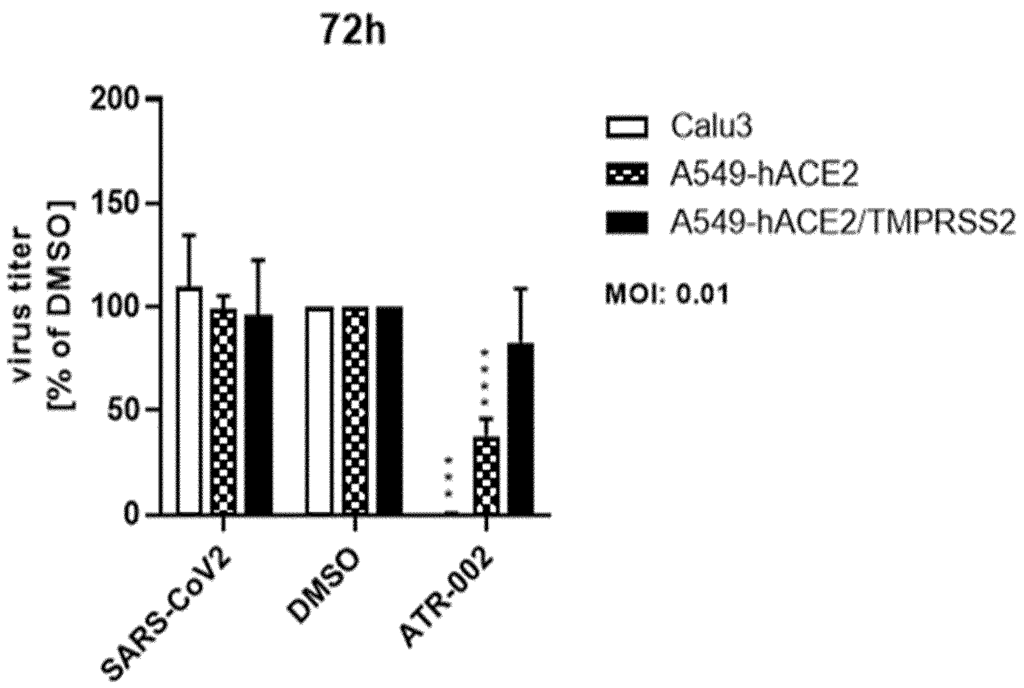

Figure 21

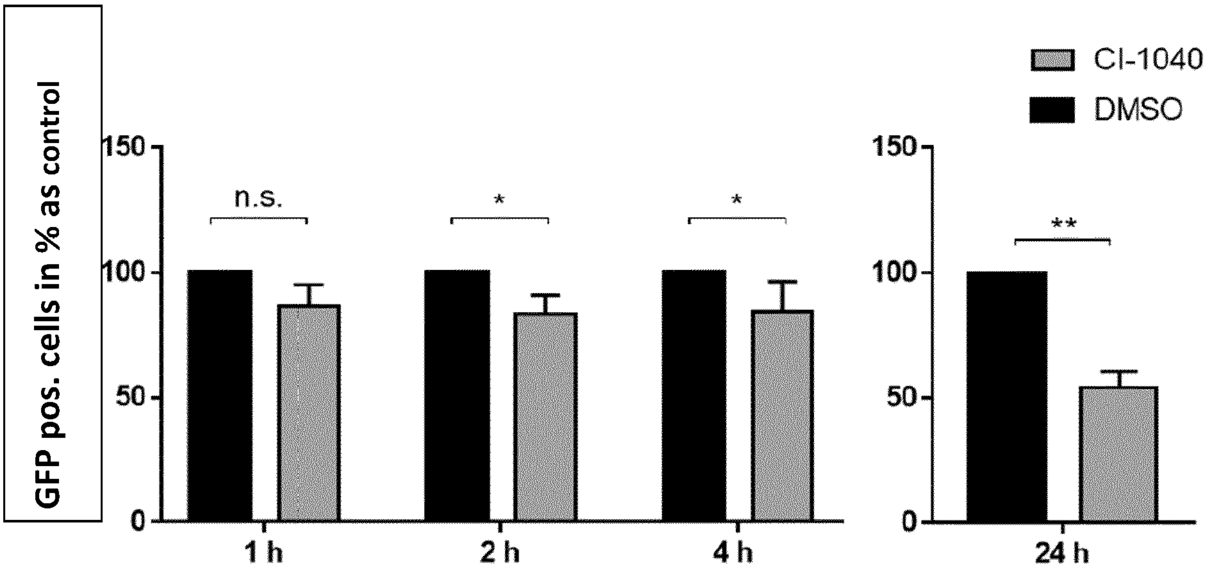


Preincubation with CI-1040

Figure 22

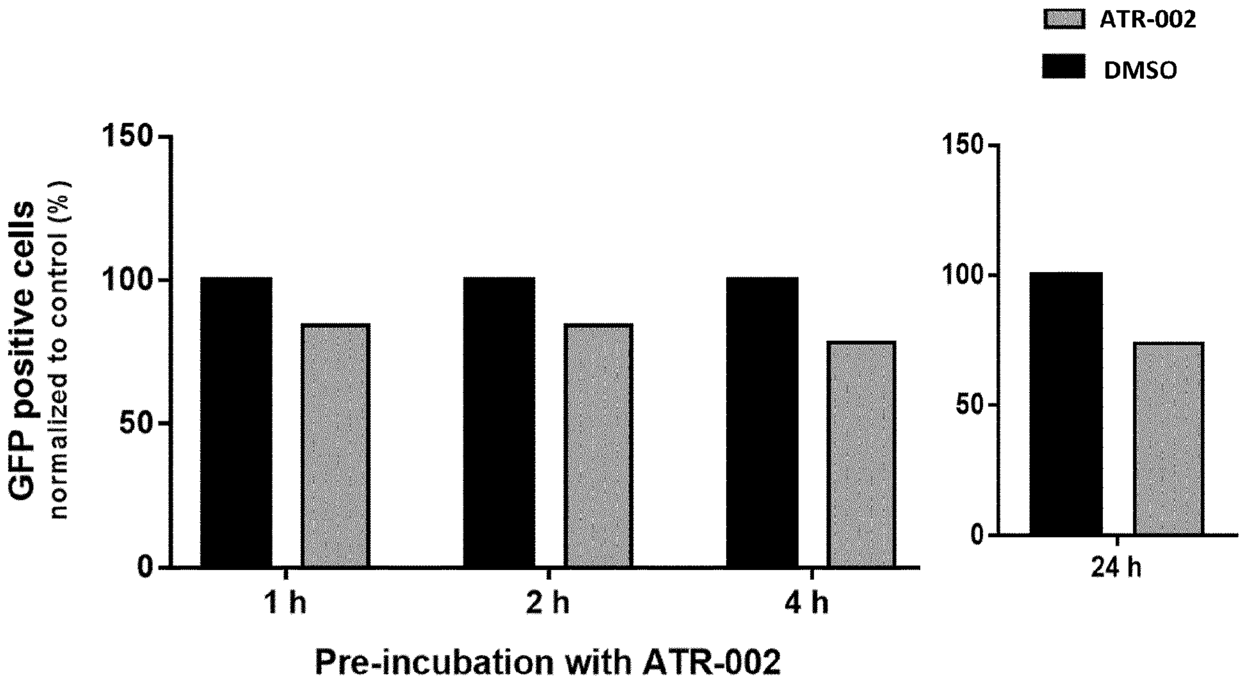
ATR-002
DMSO
150
100
50
0
GFP positive cells
normalized to control (%)
1 h
2 h
4 h
Pre-incubation with ATR-002
150
100
50
0
24 h

Figure 23

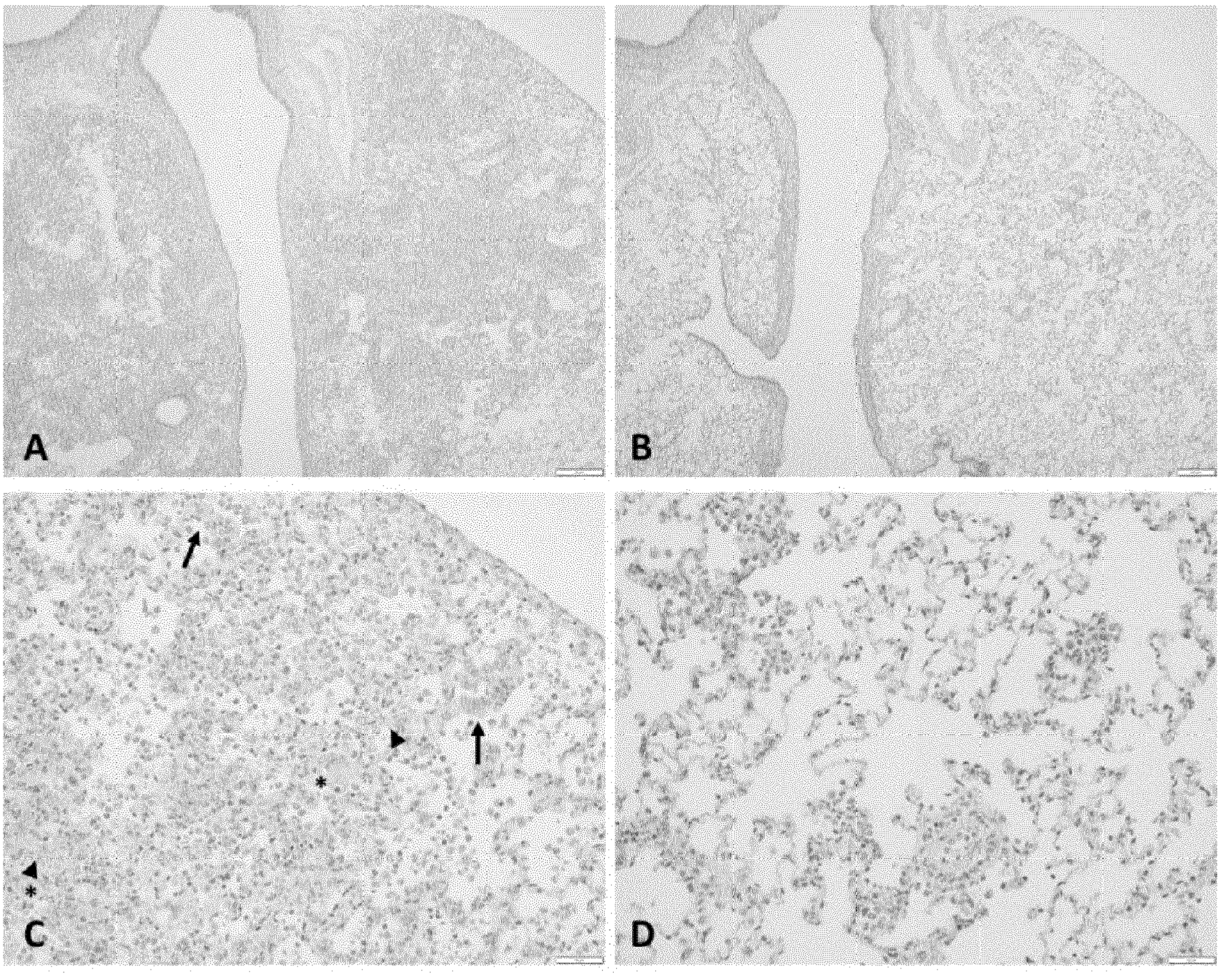

Figure 24

A

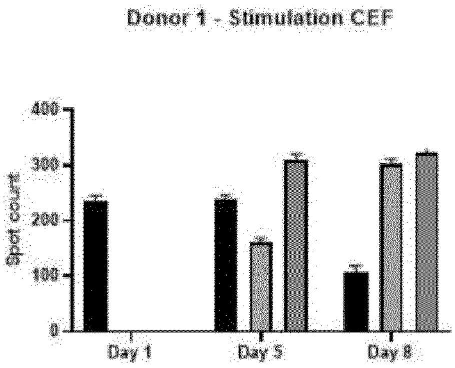


B

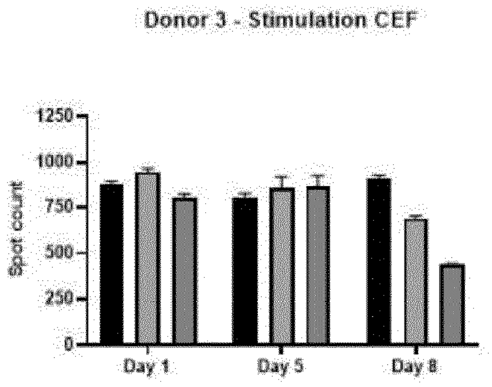


C

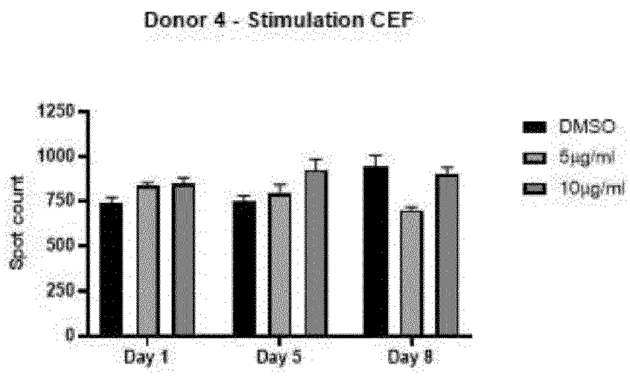


D

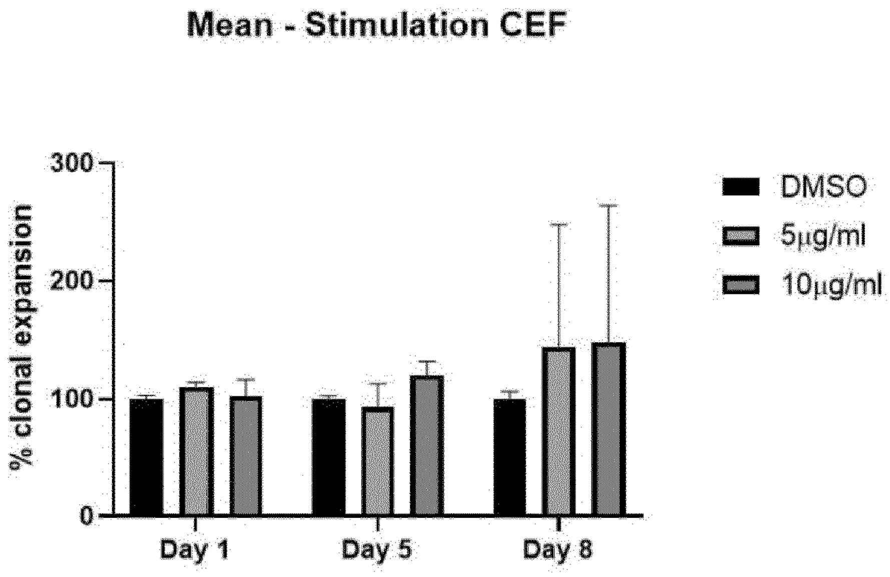

Europäisches Patentamt
European Patent Office
Office européen des brevets

# EUROPEAN SEARCH REPORT

Application Number
EP 21 21 0256

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/212478 A1 (ATRIVA THERAPEUTICS GMBH [DE]) 22 October 2020 (2020-10-22) * claims 1,9-11,18-21 * ----- | 1-5,8,9, 15 | INV. A61K31/196 A61P31/14 |
| X | WO 2020/188034 A1 (ATRIVA THERAPEUTICS GMBH [DE]) 24 September 2020 (2020-09-24) * page 4, paragraph 2; claims 1,5,8,9,12-15 * ----- | 15 | |
| Y | WO 2021/216562 A1 (FINZI ERIC [US]) 28 October 2021 (2021-10-28) * the whole document * ----- | 1-15 | |
| Y | Anonymous: "Atriva Therapeutics' lead candidate Zapnometinib shows substantial efficacy against SARS-CoV-2 ¦ Atriva", , 9 November 2021 (2021-11-09), XP055916082, Retrieved from the Internet: URL:https://www.atriva-therapeutics.com/2021/11/09/atriva-therapeutics-lead-candidate-zapnometinib-shows-substantial-efficacy-against-sars-cov-2/ [retrieved on 2022-04-28] * the whole document * ----- | 1-15 | TECHNICAL FIELDS SEARCHED (IPC) A61K A61P |
| Y | Anonymous: "RESPIRE trial of therapeutic for all strains of COVID-19 gets underway in Germany - International Hospital", , 13 April 2021 (2021-04-13), XP055916079, Retrieved from the Internet: URL:https://interhospi.com/respire-trial-of-therapeutic-for-all-strains-of-covid-19-gets-underway-in-germany/ [retrieved on 2022-04-28] * the whole document * ----- -/-- | 1-15 | |

The present search report has been drawn up for all claims

| Place of search | Date of completion of the search | Examiner |
|---|---|---|
| Munich | 28 April 2022 | Ganschow, Silke |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 21 21 0256

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Anonymous: "Erster Patient erhält Medikation in Atriva Therapeutics Phase II-Studie RESPIRE bei COVID-19 ¦ BioRegio STERN ¦ Thinking business forward", , 13 April 2021 (2021-04-13), XP055916075, Retrieved from the Internet: URL:https://www.bioregio-stern.de/de/BranchenNews/erster-patient-erhaelt-medikation-in-atriva-therapeutics-phase-ii-studie-respire-bei [retrieved on 2022-04-28] * the whole document * ----- | 1-15 | |
| E | EP 3 912 623 A1 (ATRIVA THERAPEUTICS GMBH [DE]) 24 November 2021 (2021-11-24) * the whole document * ----- | 1-5,8,9, 15 | |

TECHNICAL FIELDS SEARCHED (IPC)

The present search report has been drawn up for all claims

1

| Place of search | Date of completion of the search | Examiner |
|---|---|---|
| Munich | 28 April 2022 | Ganschow, Silke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 21 21 0256

This annex lists the patent family members relating to the patent documents cited in the above-mentioned European search report. The members are as contained in the European Patent Office EDP file on
The European Patent Office is in no way liable for these particulars which are merely given for the purpose of information.

28-04-2022

| Patent document cited in search report | Publication date | Patent family member(s) | Publication date |
|---|---|---|---|
| WO 2020212478 A1 | 22-10-2020 | AU 2020258002 A1 | 18-11-2021 |
| | | CA 3136171 A1 | 22-10-2020 |
| | | CN 114007598 A | 01-02-2022 |
| | | EA 202192454 A1 | 22-03-2022 |
| | | EP 3955908 A1 | 23-02-2022 |
| | | KR 20210153642 A | 17-12-2021 |
| | | LU 101183 B1 | 16-10-2020 |
| | | WO 2020212478 A1 | 22-10-2020 |
| WO 2020188034 A1 | 24-09-2020 | AU 2020243160 A1 | 14-10-2021 |
| | | CA 3133044 A1 | 24-09-2020 |
| | | CN 113874012 A | 31-12-2021 |
| | | EP 3941464 A1 | 26-01-2022 |
| | | KR 20210142125 A | 24-11-2021 |
| | | WO 2020188034 A1 | 24-09-2020 |
| WO 2021216562 A1 | 28-10-2021 | NONE | |
| EP 3912623 A1 | 24-11-2021 | EP 3912623 A1 | 24-11-2021 |
| | | WO 2021234097 A1 | 25-11-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HUANG et al.** *The Lancet,* 15 February 2020, vol. 395, 497-506
- **SHIN JIE YONG.** Long COVID or post-COVID-19 syndrome: putative pathophysiology, risk factors, and treatments. *INFECTIOUS DISEASES,* 2021, vol. 0 (0), 1-18
- **GREENHALGH, T. ; KNIGHT, M. ; COURT, C. ; BUXTON, M. ; HUSAIN, L.** Management of post-acute Covid-19 in primary care. *BMJ,* 2020, vol. 370, m3026
- **LOPEZ-LEON, S. ; WEGMAN-OSTROSKY, T. ; PERELMAN, C. ; SEPULVEDA, R. ; REBOLLEDO, P.A. ; CUAPIO, A. ; VILLAPOL, S.** More than 50 long-term effects of Covid-19: a systematic review and meta-analysis. *medRxiv,* 2021
- **HUANG, C. ; HUANG, L. ; WANG, Y. ; LI, X. ; REN, L. ; GU, X. ; CAO, B.** 6-month consequences of Covid-19 patients discharged from hospital: a cohort study. *Lancet,* 2021, vol. 397, 220-232
- **TOWNSEND, L. ; DOWDS, J. ; O'BRIEN, K. ; SHEILL, G. ; DYER, A. ; O'KELLY, B. ; BANNAN, C.** Persistent poor health post-Covid-19 is not associated with respiratory complications or initial severity. *AnnalsATS,* 2021
- **MAHMUD, R. ; RAHMAN, M. ; RASSEL, M. ; MONAYEM, F. ; SAYEED, S. ; ISLAM, M. S. ; ISLAM, M. M.** Post-Covid-19 syndrom among symptomatic Covid-19 patients. A prospective cohort study in a tertiary care center of Bangladesh. *PLOS ONE,* 2021, vol. 16 (4
- **ASHER MULLARD.** Long COVID's long R&D agenda. *NATURE REVIEWS, DRUG DISCOVERY,* 20 May 2021, 329-331
- **SHIN JIE YONG.** Long COVID or post-COVID-19 syndrome: putative pathophysiology, rsik factors, and treatments. *INFECTIOUS DISEASES,* 2021, vol. 0 (0), 1-18
- **JAMILLOUX et al.** *Autoimmunity Reviews 2020*
- **CORMAN et al.** Detection of 2019 novel coronavirus (2019-nCoV) by real-time RT-PCR. *Euro Surveill,* vol. 25 (3
- **NELDE, A. ; BILICH, T. ; HEIMANN, J.S. et al.** SARS-CoV-2-derived peptides define heterologous and COVID-19-induced T cell recognition. *Nat Immunol,* 2021, vol. 22, 74-85
- **HAASBACH, E. et al.** The MEK-inhibitor CI-1040 displays a broad anti-influenza virus activity in vitro and provides a prolonged treatment window compared to standard of care in vivo. *Antiviral research,* 2017, vol. 142, 178-184
- **HASAN et al.** COVID-19 Illness in Native and Immunosuppressed States: A Clinical-Therapeutic Staging Proposal. *J Heart Lung Transplant,* 20 March 2020
- **JAMILLOUX et al.** Should we stimulate or suppress immune response in COVID-19? Cytokine and anticytokine interventions. *Autoimmunity Reviews, AUTREV 102567,* 28 April 2020
- **LOPEZ-LEON, S. ; WEGMAN-OSTROSKY, T. ; PERELMAN, C. ; SEPULVEDA, R. ; REBOLLEDO, P.A. ; CUAPIO, A. ; VILLAPOL, S.** More than 50 long-term effects of Covid-19: a systematic review and meta- analysis. *medRxiv,* 2021
- **LORUSSO, P. ; ADJEI, A. ; VARTERASIAN, M. ; GADGEEL, S. ; REID, J. ; MITCHELL, D. et al.** Phase I and Pharmacodynamic Study of the Oral MEK Inhibitor CI-1040 in Patients With Advanced Malignancies. *Journal of Clinical Oncology,* 2005, vol. 23 (23), 5281-5293
- **LUDWIG, S.** Targeting cell signaling pathways to fight the flu: towards a paradigm change in anti-influenza therapy. *Journal of Antimicrobial Chemotherapy,* 2009, vol. 64, 1-4
- **MACKEY, T.K. ; LIANG, B.A.** Lessons from SARS and H1N1/A: employing a WHO-WTO forum to promote optimal economic-public health pandemic response. *J Public Health Policy,* 2012, vol. 33, 119-130
- **PLESCHKA, S. ; WOLFF, T. ; EHRHARDT, C. ; HOBOM, G. ; PLANZ, O. ; RAPP, U.R. ; LUDWIG, S.** Influenza virus propagation is impaired by inhibition of the Raf/MEK/ERK signalling cascade. *Nat Cell Biol,* 2001, vol. 3, 301-305
- **WABNITZ, A. ; MITCHELL, D ; WABNITZ, D.** In Vitro and in Vivo Metabolism of the Anti-Cancer Agent CI-1040, a MEK Inhibitor, in Rat, Monkey, and Human. *Pharmaceutical Research,* 2004, vol. 21 (9), 1670-1679